(19) Europäisches Patentamt
European Patent Office
Office européen des brevets

(11) EP 1 038 864 A1

(12) EUROPEAN PATENT APPLICATION
published in accordance with Art. 158(3) EPC

(43) Date of publication:
**27.09.2000 Bulletin 2000/39**

(21) Application number: **98959181.3**

(22) Date of filing: **11.12.1998**

(51) Int. Cl.[7]: **C07C 259/06**

(86) International application number:
**PCT/JP98/05620**

(87) International publication number:
**WO 99/31052 (24.06.1999 Gazette 1999/25)**

(84) Designated Contracting States:
**AT BE CH CY DE DK ES FI FR GB GR IE IT LI LU MC NL PT SE**

(30) Priority: **12.12.1997 JP 36236497**
**17.07.1998 JP 21867698**

(71) Applicant:
**FUJI YAKUHIN KOGYO KABUSHIKI KAISHA**
**Takaoka-shi Toyama 933-8511 (JP)**

(72) Inventors:
- **FUJISAWA, Tetsunori**
  **Takaoka-shi, Toyama 933-8511 (JP)**
- **ODAKE, Shinjiro**
  **Takaoka-shi, Toyama 933-8511 (JP)**
- **HONGO, Kazuya**
  **Takaoka-shi, Toyama 933-8511 (JP)**
- **OHTANI, Miwa**
  **Takaoka-shi, Toyama 933-8511 (JP)**
- **YASUDA, Junko**
  **Takaoka-shi, Toyama 933-8511 (JP)**
- **MORIKAWA, Tadanori**
  **Takaoka-shi, Toyama 933-8511 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(54) **NOVEL METALLOPROTEINASE INHIBITORS**

(57)

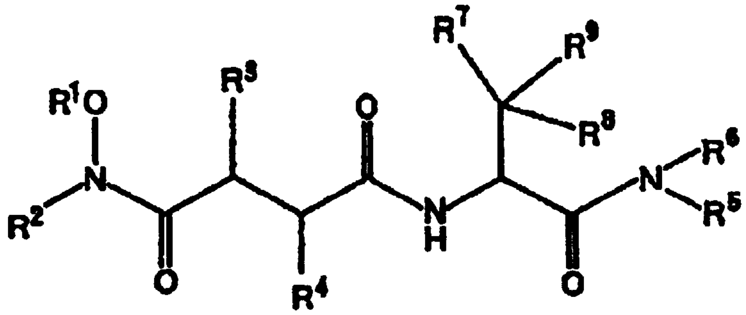

(I)

Compounds having not only a high metalloproteinase inhibitory activity but also remarkably improved medicinal applicability *in vivo* (oral absorbability, etc.) and biological activities and thus being useful a drugs; intermediates in the process for producing the same; and a process for producing these compounds. Specifically, compounds represented by general formula (I) or salts thereof, useful in medicinal and/or veterinary compositions, in particular; metalloproteinase inhibitors inhibiting matrix metalloproteinases and tumor necrosis factor-α (TNF-α) convertase; wherein $R^1$ represents hydrogen or a hydroxy-protective group; $R^2$ represents hydrogen or an amino-protective group; $R^3$, $R^7$ and $R^8$ represent each hydrogen, hydroxy, alkyl, etc.; $R^4$ represents alkyl, arylalkyl, etc.; $R^5$ and $R^6$ are the same or different and each represents hydrogen, alkyl, cycloalkyl, etc.; and $R^9$ represents hydrogen, hydroxy, amino, etc.

EP 1 038 864 A1

## Description

Field of the Invention

The present invention relates to novel compounds which inhibit metalloproteinases, intermediates for the production thereof and processes for producing the same. More preferably, the present invention relates to novel compounds, having advantageous biological properties such as excellent oral bioavailability, which inhibit vertebrate matrix metalloproteinases (MMPs) and/or tumor necrosis factor-α (TNF-α )-converting enzymes (TNF-α convertases), intermediates for the production thereof and processes for producing the same.

Background of the Invention

Among metalloproteinases, matrix metalloproteinases ("MMPs") are a family of endoproteinases containing zinc. The MMPs are involved in the degradation of extracellular matrices in connective tissues. Up until now, it has been known that the MMPs include dozens of MMP species. The expression of these enzymes is strictly controlled in healthy persons; however, abnormal increases of MMPs are observed in Alzheimer's disease, Parkinson's disease, pancreatitis, ulcerative colitis, aphthous ulcer, autoimmune diseases (including chronic rheumatoid arthritis, Crohn's disease, and anemia associated with autoimmune diseases), osteoarthritis, periodontal diseases and disorders, corneal ulcer, uveitis, a variety of bullae (including congenital epidermolysis bullosa, acquired epidermolysis bullosa, porphyria cutanea tarda (PCT), pemphigoid, and pemphigus vulgaris), refractory dermal ulcers (including bedsore, dermal ulcer in radiotherapeutic patients, dermal ulcer in diabetic patients, and dermal ulcer in patients suffering from arteriosclerosis obliterans), osteoporosis, Behcet's disease, aberrant angiogenesis (accompanying tumor growth, and including lymphoma, ovary cancer, and tumor metastasis and invasion), cachexia, various infectious diseases (including malaria, hepatitis C, HIV infection, tuberculosis, and septicemia), multiple sclerosis, psoriasis, diabates, schizophrenia, depression, etc., and these MMPs are thought to be involved in the breakdown of extracellular matrix (D.Brown et al., Current Eye Research, 12, 571(1993); Y. Okada et al., Virchow Archiv B Cell Pathol., 59, 305(1990); W. G. Stetler-Stevenson, Cancer and Metastasis Reviews, 9, 289 (1990); H. Birkedal-Hansen et al., Critical Reviews in Oral Biology and Medicine, 4(2), 197 (1993)).

TNF-α is produced as a membrane-coupled type precursor with a molecular weight of 26 k. An excessive extracellular release of TNF-α is thought to lead to disorders including septicemia, chronic rheumatoid arthritis and the like. It has been recently reported that an enzyme capable of inducing the release of TNF-α (i.e., TNF-α convertase) is a kind of metalloproteinases and its activity can be controlled by inhibitors against MMPs (A. J. H. Gearing et al., Journal of Leukocyte Biology, 57, 774(1995); K. M. Mohler et al., Nature, 370, 218(1994); G. M. NcGeehan et al., Nature, 370, 558(1994)).

Accordingly, inhibition of the action of such enzymes is thought to be potentially effective in therapeutically treating the above diseases and disorders. The prior art compounds which inhibit the action of MMPs are known to include 4 groups, i.e., oxygenated phosphorus-based derivatives, hydroxamic acid-based derivatives, derivatives having a mercapto group, and derivatives having a carboxyl group. Especially, hydroxamic acid-based derivatives have been proposed with a variety of backbones (see: U.S.Pat.No. 4,599,361; EP 0 575 844 A2; U.S.Pat.No. 5,412,145; WO 92/13831 A; U.S.Pat. No. 5,183,900; WO 94/02447 A; EP 0 606 046 A1; & GB 2,268,933 A). A number of these compounds have the property of inhibiting various MMPs.

Exceptionally, WO 96/33968 A discloses a class of compounds with remarkably improved water-solubility though the water-solubility is a subject to be solved in the prior art.

For administration of drugs, injections not only put too heavy a load on patients but also have serious drawbacks in view of readiness and convenience for cases where patients take the drug by themselves. Furthermore, most of diseases and disorders associated with the degradation of tissues often turn to chronic, thereby necessitating the continued use of drugs lasting for a long time. In such cases, oral administration is thought to be the most suitable route.

However, it is hard to orally administer the prior art compounds in such manners. Therefore, they are still unsatisfactory therapeutic agents at this stage.

Disclosure of the Invention

The present inventors have conducted an extensive research on hydroxamic acid-based derivatives with high inhibitory activity against MMPs with a view to improving and increasing their utilizability. As a result, the present inventors have succeeded in (1) producing novel hydroxamic acid derivatives which not only have unexpectedly high inhibitory activity against MMPs but also exert (i) potent bioavailability, including the property of being significantly well absorbed by oral routes, and (ii) excellent biological activity, superior to those of the prior art compounds, and (2) providing the present invention.

Thus, the present invention provides

(1) a compound having the following formula (I):

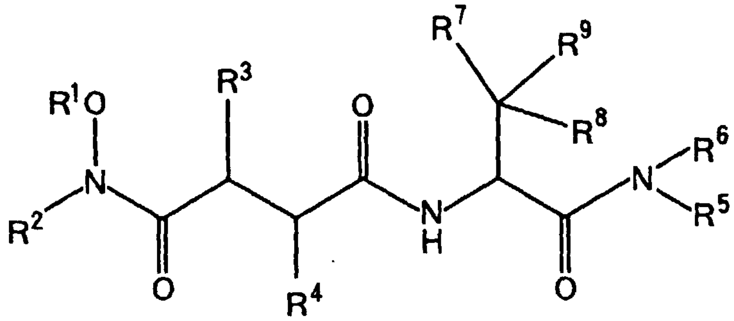

(I)

wherein $R^1$ is selected from the group consisting of hydrogen, unsubstituted or optionally substituted aralkyl, silyl which may optionally have three substituents, tetrahydropyranyl, unsubstituted or optionally substituted aralkyloxycarbonyl, unsubstituted or optionally substituted alkyloxycarbonyl, unsubstituted or optionally substituted alkyl, and a hydroxy-protecting group;
$R^2$ is selected from the group consisting of hydrogen, unsubstituted or optionally substituted aralkyloxycarbonyl, unsubstituted or optionally substituted alkyloxycarbonyl, 9-fluorenylmethyloxycarbonyl, and an amino-protecting group;
$R^3$ is selected from the group consisting of hydrogen, hydroxy, unsubstituted or optionally substituted alkyl, and unsubstituted or optionally substituted aralkyl;
$R^4$ is selected from the group consisting of unsubstituted or optionally substituted alkyl, and unsubstituted or optionally substituted aralkyl;
$R^5$ and $R^6$, which may be identical or different, are each independently selected from the group consisting of hydrogen, unsubstituted or optionally substituted alkyl, unsubstituted or optionally substituted cycloalkyl, an unsubstituted or optionally substituted heterocyclic group, and an amino-protecting group, or $R^5$ and $R^6$ taken together with the nitrogen atom to which they are attached form an unsubstituted or optionally substituted heterocyclic group;
$R^7$ is selected from the group consisting of hydrogen, hydroxy, unsubstituted or optionally substituted alkyl, and unsubstituted or optionally substituted aralkyl;
$R^8$ as selected from the group consisting of hydrogen, hydroxy, unsubstituted or optionally substituted alkyl, and unsubstituted or optionally substituted aralkyl; and
$R^9$ is selected from the group consisting of hydrogen, hydroxy, amino, and a group of the formula: -X-Y

wherein X is selected from the group consisting of unsubstituted or optionally substituted ($C_1$-$C_6$) alkylene, and unsubstituted or optionally substituted phenylene, and
Y is a group of the formula: -A-B or -B,
wherein A is selected from the group consisting of unsubstituted or optionally substituted ($C_1$ -$C_6$) alkylene, oxygen, sulfur, imino, and unsubstituted or optionally substituted ($C_1$-$C_6$) alkyleneimino, and
B is selected from the group consisting of hydrogen, amino, amidino, acylimidoyl, unsubstituted or optionally substituted imidazolyl, unprotected or optionally protected bis(phosphono)methyl, and unprotected or optionally protected bis(phosphono)hydroxymethyl;

or a pharmaceutically acceptable salt or solvate thereof;
(2) the compound according to the above (1) wherein

$R^1$ and $R^2$ are hydrogen;
$R^3$ is selected from the group consisting of ($C_1$-$C_9$) alkyl, ($C_3$-$C_7$) cycloalkyl-substituted lower ($C_1$-$C_4$) alkyl, hydroxy, amino-substituted ($C_1$-$C_6$) alkyl, phenyl-lower ($C_1$-$C_4$) alkyl, guanido-substituted phenyl-lower ($C_1$-$C_4$) alkyl, amino-substituted phenyl-lower ($C_1$-$C_4$) alkyl, carboxy-substituted phenyl-lower ($C_1$-$C_4$) alkyl, carbamoyl-substituted phenyl-lower ($C_1$-$C_4$) alkyl, hydroxy-substituted phenyl-lower ($C_1$-$C_4$) alkyl, guanido-substi-

tuted lower ($C_1$-$C_4$) alkyl-substituted phenyl-lower ($C_1$-$C_4$) alkyl, unprotected or optionally protected amino-substituted lower ($C_1$-$C_4$) alkyl-substituted phenyl-lower ($C_1$-$C_4$) alkyl, hydroxy-substituted lower ($C_1$-$C_4$) alkyl-substituted phenyl-lower ($C_1$-$C_4$) alkyl, lower ($C_1$-$C_4$) alkoxycarbonyl-substituted phenyl-lower ($C_1$-$C_4$) alkyl, lower ($C_1$-$C_4$) alkylimino-substituted ($C_1$-$C_6$) alkyl, lower ($C_1$-$C_4$) acylimidoylimino-substituted ($C_1$-$C_6$) alkyl, arylmethylimino-substituted ($C_1$-$C_6$) alkyl, nitrogen-containing heterocyclic radical-substituted lower ($C_1$-$C_4$) alkylimino-substituted ($C_1$-$C_6$) alkyl, nitrogen-containing heterocyclic radical-substituted lower ($C_1$-$C_4$) alkyl, oxygen-containing ($C_1$-$C_8$) straight chain or branched alkyl, arylsulfonamido-substituted lower ($C_1$-$C_4$) alkyl-substituted phenyl-lower ($C_1$-$C_4$) alkyl, alkylsulfonamido-substituted lower ($C_1$-$C_4$) alkyl-substituted phenyl-lower($C_1$-$C_4$) alkyl, aryloxy-substituted lower ($C_1$-$C_4$) alkyl, and hydroxy-substituted ($C_1$-$C_8$) alkyl;

$R^4$ is selected from the group consisting of ($C_3$-$C_9$) alkyl, hydroxy-substituted ($C_3$-$C_8$) alkyl, and unsubstituted or optionally substituted aryl-lower ($C_1$-$C_4$) alkyl;

$R^5$ is selected from the group consisting of lower ($C_1$-$C_4$) alkyl, ($C_3$-$C_7$) cycloalkyl, mono- or di-lower ($C_1$-$C_4$) alkylamino-substituted lower ($C_1$-$C_4$) alkyl, carboxy-substituted lower ($C_1$-$C_4$) alkyl, hydroxy-substituted ($C_1$-$C_6$) alkyl, bis(phosphono)hydroxymethyl-substituted ($C_1$-$C_{11}$) alkyl, tetrabenzyl bis(phosphono)hydroxymethyl-substituted ($C_1$-$C_{11}$) alkyl, and a nitrogen-containing heterocyclic radical;

$R^6$ is hydrogen;

$R^7$ is hydrogen or lower ($C_1$-$C_4$) alkyl;

$R^8$ is hydrogen or lower ($C_1$-$C_4$) alkyl; and

$R^9$ is selected from the group consisting of hydrogen, hydroxy, amino, and a group of the formula: -X-Y

wherein X is ($C_1$-$C_6$) alkylene or phenylene, and

y is a group of the formula: -A-B or -B

wherein B is selected from the group consisting of hydrogen, amino, amidino, lower ($C_1$-$C_4$) acylimidoyl, unsubstituted or optionally substituted benzimidoyl, bis(phosphono)methyl, tetra-lower ($C_1$-$C_4$) alkyl bis(phosphono)methyl, tri-lower ($C_1$-$C_4$) alkyl bis(phosphono)methyl, bis(phosphono)hydroxymethyl, tetrabenzyl bis(phosphono)hydroxymethyl, and lower ($C_1$-$C_4$) alkyl-substituted imidazol-3-yl, and

A is selected from the group consisting of lower ($C_1$-$C_4$) alkylene, imino, and lower ($C_1$-$C_4$) alkylene-imino;

(3) the compound according to the above (1) wherein

$R^1$ and $R^2$ are hydrogen;

$R^3$ is selected from the group consisting of hydroxy, methyl, isobutyl, aminopropyl, phenylpropyl, guanidophenylpropyl, aminophenylpropyl, hydroxyphenylpropyl, carboxyphenylpropyl, carbamoylphenylpropyl, aminomethylphenylpropyl, guanidomethylphenylpropyl, hydroxymethylphenylpropyl, aminomethylbenzyl, toluenesulfonamidomethylbenzyl, methanesulfonamidomethylbenzyl, isobutyliminomethylbenzyl, phthalimidomethylbenzyl, phenoxyethyl, aminopentyl, acetimidoyliminopentyl, isobutyliminopentyl, pyridylmethyliminopentyl, methoxycarbonylphenylpropyl, ethoxyethoxyethyl, hydroxyoctyl, butoxyethyl, iso-butyloxyethyl, morpholinopropyl, (3,4,4-trimethyl-2,5-dioxo-imidazolidin-1-yl)propyl, cyclohexylpropyl, and piperidinopropyl;

$R^4$ is selected from the group consisting of naphthylmethyl, phenylpropyl, isobutyl, tert-butyl, isopropyl, and hydroxyoctyl;

$R^5$ is selected from the group consisting of methyl, cyclopropyl, 2-(N,N-dimethylamino)ethyl, 2-carboxyethyl, 2-hydroxyethyl, 2-hydroxy-1,1-dimethylethyl, 2-hydroxy-1-methylethyl, 6,6-bis(phosphono)-6-hydroxyhexyl, tetrabenzyl 6,6-bis(phosphono)-6-hydroxyhexyl, piperidyl, and morpholinyl;

$R^6$ is hydrogen;

$R^7$ is hydrogen or methyl;

$R^8$ is hydrogen or methyl; and

$R^9$ is selected from the group consisting of hydrogen, hydroxy, amino, and a group of the formula: -X-Y

wherein X is selected from the group consisting of methylene, ethylene, trimethylene, tetramethylene, pentamethylene, hexamethylene, and phenylene, and

Y is a group of the formula: -A-B or -B

wherein B is selected from the group consisting of amino, amidino, acetimidoyl, propionimidoyl, benzimidoyl, bis(phosphono)methyl (i.e., bis(phosphono)-methylidine), tetraethyl bis(phosphono)methyl (i.e., tetra(ethyl) bis(phosphono)-methylidine), triethyl bis(phosphono)methyl (i.e., tri(ethyl) bis(phosphono)-methylidine), tetramethyl bis(phosphono)methyl (i.e., tetra(methyl) bis(phosphono)-methylidine), trimethyl bis(phosphono)methyl (i.e., tri(methyl) bis(phosphono)-methylidine), bis(phosphono)hydroxymethyl (i.e., bis(phosphono)(hydroxy)methylidine), tetrabenzyl bis(phosphono)hydroxymethyl (i.e., tetra(benzyl) bis(phosphono)(hydroxy)methylidine), and 2-methyl-imidazol-3-yl, and

A is selected from the group consisting of imino, methyleneimino, and methylene;

(4) a pharmaceutical or veterinary composition which comprises (a) an effective amount of at least a member selected from the group consisting of a compound of the formula (I) according to the above (1) and a pharmaceutically or veterinarily acceptable salt or solvate thereof, and (b) a pharmaceutically or veterinarily acceptable excipient or carrier;
(5) a metalloproteinase inhibitor which comprises an effective amount of at least a member selected from the group consisting of a compound of the formula (I) according to the above (1) and a pharmaceutically acceptable salt or solvate thereof;
(6) the inhibitor according to the above (5) wherein the metalloproteinase is selected from the group consisting of matrix metalloproteinases and the inhibitor is a kind of inhibitors of matrix metalloproteinase;
(7) the inhibitor according to the above (5) wherein the metalloproteinase is selected from the group consisting of tumor necrosis factor-$\alpha$ (TNF-$\alpha$ )-converting enzymes and the inhibitor is a kind of inhibitors of TNF- $\alpha$ convertase;
(8) use of a compound of the formula (I) according to the above (1) for prophylactically and/or therapeutically treating diseases and/or disorders associated with tissue degradation;
(9) a process for producing a compound having the formula (I) according to the above (1) or a pharmaceutically acceptable salt or solvate thereof, which comprises:

(a) converting an ester moiety of a compound of the formula (IV):

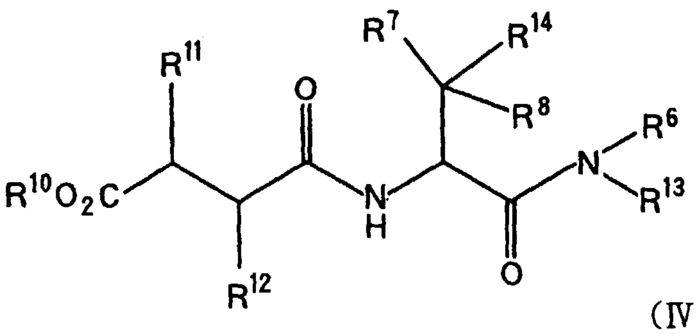

(IV)

into an amide bond-containing moiety thereof, and, if desired, further optionally converting $R^{11}$, $R^{12}$, $R^{13}$, and/or $R^{14}$ into the target functional group(s), $R^3$, $R^4$, $R^5$, and/or $R^9$, respectively, or
(b) if desired, optionally converting $R^{11}$, $R^{12}$, $R^{13}$, and/or $R^{14}$ in the compound of the formula (IV) into the target functional group(s), $R^3$, $R^4$, $R^5$, and/or $R^9$, respectively, and then converting an ester moiety thereof into an amide bond-containing moiety thereof,

wherein $R^1$ to $R^9$, all have the same meanings as defined in the above (1);
$R^{10}$ is selected from the group consisting of unsubstituted or optionally substituted alkyl, unsubstituted or optionally substituted aralkyl, and a carboxy-protecting group;
$R^{11}$ has the same meaning as defined for $R^3$, or is selected from the group consisting of protected hydroxy, protected guanido-substituted phenyl-lower ($C_1$-$C_4$) alkyl, protected amino-substituted phenyl-lower ($C_1$-$C_4$) alkyl, nitro-substituted phenyl-lower ($C_1$-$C_4$) alkyl, protected amino-substituted ($C_1$-$C_6$) alkyl, nitro-substituted ($C_1$-$C_6$) alkyl, protected carboxy-substituted phenyl-lower ($C_1$-$C_4$) alkyl, protected hydroxy-substituted phenyl-lower ($C_1$-$C_4$) alkyl, protected guanido-substituted lower ($C_1$-$C_4$) alkyl-substituted phenyl-lower ($C_1$-$C_4$) alkyl, protected amino-substituted lower ($C_1$-$C_4$) alkyl-substituted phenyl-lower ($C_1$-$C_4$) alkyl, protected hydroxy-substituted lower ($C_1$-$C_4$) alkyl-substituted phenyl-lower ($C_1$-$C_4$) alkyl, protected carboxy-substituted lower ($C_1$-$C_4$) alkyl-substituted phenyl-lower ($C_1$-$C_4$) alkyl, protected hydroxy-containing ($C_1$-$C_8$) straight chain or branched alkyl, and cyano-substituted phenyl-lower ($C_1$-$C_4$) alkyl;
$R^{12}$ has the same meaning as defined for $R^4$, or is protected hydroxy-substituted ($C_1$-$C_8$) alkyl;
$R^{13}$ has the same meaning as defined for $R^5$, or is selected from the group consisting of protected carboxy-substituted lower ($C_1$-$C_4$) alkyl, protected hydroxy-substituted lower ($C_1$-$C_4$) alkyl, protected bis(phosphono)hydroxymethyl-substituted ($C_1$-$C_{11}$) alkyl, and a protected nitrogen-containing heterocyclic group; and
$R^{14}$ has the same meaning as defined for $R^9$, or is selected from the group consisting of protected amino,

protected hydroxy, and a group of the formula: -X-E or -X-A-E

wherein X and A, both have the meanings as given above, and
E is selected from the group consisting of nitro, cyano, amino, carboxyl, ($C_1$-$C_{11}$) hydroxyalkyl, protected amino, protected guanido, protected amidino, protected acylimidoyl, protected benzimidoyl, protected bis(phosphono)methyl, protected bis(phosphono)hydroxymethyl, and protected ($C_1$-$C_{11}$) alkyl-substituted imidazol-3-yl;

(10) the process according to the above (9) wherein

$R^{10}$ is selected from the group consisting of ($C_1$-$C_6$) alkyl, benzyl, substituted benzyl, phenacyl, and 2,2,2-trichloroethyl;
$R^{11}$ has the same meaning as defined for $R^3$, or is selected from the group consisting of protected guanido-phenylpropyl, protected amino-phenylpropyl, nitro-phenylpropylene, protected aminopropyl, nitropropyl, protected hydroxy-phenylpropyl, protected carboxy-phenylpropyl, protected aminomethyl-phenylpropyl, protected guanidomethyl-phenylpropyl, protected hydroxymethyl-phenylpropyl, protected aminomethyl-benzyl, cyano-phenylpropyl, protected aminopentyl, cyano-benzyl, protected hydroxyoctyl, and nitropentyl;
$R^{12}$ has the same meaning as defined for $R^4$, or is protected hydroxyoctyl;
$R^{13}$ has the same meaning as defined for $R^5$, or is selected from the group consisting of protected 2-carboxyethyl, protected 2-hydroxyethyl, protected 2-hydroxy-1,1-dimethylethyl, protected 2-hydroxy-1-methylethyl, and protected 6,6-bis-phosphono-6-hydroxy-hexyl;
$R^{14}$ has the same meaning as defined for $R^9$, or is selected from the group consisting of protected amino, protected hydroxy, and a group of the formula: -X-F or -X-A-F

wherein X and A, both have the meanings as given above, and
F is selected from the group consisting of nitro, cyano, amino, carboxyl, hydroxymethyl, protected amino, protected guanido, protected amidino, protected acetimidoyl, protected propionimidoyl, protected benzimidoyl, protected bis(phosphono)methylimino, and protected bis(phosphono)hydroxymethyl;

(11) a process for producing a compound having the formula (I) according to the above (1) or a pharmaceutically acceptable salt or solvate thereof,
which comprises:

(a) reacting a hydroxamic acid backbone-containing succinic acid derivative of the formula (V):

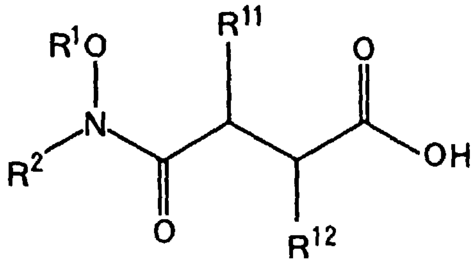

(V)

with an amine derivative of the formula (III):

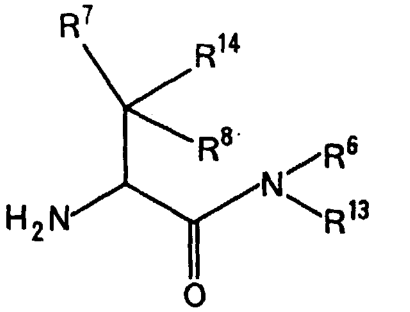

(III)

to form a compound of the formula (VI):

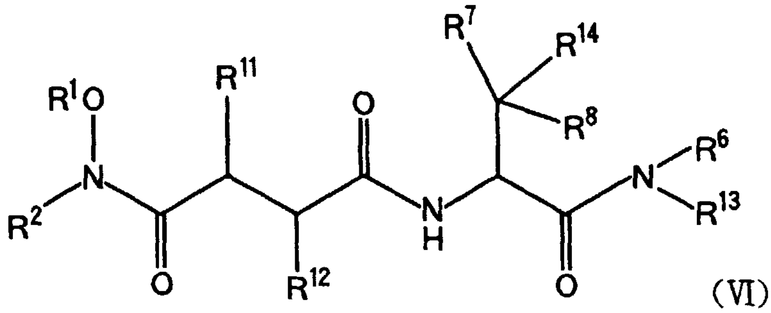

(VI)

and, if desired, optionally converting $R^{11}$ ,$R^{12}$, $R^{13}$ and/or $R^{14}$ into the target functional group(s), $R^3$, $R^4$, $R^5$ and/or $R^9$, respectively,

wherein $R^1$ to $R^9$, all have the same meanings as defined in the above (1), and $R^{11}$ to $R^{14}$, all have the same meanings as defined in the above (9);

(12) a compound having the following formula (VI):

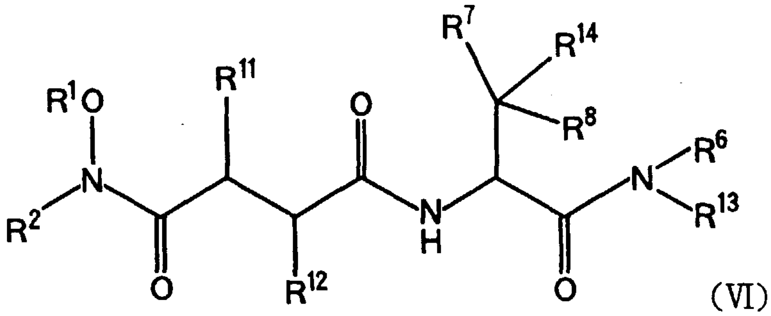

(VI)

wherein $R^1$, $R^2$, and $R^6$ to $R^8$, all have the same meanings as defined in the above (1), and $R^{11}$ to $R^{14}$, all have the same meanings as defined in the above (9), or a salt thereof;
(13) a compound having the following formula (IV):

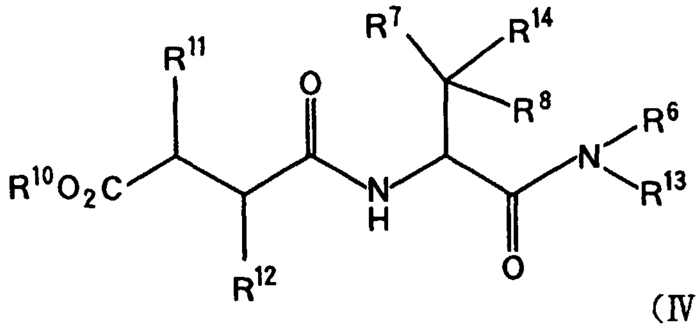

(IV)

wherein $R^6$ to $R^8$, all have the same meanings as defined in the above (1), and $R^{10}$ to $R^{14}$, all have the same meanings as defined in the above (9), or a salt thereof;

(14) a process for producing a compound having the formula (IV) according to the above (9) or a salt thereof, which comprises reacting a succinic acid derivative of the formula (II):

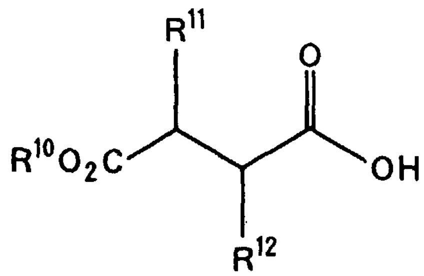

(II)

with an amine derivative of the formula (III):

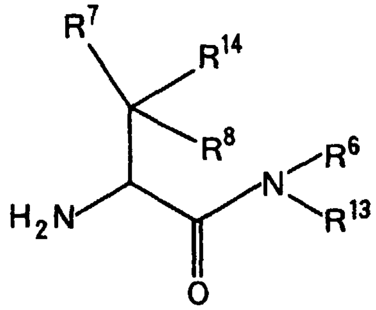

(III)

wherein $R^6$ to $R^8$, all have the same meanings as defined in the above (1), and $R^{10}$ to $R^{14}$, all have the same meanings as defined in the above (9); and

(15) a compound having the following formula (III):

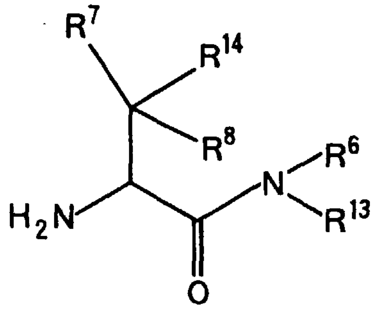

(III)

wherein $R^6$ to $R^8$, all have the same meanings as defined in the above (1), and $R^{13}$ and $R^{14}$, both have the same meanings as defined in the above (9),
or a pharmaceutically acceptable salt or solvate thereof.

In another aspect, the present invention provides:
(16) a compound having the formula (I)

wherein $R^1$ is hydrogen, or a hydroxy-protecting group;
$R^2$ is hydrogen, or an amino-protecting group;
$R^3$ is selected from the group consisting of hydrogen, hydroxy, unsubstituted or optionally substituted ($C_1$-$C_6$) alkyl, and unsubstituted or optionally substituted aryl-($C_1$-$C_6$) alkyl;
$R^4$ is selected from the group consisting of unsubstituted or optionally substituted ($C_1$-$C_6$) alkyl, and unsubstituted or optionally substituted aryl-($C_1$-$C_6$) alkyl;
$R^5$ and $R^6$, which may be identical or different, are each independently selected from the group consisting of hydrogen, unsubstituted or optionally substituted ($C_1$-$C_{11}$) alkyl, ($C_3$-$C_6$) cycloalkyl, and a heterocyclic radical;
$R^7$ is selected from the group consisting of hydrogen, hydroxy, unsubstituted or optionally substituted ($C_1$-$C_6$) alkyl, and unsubstituted or optionally substituted aryl-($C_1$-$C_6$) alkyl;
$R^8$ is selected from the group consisting of hydrogen, hydroxy, unsubstituted or optionally substituted ($C_1$-$C_6$) alkyl, and unsubstituted or optionally substituted aryl-($C_1$-$C_6$) alkyl;
$R^9$ is hydrogen, hydroxy, amino, or a group of the formula: -X-Y

wherein X is ($C_1$-$C_6$) alkylene, or phenylene; and Y is a group of the formula: -A-B or -B
wherein A is ($C_1$-$C_6$) alkylene, imino, or ($C_1$-$C_6$) alkylene-imino; and
B is selected from the group consisting of hydrogen, amino, amidino, acylimidoyl, unsubstituted or optionally substituted imidazolyl, unprotected or optionally protected bis(phosphono)methyl, and unprotected or optionally protected bis(phosphono)(hydroxy)methyl,

or a stereoisomer, pharmaceutically acceptable salt, or solvate thereof;
(17) the compound according to the above (16) wherein

$R^1$ and $R^2$ are hydrogen;
$R^3$ is selected from the group consisting of hydroxy, methyl, isobutyl, amino-propyl, 3-phenyl-propyl, p-guanidophenylpropyl, p-aminophenyl-propyl, p-hydroxyphenyl-propyl, p-carboxyphenyl-propyl, m-carboxyphenyl-propyl, p-aminomethylphenyl-propyl, p-guanidomethylphenyl-propyl, p-hydroxymethylphenyl-propyl, p-aminomethyl-benzyl, m-aminomethyl-benzyl, o-aminomethyl-benzyl, p-toluenesulfonamidomethyl-benzyl, p-methanesulfonamidomethyl-bentyl, p-isobutyliminomethyl-benzyl, p-phthalimidomethyl-benzyl, phenoxy-ethyl, o-aminomethylphenyl-propyl, amino-pentyl, acetimidoylimino-pentyl, isobutylimino-pentyl, (pyridin-4-yl)-methylimino-pentyl, p-methoxycarbonylphenyl-propyl, ethoxy-ethoxy-ethyl, 8-hydroxy-octyl, n-butoxy-ethyl, isobutyloxy-ethyl, m-methoxycarbonylphenyl-propyl, p-carbamoylphenyl-propyl, morpholino-propyl, and piperidino-propyl;
$R^4$ is selected from the group consisting of isobutyl, tert-butyl, isopropyl, and 8-hydroxy-octyl;
$R^5$ is selected from the group consisting of methyl, cyclo-propyl, 2-(N,N-dimethylamino)-ethyl, 2-carboxy-ethyl, 2-hydroxy-ethyl, 2-hydroxy-1,1-dimethyl-ethyl, 2-hydroxy-1-methyl-ethyl, 6,6-bis(phosphono)-6-hydroxy-hexyl, tetra(benzyl) 6,6-bis-(phosphono)-6-hydroxy-hexyl, piperidyl, and morpholinyl;
$R^6$ is hydrogen;
$R^7$ is hydrogen or methyl;

$R^8$ is hydrogen or methyl; and

$R^9$ is selected from the group consisting of hydrogen, hydroxy, amino, and a group of the formula: -X-Y

X is selected from the group consisting of methylene, ethylene, trimethylene, tetramethylene, pentamethylene, hexamethylene, and phenylene, and

B for Y is selected from the group consisting of amino, amidino, acetimidoyl, propionimidoyl, benzimidoyl, bis(phosphono)-methyl, tetra(ethyl) bis(phosphono)-methyl, tri(ethyl) bis(phosphono)-methyl, tetra(methyl) bis(phosphono)-methyl, tri(methyl) bis(phosphono)-methyl, bis(phosphono)-(hydroxy)-methyl, tetra(benzyl) bis(phosphono)-(hydroxy)-methyl, and 2-methyl-imidazol-3-yl, and,

when Y = -A-B ,

A is selected from the group consisting of imino, methyleneimino, and methylene;

(18) a metalloproteinase inhibitor which comprises an effective amount of one or more members selected from the group consisting of compounds of the formula (I) as given above and stereoisomers, pharmaceutically acceptable salts, and solvates thereof for inhibiting the activity of matrix metalloproteinases (MMPs) and/or tumor necrosis factor- α (TNF-α )-converting enzymes (TNF-α convertases);

(19) use of a metalloproteinase inhibitor comprising a compound of the formula (I) as given above and having the property of inhibiting the activity of matrix metalloproteinases (MMPs) and/or tumor necrosis factor-α (TNF-α )-converting enzymes (TNF-α convertases) for prophylactically and/or therapeutically treating diseases and/or disorders associated with the degradation of tissues;

(20) a process for producing a compound of the formula (I) as given above or a stereoisomer, pharmaceutically acceptable salt or solvate thereof, which comprises:

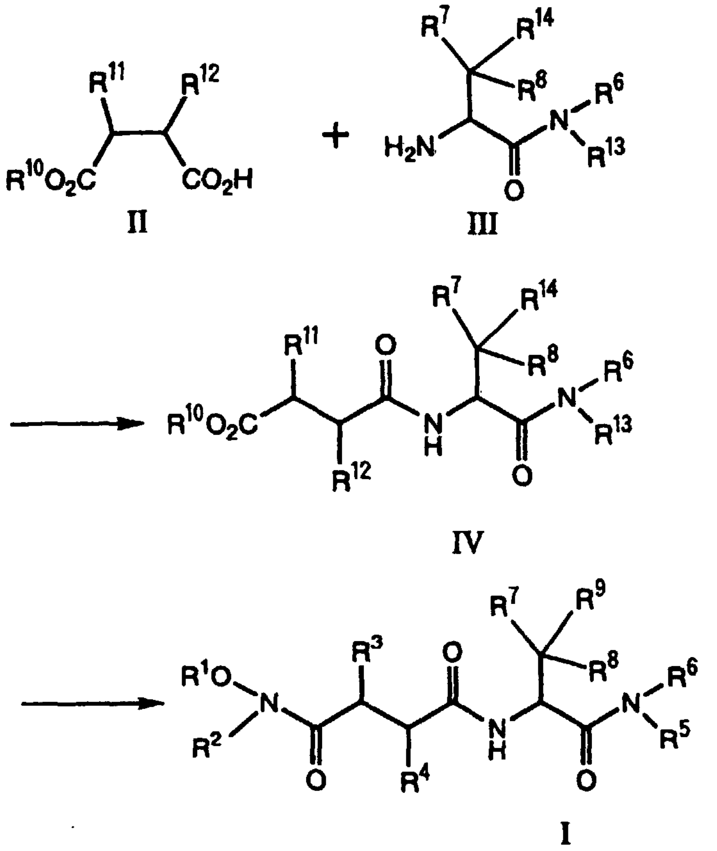

II III

IV

I

(i) reacting a succinic acid derivative of the formula (II) with an amine derivative of the formula (III) to form an ester compound of the formula (IV), if desired, then optionally converting $R^{11}$, $R^{12}$, $R^{13}$ and/or $R^{14}$ into the target functional group(s), $R^3$, $R^4$, $R^5$ and/or $R^9$, respectively, and converting the ester moiety into an amide moiety; or

(ii) reacting a succinic acid derivative of the formula (II) with an amine derivative of the formula (III) to form an ester compound of the formula (IV), then converting the ester moiety into an amide moiety, and if desired, optionally converting $R^{11}$, $R^{12}$, $R^{13}$ and/or $R^{14}$ into the target functional group(s), $R^3$, $R^4$, $R^5$ and/or $R^9$, respectively,

wherein $R^1$ to $R^9$,all have the meanings as given above;
$R^{10}$ is a carboxy-protecting group (ex., ($C_1$-$C_6$) alkyl, benzyl, substituted benzyl, phenacyl, or 2,2,2-trichloroethyl);
$R^{11}$ has the same meaning as defined for $R^3$, or is selected from the group consisting of protected p-guanidophenyl-propyl, protected p-guanidomethylphenyl-propyl, protected p-aminomethylphenyl-propyl, protected p-aminomethyl-benzyl, protected o-aminomethyl-benzyl, protected m-aminomethyl-benzyl, protected aminopentyl, nitro-pentyl, protected hydroxy-octyl, protected p-hydroxyphenyl-propyl, protected p-aminophenyl-propyl, nitrophenyl-propylene, protected amino-propyl, and nitro-propyl;
$R^{12}$ has the same meaning as defined for $R^4$, or is protected hydroxyoctyl;
$R^{13}$ has the same meaning as defined for $R^5$, or is selected from the group consisting of protected 2-carboxyethyl, protected 2-hydroxyethyl, protected 2-hydroxy-1,1-dimethylethyl, protected 2-hydroxy-1-methylethyl, and protected 6,6-bis-phosphono-6-hydroxy-hexyl;
$R^{14}$ has the same meaning as defined for $R^9$, or is a group of the formula: -X-G or -X-A-G

wherein X and A, both have the meanings as given above, and
G is selected from the group consisting of nitro, cyano, amino, carboxyl, hydroxymethyl, protected amino, protected guanido, protected amidino, protected acetimidoyl, protected propionimidoyl, protected benzimidoyl, protected bis(phosphono)methyl, and protected bis(phosphono)hydroxymethyl; and

(21) a process for producing a compound of the formula (I) as given above or a stereoisomer, pharmaceutically acceptable salt or solvate thereof, which comprises:

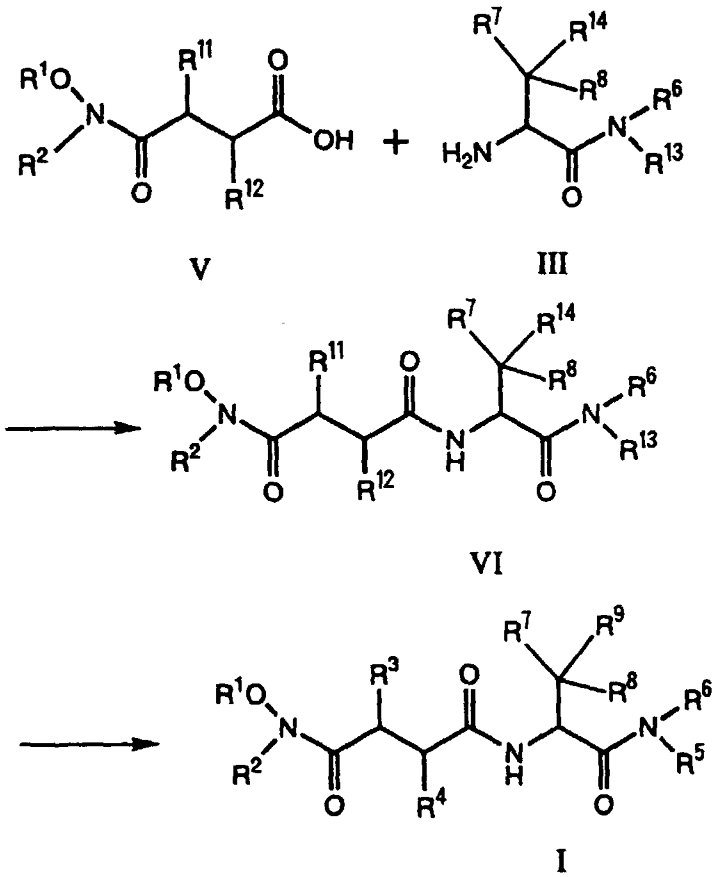

V

III

VI

I

reacting a protected hydroxamic acid-based succinic acid derivative of the formula (V) with an amine derivative of the formula (III) to form a protected precursor of the formula (VI), if desired, then optionally converting $R^{11}$, $R^{12}$, $R^{13}$ and/or $R^{14}$ into the target functional group(s), $R^3$, $R^4$, $R^5$ and/or $R^9$, respectively,
wherein $R^1$ to $R^9$, and $R^{11}$ to $R^{14}$, all have the meanings as given above.

It is apparent that there are chiral carbon atoms designated with * in the compounds of the formula (I), as illustrated in the following formula:

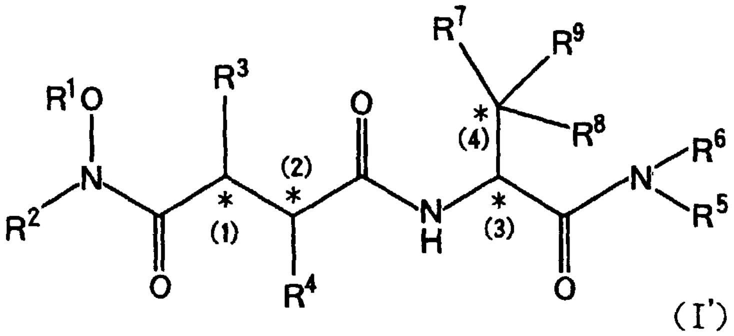

(I')

Thus, it should be understood that the compounds given in the above (1) may include not only geometric isomers, stereoisomers, each optically active isomer, racemates, and tautomers thereof, but also metabolite derivatives thereof. All such compounds, isomers, racemates, tautomers and derivatives thereof are intended to be within the scope of the present invention.

In a preferred embodiment of the present invention, the chiral carbon atoms designated with * in the above compounds (I') include the carbon atoms (1) and (4) in the "R" or "S" configuration, the carbon atom (2) in the "S" configuration, and the carbon atom (3) in the "S" configuration.

In a further aspect, the present invention provides:

(22) a compound having the formula (I-1):

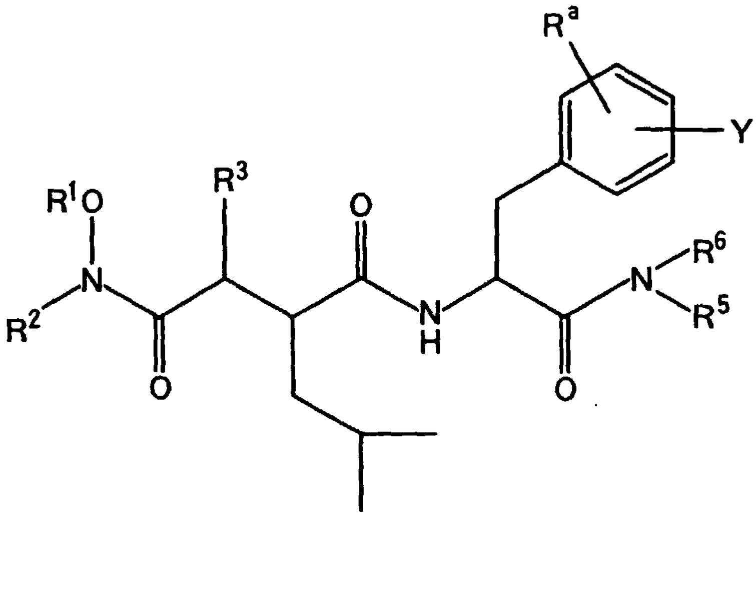

(I−1)

wherein $R^1$ to $R^3$, $R^5$, $R^6$ and Y, all have the same meanings as defined in the above (1); among them,
$R^3$ is preferably hydrogen, hydroxy, or unsubstituted or optionally substituted aryl-($C_1$-$C_6$) alkyl;
when Y is a group of the formula; -A-B,
-A- is most preferably ($C_1$-$C_6$) alkylene-imino, or imino;

-B is most preferably hydrogen, acetimidoyl, propionimidoyl, benzimidoyl, amidino, or bis(phosphono)-methyl;
when Y is a group of the formula: -B,
-B is most preferably acetimidoyl, propionimidoyl, benzimidoyl, or amidino;
$R^a$ is hydrogen, or has the same meaning as set forth for the substituent(s) on the "unsubstituted or optionally substituted phenylene" with regard to X in the above (1), or a pharmaceutically acceptable salt or solvate thereof;

(23) a pharmaceutical or veterinary composition which comprises (a) an effective amount of at least a member selected from the group consisting of a compound of the formula (I-1) according to the above (22) and a pharmaceutically or veterinarily acceptable salt or solvate thereof, and (b) a pharmaceutically or veterinarily acceptable excipient or carrier;
(24) a metalloproteinase inhibitor which comprises an effective amount of at least a member selected from the group consisting of a compound of the formula (I-1) according to the above (22) and a pharmaceutically or veterinarily acceptable salt or solvate thereof;
(25) the inhibitor according to the above (24) wherein the metalloproteinase is selected from the group consisting of matrix metalloproteinases and the inhibitor is a kind of inhibitors of matrix metalloproteinase;
(26) the inhibitor according to the above (24) wherein the metalloproteinase is selected from the group consisting of tumor necrosis factor-α (TNF-α)-converting enzymes and the inhibitor is a kind of inhibitors of TNF-α convertase; and
(27) use of a compound of the formula (I-1) according to the above (24) for prophylactically and/or therapeutically treating diseases and/or disorders associated with tissue degradation.

In still another aspect, the present invention provides:
(28) a compound having the formula (I-2):

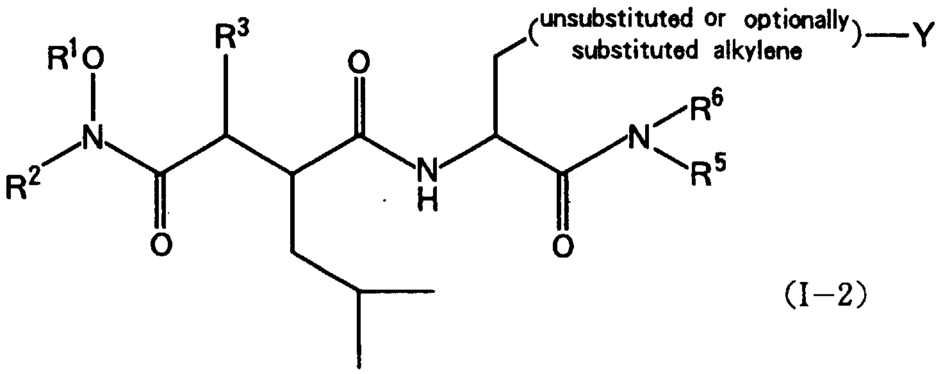

(I−2)

wherein $R^1$ to $R^3$, $R^5$, $R^6$ and Y, all have the same meanings as defined in the above (1);
the substituent on the "(unsubstituted or optionally substituted alkylene)" has the same meaning as set forth for the substituent(s) on the "unsubstituted or optionally substituted alkylene" with regard to X in the above (1), or a pharmaceutically acceptable salt or solvate thereof;

(29) a pharmaceutical or veterinary composition which comprises (a) an effective amount of at least a member selected from the group consisting of a compound of the formula (I-2) according to the above (28) and a pharmaceutically or veterinarily acceptable salt or solvate thereof, and (b) a pharmaceutically or veterinarily acceptable excipient or carrier;
(30) a metalloproteinase inhibitor which comprises an effective amount of at least a member selected from the group consisting of a compound of the formula (I-2) according to the above (28) and a pharmaceutically or veterinarily acceptable salt or solvate thereof;
(31) the inhibitor according to the above (30) wherein the metalloproteinase is selected from the group consisting of matrix metalloproteinases and the inhibitor is a kind of inhibitors of matrix metalloproteinase;
(32) the inhibitor according to the above (30) wherein the metalloproteinase is selected from the group consisting of tumor necrosis factor-α (TNF-α)-converting enzymes and the inhibitor is a kind of inhibitors of TNF-α convertase;

and

(33) use of a compound of the formula (I-2) according to the above (28) for prophylactically and/or therapeutically treating diseases and/or disorders associated with tissue degradation.

In yet another aspect, the present invention provides:

(34) the compound of the formula (III) according to the above (15) or a pharmaceutically acceptable salt or solvate thereof, wherein

$R^6$, $R^7$, and $R^8$, all have the same meanings as defined in the above (1);

$R^{13}$ is selected from the group consisting of hydrogen, unsubstituted or optionally substituted alkyl, unsubstituted or optionally substituted cycloalkyl, an unsubstituted or optionally substituted heterocyclic group, or an amino-protecting group, or taken together with $R^6$ and the nitrogen atom to which they are attached form an unsubstituted or optionally substituted heterocyclic group; and

$R^{14}$ is selected from the group consisting of hydroxy, amino, and a group of the formula: -X-G or -X-A-G

wherein X has the meaning as set forth in the above (1),

and A is selected from the group consisting of unsubstituted or optionally substituted ($C_1$-$C_6$) alkylene, oxygen, imino, and unsubstituted or optionally substituted ($C_1$-$C_6$) alkyleneimino, and G is unprotected or optionally protected bis(phosphono)methyl or bis(phosphono)(hydroxy)-methyl;

(35) a process for producing a compound of the formula (III) according to the above (34) or a pharmaceutically acceptable salt or solvate thereof;

(36) a pharmaceutical or veterinary composition which comprises (a) an effective amount of at least a member selected from the group consisting of a compound of the formula (III) according to the above (34) and a pharmaceutically or veterinarily acceptable salt or solvate thereof, and (b) a pharmaceutically or veterinarily acceptable excipient or carrier;

(37) an inhibitor of the destruction of bones comprising an effective amount of at least a member selected from the group consisting of a compound of the formula (III) according to the above (34) and a pharmaceutically or veterinarily acceptable salt or solvate thereof;

(38) an agent for the prophylaxis and/or treatment of osteoporosis comprising an effective amount of at least a member selected from the group consisting of a compound of the formula (III) according to the above (34) and a pharmaceutically or veterinarily acceptable salt or solvate thereof; and

(39) use of a compound of the formula (III) according to the above (34) for prophylactically and/or therapeutically treating diseases and/or disorders associated with the degradation of bone tissues.

The above objects and other objects, features, advantages, and aspects of the present invention are readily apparent to those skilled in the art from the following disclosures. It should be understood, however, that the description of the specification including the following best modes of carrying out the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

The term "and/or" used herein means the presence of both (1) a jointly connecting relation and (2) a selectively connecting relation. For example, in the case of "prophylactically and/or therapeutically", it is used in such a sense that said expression covers both (1) "prophylactically and therapeutically" and (2) "prophylactically or therapeutically". In other cases, the term "and/or" is used in the same sense that it covers both (1) a jointly connecting relation and (2) a selectively connecting relation as well.

<u>Best Modes of Carrying out the Invention</u>

As used herein for $R^1$, $R^3$, $R^4$, $R^5$, $R^6$, $R^7$, $R^8$ and $R^{10}$ in the compounds given above, specifically the compounds (I), (IV), and (VI), the term "unsubstituted or optionally substituted alkyl" refers to a straight chain or branched alkyl moiety, preferably having from 1 to 20 carbon atoms, more preferably from 1 to 12 carbon atoms, and most preferably from 1 to 9 carbon atoms, which can optionally be unsubstituted or substituted with one or more substituents which can be selected from the group given hereinbelow, including for example, methyl, ethyl, propyl, isopropyl, butyl, isobutyl, sec-butyl, tert-butyl, n-pentyl, isopentyl, neopentyl, hexyl, heptyl, and octyl.

As used herein for $R^1$, $R^3$, $R^4$, $R^7$, $R^8$ and $R^{10}$ in the compounds given above, specifically the compounds (I), (IV), and (VI), the term "unsubstituted or optionally substituted aralkyl" refers to a group having (1) an aryl moiety,

preferably having from 6 to 10 carbon atoms, and more preferably 6 carbon atoms, and (2) a straight chain or branched alkylene moiety, preferably having from 1 to 8 carbon atoms, and more preferably from 1 to 4 carbon atoms, said aryl moiety and/or said alkylene moiety which can optionally be unsubstituted or substituted with one or more substituents which can be selected from the group given hereinbelow. Representatives of said "aralkyl" include for example unsubstituted or optionally substituted phenyl-substituted lower ($C_1$-$C_4$) alkyl (also termed "phenyl-lower ($C_1$-$C_4$) alkylene"). Examples of said aralkyl are unsubstituted or optionally substituted benzyl such as benzyl, 2- or 4-nitrobenzyl and 4-methoxybenzyl, unsubstituted or optionally substituted phenethyl, unsubstituted or optionally substituted phenylpropyl (also termed "phenyl-trimethylene"), unsubstituted or optionally substituted naphthylpropyl (also termed "naphthyl-trimethylene") and the like.

As used herein for $R^1$ and $R^2$ in connection with the compounds (I), the term "unsubstituted or optionally substituted alkyloxycarbonyl" refers to a group having the "unsubstituted or optionally substituted alkyl moiety" as set forth for said "unsubstituted or optionally substituted alkyl" in connection with $R^1$, $R^3$, $R^4$, $R^5$, $R^6$, $R^7$, $R^8$ or $R^{10}$. Said unsubstituted or optionally substituted alkyloxycarbonyl includes for example ($C_1$-$C_6$) alkyloxycarbonyl such as tert-butyloxycarbonyl.

As used herein for $R^1$ and $R^2$ in connection with the compounds (I), the term "unsubstituted or optionally substituted aralkyloxycarbonyl" refers to a group having the "unsubstituted or optionally substituted aralkyl moiety" as set forth for said "unsubstituted or optionally substituted aralkyl". Said "unsubstituted or optionally substituted aralkyloxycarbonyl" includes for example, unsubstituted or optionally substituted benzyloxycarbonyl, such as benzyloxycarbonyl, 2- or 4-nitro-benzyloxycarbonyl, and 4-methoxybenzyloxycarbonyl, unsubstituted or optionally substituted phenethyloxycarbonyl, and the like.

As used herein for $R^1$ in the above compounds (I), the term "silyl which may optionally have three substituents" refers to those silyl groups optionally having, as the substituent(s), unsubstituted or optionally substituted alkyl, and/or unsubstituted or optionally substituted aryl, and/or unsubstituted or optionally substituted aralkyl, wherein the "unsubstituted or optionally substituted aralkyl" as said substituent has the meaning as given above, the "unsubstituted or optionally substituted alkyl" has similarly the same meaning as set forth for the "unsubstituted or optionally substituted alkyl" in connection with $R^1$, $R^3$, $R^4$, $R^5$, $R^6$, $R^7$, $R^8$ and $R^{10}$, and the "unsubstituted or optionally substituted aryl" as said substituent also has the same meaning as set forth for the "aryl moiety" of the "unsubstituted or optionally substituted aralkyl" in connection with $R^1$, $R^3$, $R^4$, $R^7$, $R^8$ and $R^{10}$, including for example, phenyl, naphthyl, etc. Representatives of the "silyl which may optionally have three substituents" are trialkylsilyl such as trimethylsilyl, and tert-butyldimethylsilyl, alkyldiarylsilyl such as tert-butyldiphenylsilyl, and the like.

As used herein for $R^5$ and $R^6$ in connection with the compounds (I), the term "unsubstituted or optionally substituted cycloalkyl" refers to a radical being monocyclic or having multiple condensed rings, such as a bicyclic radical, preferably from 3 to 10, more preferably from 3 to 7, and most preferably from 3 to 6 carbon atoms, which can optionally be unsubstituted or substituted with one or more substituents (said substituent is selected from those given hereinbelow), including for example, cyclopropyl, cyclobutyl, cyclopentyl, cyclohexyl, cycloheptyl, [2.2.1]bicycloheptyl, etc.

As used herein for $R^5$ and $R^6$ in connection with the compounds (I), the term "unsubstituted or optionally substituted heterocyclic group" refers to a suturated or unsaturated radical, being monocyclic or having multiple condensed rings, such as a bicyclic radical, containing one or more hetero atoms (which are identical or different) selected from nitrogen, oxygen, sulfur, etc., which can optionally be unsubstituted or substituted with one or more substituents (said substituent is selected from those given hereinbelow). Said unsubstituted or optionally substituted heterocyclic group includes for example, radicals having a 5-, 6- or 7-membered heterocyclic ring. Representatives of the "heterocyclic ring" include imidazole, pyrazole, imidazoline, imidazolidine, pyridine, pyrimidine, benzimidazole, quinazoline, pteridine, purine, 1,3-azepine, aziridine, azetidine, pyrrole, pyrrolidine, tetrahydropyridine, piperidine, azepine, indole, quinoline, isoquinoline, morpholine, piperazine, etc.

When $R^5$ and $R^6$ taken together with the nitrogen atom to which they are attached form an "unsubstituted or optionally substituted heterocyclic group", said term "unsubstituted or optionally substituted heterocyclic group" refers to a suturated or unsaturated nitrogen-containing radical, being monocyclic or having multiple condensed rings, such as a bicyclic radical, wherein said heterocycle includes, for example, aziridine, azetidine, pyrrole, pyrrolidine, pyridine, tetrahydropyridine, piperidine, azepine, indole, quinoline, isoquinoline, morpholine, piperazine, etc.

As used herein for the term "hydroxy-protecting group" in connection with $R^1$ and the term "protected hydroxy" in connection with $R^{11}$ and $R^{14}$ in the compounds (I) and (IV), suitable protecting groups are those known to artisans in the organic synthesis fields, for example, selected from those which have been employed in the technical fields including peptide synthesis, penicillin synthesis, cephalosporin synthesis, sugar synthesis, and the like. Said "hydroxy-protecting groups" and said "protecting groups" include those removable by treatment with water, those removable by hydrogenolysis, those removable with Lewis catalysts such as $AlCl_3$, those removable with zinc/acetic acid, those removable with thiourea, those removable with acids or weak bases, and the like. Representative hydroxy-protecting groups include benzyl, 2,2,2-trichloroethoxycarbonyl, allyloxycarbonyl, 2-methoxyethoxymethyl, formyl,

acetyl, chloroacetyl, dichloroacetyl, trityl, and the like. Such groups are any as long as they are capable of forming, or convertible into, a hydroxy group, chemically or under biological conditions, i.e., physiological conditions (for example, bioreactions such as oxidation, reduction, and hydrolysis, catalyzed by in vivo enzymes and the like). Said "hydroxy-protecting groups" and said "protecting groups" may also be selected from the group consisting of the above defined "silyl which may optionally have three substituents", "unsubstituted or optionally substituted aralkyl", tetrahydropyranyl, "unsubstituted or optionally substituted alkyloxycarbonyl", "unsubstituted or optionally substituted aralkyloxycarbonyl", and the like.

As used herein for $R^2$, $R^5$, $R^6$, $R^{11}$, $R^{14}$ and F in the compounds (I) and (IV), the term "amino-protecting group" refers to protecting groups known to artisans in the organic synthesis fields, for example, selected from those which have been employed in the technical fields including peptide synthesis, penicillin synthesis, cephalosporin synthesis, sugar synthesis, and the like. Said "amino-protecting groups" include those removable by hydrogenolysis, those removable with fluorinated compounds, those removable with acids, and the like. Illustrative examples of such "amino-protecting groups" include benzyloxycarbonyl, p-nitro-benzyloxycarbonyl, trityl, tert-butoxycarbonyl; ($C_1$-$C_6$) aliphatic acyl which can optionally be unsubstituted or substituted with halogen, such as formyl, chloroacetyl, and dichloroacetyl; alkoxycarbonyl such as allyloxycarbonyl, 2-trimethylsilylethoxycarbonyl, and 2,2,2-trichloroethoxycarbonyl; 2-methoxyethoxymethyl, and the like. Such groups are any as long as they are capable of forming, or convertible into, a free or protonated amino group, chemically or under biological conditions, i.e., physiological conditions (for example, bioreactions such as oxidation, reduction, and hydrolysis, catalyzed by in vivo enzymes and the like). Said "amino-protecting groups" may also be selected from the group consisting of the above defined "unsubstituted or optionally substituted alkyloxycarbonyl", "unsubstituted or optionally substituted aralkyloxycarbonyl", 9-fluorenylmethyloxycarbonyl, and the like.

In connection with the above defined "unsubstituted or optionally substituted alkyl", "unsubstituted or optionally substituted aralkyl", "unsubstituted or optionally substituted aryl", "unsubstituted or optionally substituted cycloalkyl", and "unsubstituted or optionally substituted heterocyclic group", suitable substituents include the above defined alkyl, the above defined aryl, hydroxy, unsubstituted or optionally substituted amino (for example, amino, N-lower ($C_1$-$C_4$) alkylamino such as methylamino, ethylamino and isopropylamino, N-arylamino such as phenylamino, aralkylamino such as pyridylmethylamino and benzylamino, alicyclic amino such as morpholino, piperidino, piperazino and N-phenyl-piperazino, guanidino, and the like), amidino, acylimidoyl (for example, derivatives from ($C_2$-$C_5$) lower alkanoic acids, such as acetimidoyl and propionimidoyl, derivatives from ($C_7$-$C_{11}$) aromatic carboxylic acids, such as benzimidoyl, and the like), halogen (for example, F, Cl, Br, etc.), nitro, ($C_1$-$C_4$) lower alkoxy (for example, methoxy, ethoxy, etc.), ($C_1$-$C_4$) lower alkylthio (for example, methylthio, ethylthio, etc.), carboxyl, ($C_2$-$C_6$) lower alkanoyloxy, ($C_1$-$C_6$) lower alkoxycarbonyloxy, phosphono which can optionally be unprotected or protected at its hydroxy wherein said protecting group includes the above defined "hydroxy-protecting group", "unsubstituted or optionally substituted alkyl", "unsubstituted or optionally substituted aralkyl", "unsubstituted or optionally substituted aryl", "unsubstituted or optionally substituted alkyloxycarbonyl", "unsubstituted or optionally substituted aralkyloxycarbonyl", and the like.

As used herein for X, A and B in the above compounds, particularly the compounds (I) and (IV), in connection with the "unsubstituted or optionally substituted ($C_1$-$C_6$) alkylene", the "unsubstituted or optionally substituted phenylene", the "unsubstituted or optionally substituted ($C_1$-$C_6$) alkyleneimino", and the "unsubstituted or optionally substituted imidazolyl", suitable substituents may be selected from those listed for the above defined substituent in the "unsubstituted or optionally substituted alkyl", etc.

As used herein for B in the above compounds, particularly the compounds (I) and (IV), in connection with the "unprotected or optionally protected bis(phosphono)methyl", and the "unprotected or optionally protected bis(phosphono)hydroxymethyl", suitable protecting groups may include the above defined "hydroxy-protecting group", "unsubstituted or optionally substituted alkyl", "unsubstituted or optionally substituted aralkyl", "unsubstituted or optionally substituted aryl", "unsubstituted or optionally substituted alkyloxycarbonyl", and "unsubstituted or optionally substituted aralkyloxycarbonyl"

As used herein for B and E in the above compounds, particularly the compounds (I) and (IV), the "acylimidoyl" includes a radical derived from a ($C_2$-$C_5$) lower alkanoic acid, such as acetimidoyl and propionimidoyl; a radical derived from a ($C_7$-$C_{11}$) aromatic carboxylic acid, such as benzimidoyl; and the like, as described hereinabove.

As used herein for $R^{10}$ in the above compounds, particularly the compounds (IV), the "carboxy-protecting group" may be selected from protecting groups known to artisans in the organic synthesis fields, for example, those which have been employed in the technical fields including peptide synthesis, penicillin synthesis, cephalosporin synthesis, sugar synthesis, and the like. Said "carboxy-protecting groups" have the same meanings as defined for the above "hydroxy-protecting group", including those removable by hydrogenolysis, those removable by treatment with acids or weak bases, and the like. Illustrative examples of such "carboxy-protecting groups" include the above defined "unsubstituted or optionally substituted alkyl", "unsubstituted or optionally substituted aralkyl", "unsubstituted or optionally substituted aryl", "unsubstituted or optionally substituted alkyloxycarbonyl", "unsubstituted or optionally substituted aralkyloxycarbonyl", and the like.

As used herein for $R^{11}$, $R^{12}$, $R^{13}$, $R^{14}$, E and F in the above compounds, particularly the compounds (IV), in connection with the "protected hydroxy", "protected guanido-substituted phenyl-lower ($C_1$-$C_4$) alkyl", "protected amino-substituted phenyl-lower ($C_1$-$C_4$) alkyl", "protected amino-substituted ($C_1$-$C_6$) alkyl", "protected carboxy-substituted phenyl-lower ($C_1$-$C_4$) alkyl", "protected hydroxy-substituted phenyl-lower ($C_1$-$C_4$) alkyl", "protected guanido-substituted lower ($C_1$-$C_4$) alkyl-substituted phenyl-lower ($C_1$-$C_4$ ) alkyl", "protected amino-substituted lower ($C_1$-$C_4$) alkyl-substituted phenyl-lower ($C_1$-$C_4$) alkyl", "protected hydroxy-substituted lower ($C_1$-$C_4$) alkyl-substituted phenyl-lower ($C_1$-$C_4$) alkyl", "protected carboxy-substituted lower ($C_1$-$C_4$) alkyl-substituted phenyl-lower ($C_1$-$C_4$) alkyl", "protected hydroxy-containing ($C_1$-$C_8$) straight chain or branched alkyl", "protected hydroxy-substituted ($C_1$-$C_8$) alkyl", "protected carboxy-substituted lower ($C_1$-$C_4$) alkyl", "protected hydroxy-substituted lower ($C_1$-$C_4$) alkyl", "protected bis(phosphono)hydroxymethyl-substituted ($C_1$-$C_{11}$) alkyl", "protected nitrogen-containing heterocyclic group", "protected amino", "protected guanido", "protected amidino", "protected acylimidoyl", "protected benzimidoyl", "protected bis(phosphono)methyl", "protected bis(phosphono)hydroxymethyl", and "protected ($C_1$-$C_{11}$) alkyl-substituted imidazol-3-yl", suitable protecting groups may include the above defined "hydroxy-protecting group", "amino-protecting group", and "carboxy-protecting group".

The compounds (I) of the present invention may exist as alternative tautomers. There are several chiral centers in the compounds (I) of the present invention because of the presence of asymmetric carbon atoms. The presence of several asymmetric carbon atoms gives rise to a number of optical isomers with regard to each chiral center. All mixtures of such isomers and each individual optical isomer are intended to be within the scope of the present invention. The compounds of the present invention can also exist as separate enantiomers, as racemates, or as diastereomers. The compounds of the present invention can also be in the form of solvates or acid-addition salts. Further, the compounds of the present invention may be in the form of prodrugs, including those prodrugs of (i) compounds containing a carboxyl radical and/or a hydroxy radical and/or an optionally substituted or unsubstituted amino radical or (ii) derivatives thereof. The prodrugs of the compounds according to the present invention include those compounds which can be transformed in vivo, for example by metabolic processes, including hydrolysis, oxidation, reduction, trans-esterification and the like, to yield the parent compounds of the formula (I), etc. Representatives of such prodrugs are ester-, ether-, amide-, alcohol-, and amine-derivatives thereof.

The aforementioned compounds (I) according to the present invention and intermediates for the production thereof can be prepared according to the procedures illustrated in Reaction Schemes hereinbelow. In the disclosures given below, each compound is assigned a specific serial number in the same manner as conventionally employed in a number of chemical literatures and denoted by such a serial number.

The first disclosure illustrates hereinbelow the preparation of compounds of the formula (II) which are useful intermediates for the production thereof.

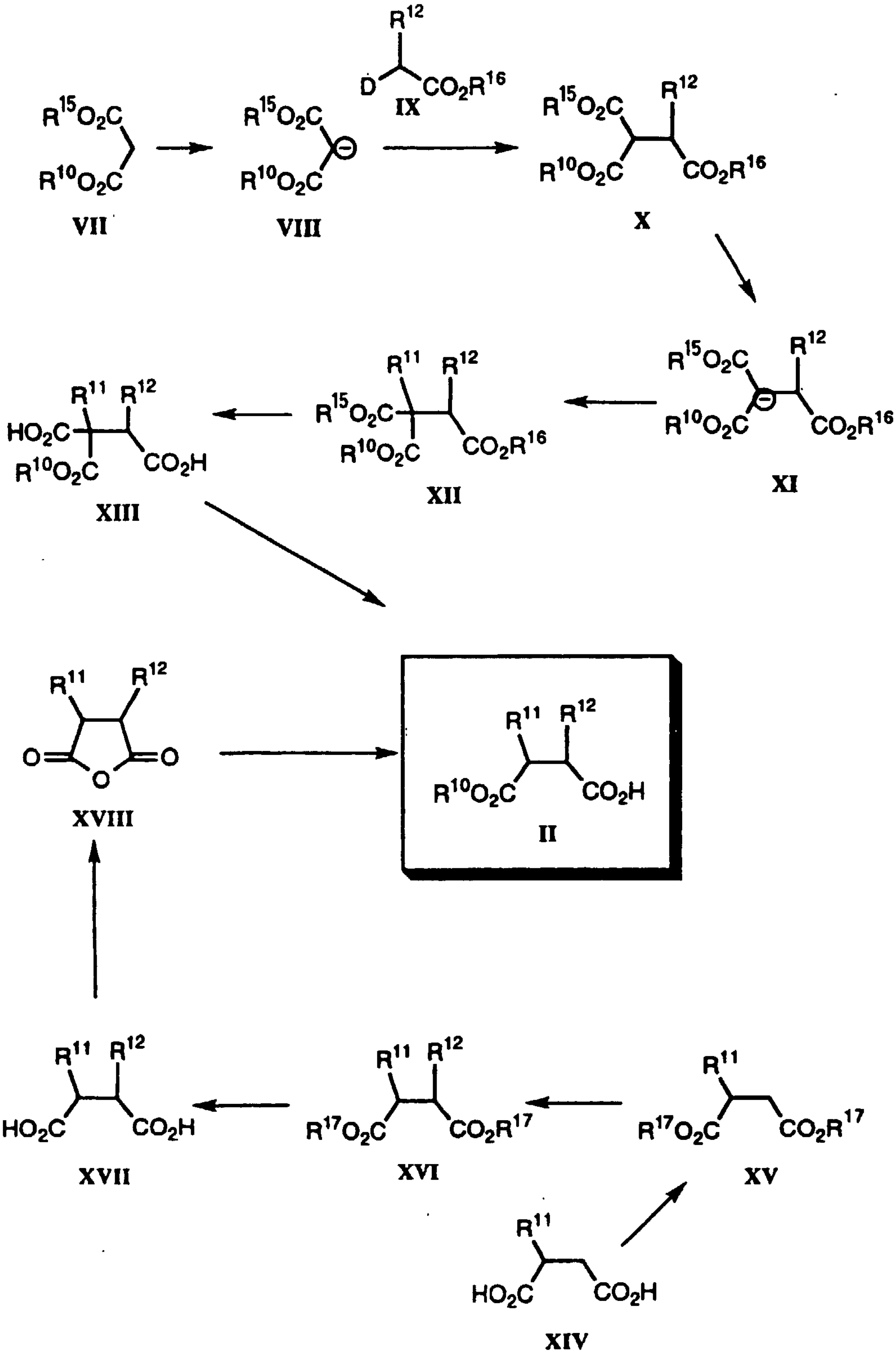

VII VIII IX X XI XII XIII II XVIII XVII XVI XV XIV

In the above Reaction Scheme, $R^{10}$, $R^{11}$ and $R^{12}$, all have the same meanings as defined above, and $R^{15}$, $R^{16}$ and $R^{17}$ which are the same or different, are each independently a carboxy-protecting group, including for example, unsubstituted or optionally substituted ($C_1$-$C_6$) alkyl, benzyl, substituted benzyl, phenacyl and 2,2,2-trichloroethyl, and D is a replaceable group such as halogen.

Preferred is a route for producing a useful intermediate compound of the formula (II) for the production, which comprises employing, as a starting material, a malonic diester of the formula (VII) wherein $R^{11}$ is any radical except hydrogen and hydroxy.

Compounds of the formula (X) may be prepared by reacting an $\alpha$ -halogenocarboxylic acid ester (IX) wherein D is halogen with a compound of the formula (VIII) which is an anion of the malonic diester of the formula (VII) wherein halogen includes fluorine, chlorine, bromine, iodine, preferably bromine.

Suitable bases which are used for forming an anion of said malonic diester (VII) include those ordinarily utilizable in conventional reactions. Illustrative examples of such bases are alkali metal hydrides such as sodium hydride, lithium diisopropylamide (LDA), lithium bis(trimethylsilyl)amide, alcoholates including for example, alkali metal alcoholates such as sodium methoxide, sodium ethoxide, sodium propoxide, potassium tert-butoxide and potassium ethoxide, etc. Preferred bases are sodium hydride, and potassium tert-butoxide.

Suitable solvents for carrying out the reaction include inert organic solvents. Illustrative examples of such solvents are saturated hydrocarbons (e.g., n-hexane, etc.), ethers (e.g., tetrahydrofuran (THF), etc.), aromatic hydrocarbons (e.g., benzene, toluene, etc.), amides (e.g., dimethylformamide (DMF), dimethylacetamide (DMAc), etc.), and halogenated hydrocarbons (e.g., dichloromethane, chloroform, etc.). A preferred solvent is DMF.

The reaction temperature range is ordinarily from -78 to 50°C and preferably from 0 to 20°C. The reaction time varies depending on the particular starting material, solvent, reaction temperature, and the like employed, but a reaction period of from 30 minutes to 4 hours is sufficient. The reaction is ordinarily carried out for 1 to 2 hours.

Following the formation of the compound (VIII), it can be reacted with the $\alpha$ -halogenocarboxylic acid ester (IX) in a solvent. Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is DMF. The reaction temperature range is ordinarily from -10 to 50°C and preferably from -5 to 0°C. The reaction time varies depending on the particular starting material, solvent, reaction temperature, and the like employed, but it is ordinarily from 2 to 24 hours, and preferably from 10 to 20 hours.

Compounds of the formula (XII) may be prepared by conversion of the compound (X) into the anion (XI) followed by treatment with a reactant selected from the group consisting of ($C_1$-$C_6$) alkyl halides (preferably ($C_1$-$C_6$) alkyl iodides such as methyl iodide), substituted ($C_1$-$C_6$) alkyl halides (preferably 1-benzyloxycarbonylamino-3-iodo-propane, 1-benzyloxycarbonylamino-5-iodo-pentane, 1-tetrahydropyranyloxy-8-iodo-octane, 1-ethoxy-ethoxy-2-iodo-ethane, 1-n-butyloxy-2-iodo-ethane, 1-iso-butyloxy-2-iodo-ethane, 1-phenoxy-2-iodo-ethane, 1-tetrahydropyranyloxy-3-iodo-propane, etc.), alkenyl halides (preferably cinnamyl bromide ($C_6H_5$-CH=CH-$CH_2$-Br), methallyl iodide [CH=C($CH_3$)-$CH_2$-I], etc.), and substituted alkenyl halides (preferably nitro-cinnamyl bromide ($O_2N$-$C_4H_5$-CH=CH-$CH_2$-Br), methoxycarbonyl-cinnamyl bromide, 1-methoxycarbonyl-phenyl-3-iodo-propane, cyano-cinnamyl bromide, 1-cyano-phenyl-3-iodo-propane, cyano-benzyl bromide, 1-benzyloxy-phenyl-3-iodo-propane, etc.), and if desired, hydrogenation.

Suitable bases which are used for forming the compound (XI) include those customarily utilizable in such reactions. Illustrative examples of such bases are LDA, lithium bis(trimethylsilyl)amide, alkali metal hydrides such as sodium hydride, alcoholates including for example, alkali metal alcoholates such as sodium methoxide, sodium ethoxide, sodium propoxide, potassium tert-butoxide and potassium ethoxide, etc. A preferred base is sodium hydride.

Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is DMF. The reaction temperature range is ordinarily from -10 to 50°C and preferably from 10 to 30°C. The reaction time varies depending on the particular starting material, solvent, reaction temperature, and the like employed, but it is ordinarily from 30 minutes to 4 hours, and preferably from 1 to 2 hours.

Following the formation of the compound (XI), it can be reacted with the alkyl halide in a solvent. Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is DMF. The reaction temperature range is ordinarily from -10 to 100°C and preferably from -5 to 70°C. The reaction time varies depending on the particular starting material, solvent, reaction temperature, and the like employed, but it is ordinarily from 2 to 48 hours, and preferably from 10 to 20 hours.

Further, as required, hydrogenation is conducted to form the compound of the formula (XII). Suitable catalysts for the hydrogenation include palladium catalysts such as palladium on carbon, platinum catalysts, and preferably palladium on carbon. Suitable solvents for carrying out the reaction include inert organic solvents and the like as long as they do not poison the catalyst. Illustrative examples of such solvents are alcohols (e.g., methanol, ethanol, etc.), amides (e.g., DMF, DMAc, etc.), acetic acid, water, and mixtures thereof, and preferably methanol and ethanol. The reaction temperature range is ordinarily from 0 to 50°C and preferably from 10 to 30°C. The reaction time varies

depending on the particular starting material, solvent, reaction temperature, and the like employed, but it is ordinarily from 1 to 24 hours, and preferably from 1 to 10 hours.

Compounds of the formula (XIII) may be prepared by de-esterification of the compound (XII). $R^{10}$, $R^{15}$ and $R^{16}$, all have the meanings as given above, but preferably $R^{10}$ is tert-butyl, and $R^{15}$ and $R^{16}$ are both benzyl, for the preparation of the compound (XIII). For instance, when $R^{15}$ and $R^{16}$ are both benzyl, the de-esterification can be achieved by hydrogenation.

Suitable catalysts for the hydrogenation are those including palladium on carbon, platinum, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), amides (e.g., DMF, DMAc, etc.), acetic acid, water, and mixtures thereof, and preferably methanol and ethanol. The reaction temperature range is ordinarily from 0 to 50°C and preferably from 10 to 30°C. The reaction time varies depending on the particular starting material, solvent, reaction temperature, and the like employed, but it is ordinarily from 1 to 24 hours, and preferably from 1 to 10 hours.

A route for producing a compound of the formula (II) which is a useful intermediate for the production, employing a compound of the formula (XIV) as a starting material, is preferred when $R^3$ is hydrogen or hydroxy.

Compounds of the formula (XV) may be prepared by

(a) reacting a succinic acid derivative of the formula (XIV) with an alcohol in the presence of a suitable coupling agent such as N,N'-dicyclohexylcarbodiimide (DCC), 1-ethyl-3-(3-dimethylaminopropyl)-carbodiimide hydrochloride (EDC • HCl) and a base such as N,N-dimethylaminopyridine,
(b) converting the compound (XVI) into a salt thereof formed with a member selected from the group consisting of sodium, potassium, cesium and the like, followed by treatment with an alkyl halide or benzyl halide, or
(c) reacting the compound (XIV) with a complex of thionyl chloride and an alcohol.

For instance, in the case (c) where the compound (XIV) is reacted with the thionyl chloride-alcohol complex, suitable alcohols which are used in the reaction include, but are not limited to, methanol, ethanol, n-propyl alcohol, iso-propyl alcohol, tert-butyl alcohol, phenol, benzyl alcohol, phenacyl alcohol, 2,2,2-trichloroethanol (preferably methanol, ethanol, iso-propyl alcohol, and tert-butyl alcohol, and more preferably iso-propyl alcohol). The alcohol also serves as a solvent. The reaction temperature range is ordinarily from -30 to 10°C and preferably from -10 to 0°C. The reaction time varies depending on the particular starting material, solvent, reaction temperature, and the like employed, but it is ordinarily from 5 to 24 hours, and preferably from 10 to 15 hours.

Compounds of the formula (XVI) may be prepared by converting the succinic diester of the formula (XV) into an anion thereof which is then treated with methallyl iodide, followed by hydrogenation.

Suitable bases which are used for forming the anion of the compound (XV) include those customarily utilizable in such reactions. Illustrative examples of such bases are alkali metal hydrides such as sodium hydride, LDA, lithium bis(trimethylsilyl)amide, alcoholates including for example, alkali metal alcoholates such as sodium methoxide, sodium ethoxide, sodium propoxide, potassium tert-butoxide and potassium ethoxide, etc. A preferred base is LDA. Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is THF. The reaction temperature range is ordinarily from -78 to 0°C and preferably from -70 to -10°C. The reaction time varies depending on the particular starting material, solvent, reaction temperature, and the like employed, but it is ordinarily from 30 minutes to 24 hours, and preferably from 4 to 12 hours.

Following the process, the reaction with methallyl iodide is carried out in a solvent. Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is THF. The reaction temperature range is ordinarily from -78 to 0°C and preferably from -70 to -10°C. The reaction time varies depending on the particular starting material, solvent, reaction temperature, and the like employed, but it is ordinarily from 2 to 48 hours, and preferably from 10 to 20 hours.

Further, as required, hydrogenation is conducted to form the compound of the formula (XVI). Suitable catalysts for the hydrogenation include palladium on carbon, platinum catalysts, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), amides (e.g., DMF, DMAc, etc.), acetic acid, and water, and preferably methanol. The reaction temperature range is ordinarily from 0 to 50°C and preferably from 10 to 30°C. The reaction time varies depending on the particular starting material, solvent, reaction temperature, and the like employed, but it is ordinarily from 1 to 24 hours, and preferably from 1 to 6 hours.

Compounds of the formula (XVII) may be prepared by de-esterification of the compound (XVI). For instance,

when $R^{17}$ is iso-propyl, the de-esterification can be achieved by alkaline hydrolysis. Suitable bases which are used for the hydrolysis are not limited as long as they are customarily employed as bases in ordinary reactions. Illustrative examples of such bases are sodium carbonate, potassium carbonate, sodium hydroxide, potassium hydroxide, sodium bicarbonate, lithium hydride, sodium hydride, etc. Preferred bases are sodium hydroxide, and potassium hydroxide. Suitable solvents for carrying out the reaction are not limited as long as they do not prevent the processing and are capable of dissolving starting materials. Illustrative examples of such solvents are amides (e.g., DMF, DMAc, etc.), alcohols, ethers (e.g., diethyl ether, tetrahydrofuran, dioxane, etc.), water, ketones (e.g., acetone, etc.), and preferably alcohols, water, dioxane, acetone, and mixtures thereof. The reaction temperature range is ordinarily from -20 to 150°C and preferably from -10 to 100°C. The reaction time is ordinarily from 5 minutes to 36 hours, and preferably from 10 minutes to 24 hours.

The compounds (II) which are useful intermediates for the production can be prepared by (a) decarboxylation of the compound of the formula (XIII), or (b) reacting an alcohol with an acid anhydride compound of the formula (XVIII) derived from the dicarboxylic acid of the formula (XVII).

(a) The decarboxylation of the compound (XIII) is carried out in the presence of a tertiary amine such as triethylamine, N-methylmorpholine, and N-ethylmorpholine. Suitable solvents for carrying out the reaction include inert organic solvents. Illustrative examples of such solvents are saturated hydrocarbons (e.g., n-hexane, etc.), aromatic hydrocarbons (e.g., benzene, toluene, etc.), and the like. A preferred solvent is toluene. The reaction temperature range is ordinarily from 70 to 150°C, and preferably from 100 to 120°C. The reaction time varies depending on the particular starting material, solvent, reaction temperature, and the like employed, but it is ordinarily from 1 to 5 hours, and preferably from 2 to 3 hours.

(b) The process for producing the intermediate (II) via the acid anhydride (XVIII) from the dicarboxylic acid (XVII) is carried out for example by reference to J. Org. Chem., 47, 4928(1982). Suitable alcohols which are reacted with the acid anhydride (XVIII) include, but are not limited to, methanol, ethanol, n-propyl alcohol, iso-propyl alcohol, tert-butyl alcohol, phenol, benzyl alcohol, phenacyl alcohol, 2,2,2-trichloroethanol, etc. Preferred alcohols are methanol, ethanol, benzyl alcohol, and 2,2,2-trichloroethanol.

Described below are processes for producing the intermediates (III).

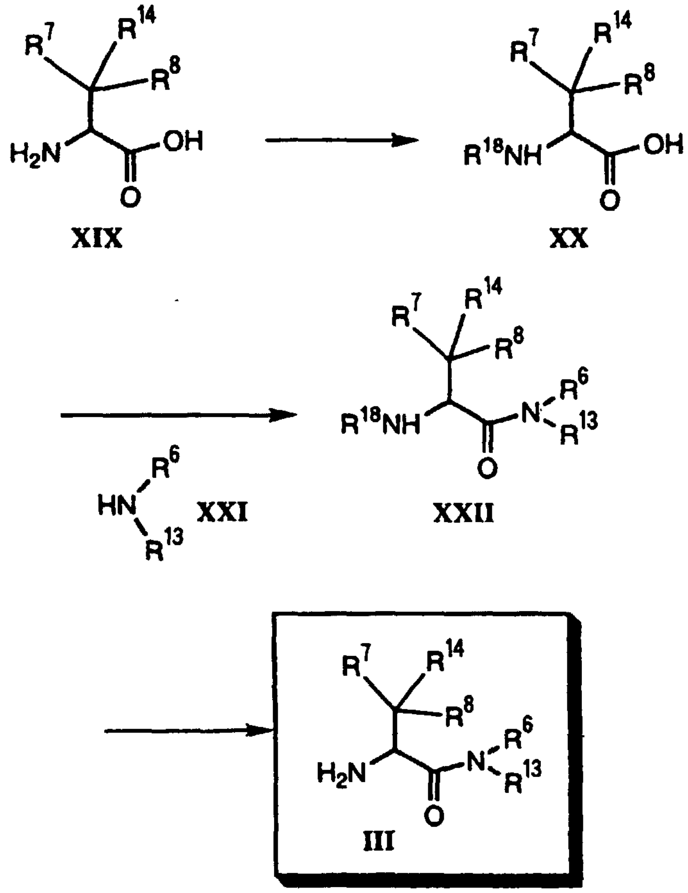

XIX XX XXI XXII III

In the above Reaction Scheme, $R^6$, $R^7$, $R^8$, $R^{13}$ and $R^{14}$, all have the same meanings as defined above, and $R^{18}$ is an amino-protecting group, including for example, tert-butyloxycarbonyl (Boc), bentyloxycarbonyl (Z), substituted benzyloxycarbonyl and the like.

The intermediates (III) wherein $R^{14}$ is a group of the formula: -X-E or -X-A-E, and E is (a) protected bis(phosphono)-methyl, (b) protected bis(phosphono)(hydroxy)-methyl, or (c) substituted imidazolyl-methyl, can be prepared from a compound of the formula (XXII) wherein E is amino, carboxyl, or hydroxymethyl.

For instance, when (a) E is protected bis(phosphono)-methyl, the compounds (III) can be prepared by reacting protected 4'-aminophenylalanine with an ester of formic acid, such as ethyl orthoformate or an ester of phosphorous acid, such as diethyl phosphonate (see: Phosphorous and Sulfur, 11, 311 (1981)).

When (b) E is protected bis(phosphono)(hydroxy)-methyl, the compounds (III) can be prepared by converting protected 4'-carboxyphenylalanine into an acid halide thereof, followed by treatment with a phosphite compound such as tribenzyl phosphite (see: Phosphorous, Sulfur, and Silicon, 113, 295 (1996)).

Further, when (c) E is substituted imidazolyl-methyl, the compounds (III) can be prepared by converting protected 4'-hydroxymethylphenylalanine into a methanesulfonate or p-toluenesulfonate derivative thereof, followed by treatment with an imidazole derivative (see: J. Med. Chem., 31, 2193 (1988)).

(a) In the process for forming protected bis(phosphono)methyl, 4'-aminophenylalanine may be mixed for example with ethyl orthoformate and diethyl phosphonate, and then heated. The reaction is customarily carried out in the absence of a solvent. The reaction temperature range is ordinarily from 100 to 180°C, and preferably from 140 to 160°C. The reaction time varies depending on the particular starting material, reaction temperature, and the like employed, but it is ordinarily from 1 to 8 hours, and preferably from 1 to 6 hours.

(b) In the process for forming protected bis(phosphono)(hydroxy)-methyl, conversion of protected 4'-carboxyphenylalanine into an acid halide thereof can be achieved for example by treatment with thionyl chloride, or with dimethylchloroformiminium chloride formed from oxalyl chloride and DMF. Suitable solvents for carrying out the reaction

include inert solvents. Illustrative examples of such solvents are amides (e.g., DMF, etc.), ethers (e.g., diethyl ether, etc.), halogenated hydrocarbons (e.g., dichloromethane, chloroform, etc.), and the like. The reaction temperature range is ordinarily from -20 to 100°C, and preferably from -5 to 80°C. The reaction time is ordinarily from 5 minutes to 24 hours, and preferably from 10 minutes to 8 hours.

Following the formation of the acid halide, formation of protected bis(phosphono)(hydroxy)-methyl can be achieved for example by reaction with trialkyl phosphite in the presence of an equimolar proton donor(preferably, alcohol, acetic acid, etc.). Suitable solvents for carrying out the reaction include inert solvents. Illustrative examples of such solvents are amides (e.g., DMF, etc.), ethers (e.g., diethyl ether, etc.), halogenated hydrocarbons (e.g., dichloromethane, chloroform, etc.), and the like. The reaction temperature range is ordinarily from -20 to 100°C, and preferably from -5 to 50°C. The reaction time is ordinarily from 10 minutes to 24 hours, and preferably from 30 minutes to 8 hours.

(c) The sulfonated intermediates may be prepared by reacting protected 4'-hydroxymethylphenylalanine with sulfonating agents in the presence of a base. Suitable sulfonating agents include alkylsulfonyl halides such as methanesulfonyl chloride, arylsulfonyl halides such as p-toluenesulfonyl chloride, and the like. Preferred sulfonating agents are methanesulfonyl chloride, p-toluenesulfonyl chloride, etc. Suitable bases include alkylamines such as trialkylamines, nitrogen-containing heterocyclic compounds such as pyridine, and the like. A preferred base is triethylamine. Suitable solvents for carrying out the reaction include inert solvents. Illustrative examples of such solvents are amides (e.g., DMF, DMAc, etc.), aromatic hydrocarbons (e.g., benzene, etc.), ethers (e.g., diethyl ether, etc.), halogenated hydrocarbons (e.g., dichloromethane, chloroform, etc.), and the like. A preferred solvent is dichloromethane. The reaction temperature range is ordinarily from -20 to 50°C and preferably from -5 to 20°C. The reaction time is ordinarily from 10 minutes to 4 hours, and preferably from 30 minutes to 2 hours.

The resulting sulfonated intermediate is reacted with an anion of an imidazole derivative to give a compound of the formula (XXII) wherein E is substituted imidazolyl-methyl. Reagents for forming the anion include those ordinarily utilizable in conventional reactions. Illustrative examples of such reagents are alkali metal hydrides such as sodium hydride, LDA, lithium bis(trimethylsilyl)-amide, alcoholates including for example, alkali metal alcoholates such as sodium methoxide, sodium ethoxide, sodium propoxide, potassium tert-butoxide and potassium ethoxide, etc. A preferred base is sodium hydride.

Compounds of the formula (XX) may be prepared by introducing a protecting group such as a Boc group, a Z radical, and a substituted benzyloxycarbonyl moiety, into an amino moiety of a compound of the formula (XIX).

For example, introduction of a Z radical can be achieved by treatment with chlorobenzylformate in the presence of an ordinarily utilizable base (e.g., sodium carbonate, potassium carbonate, sodium bicarbonate, sodium hydroxide, potassium hydroxide, etc.). Suitable solvents for carrying out the reaction include ethers (e.g., dioxane, etc.), ketones (e.g., acetone, etc.), water, and mixture thereof. The reaction temperature range is ordinarily from -20 to 30°C, and preferably from -5 to 5°C. The reaction time is ordinarily from 2 to 24 hours, and preferably from 6 to 15 hours.

The compounds of the formula (XXII) may be prepared by reacting the compound (XX) with an amine compound of the formula (XXI) according to conventional coupling techniques wherein $R^6$ has the meaning as given above, but is preferably hydrogen, and $R^{13}$ has the meaning as given above, but is preferably ($C_1$-$C_4$) alkyl (more preferably methyl), ($C_3$-$C_6$) alkyl (more preferably cyclopropyl), optionally substituted ($C_1$-$C_4$) alkyl (more preferably 2-N,N-dimethylethyl, protected 2-carboxyethyl, protected 2-hydroxyethyl, protected 2-hydroxy-1,1-dimethylethyl, protected 2-hydroxy-1-methylethyl, or protected 6,6-bis(phosphono)-6-(hydroxy)-hexyl), or a heterocyclic group (more preferably piperidinyl, or morpholinyl). Suitable coupling agents include DCC, EDC • HCl, N-ethoxycarbonyl-2-ethoxy-1,2-dihydroquinoline (EEDQ), 1-hydroxybenzotriazole (HOBT) derivatives, N-hydroxy-5-norbornene-2,3-dicarboximide (HONB) derivatives, N-hydroxysuccinimide (HOSu) derivatives, carbonate monoalkyl ester derivatives formed by treatment with isobutyloxycarbonyl chloride or ethyloxycarbonyl chloride, diphenylphosphorylazide (DPPA), and the like. A preferred coupling agent is EDC • HCl. Suitable solvents for carrying out the reaction are not limited as long as they do not inhibit the process of reactions but are capable of dissolving starting materials. Illustrative examples of such solvents include DMF, DMAc, ethyl acetate, diethyl ether, dichloromethane, chloroform, dioxane, etc. A preferred solvent is DMF. The reaction temperature range is ordinarily from -20 to 50°C, and preferably from -15 to 30°C. The reaction time is ordinarily from 1 to 24 hours, and preferably from 2 to 15 hours.

The compounds of the formula (III) which are useful as intermediates for the production may be prepared by removal of an amino-protecting group from the compound (XXII). Deprotection may be carried out according to a prior art technique well known to artisans, which varies depending on the particular protecting group employed. For example, the protecting group, Z, can be readily removed by hydrogenation. Suitable catalysts for the hydrogenation include palladium on carbon, platinum catalysts, etc., and preferably palladium on carbon. Suitable solvents for carrying out the reaction include, but are not limited to, inert organic solvents and the like as long as they do not poison the catalyst. Illustrative examples of such solvents are alcohols (e.g., methanol, ethanol, etc.), amides (e.g., DMF, DMAc, etc.), acetic acid, and water, and preferably methanol. The reaction temperature range is ordinarily from 0 to 50°C and preferably

from 10 to 30°C. The reaction time varies depending on the particular starting material, solvent, reaction temperature, and the like employed, but it is ordinarily from 1 to 24 hours, and preferably from 1 to 6 hours.

Described below are processes for the preparation of intermediates (V).

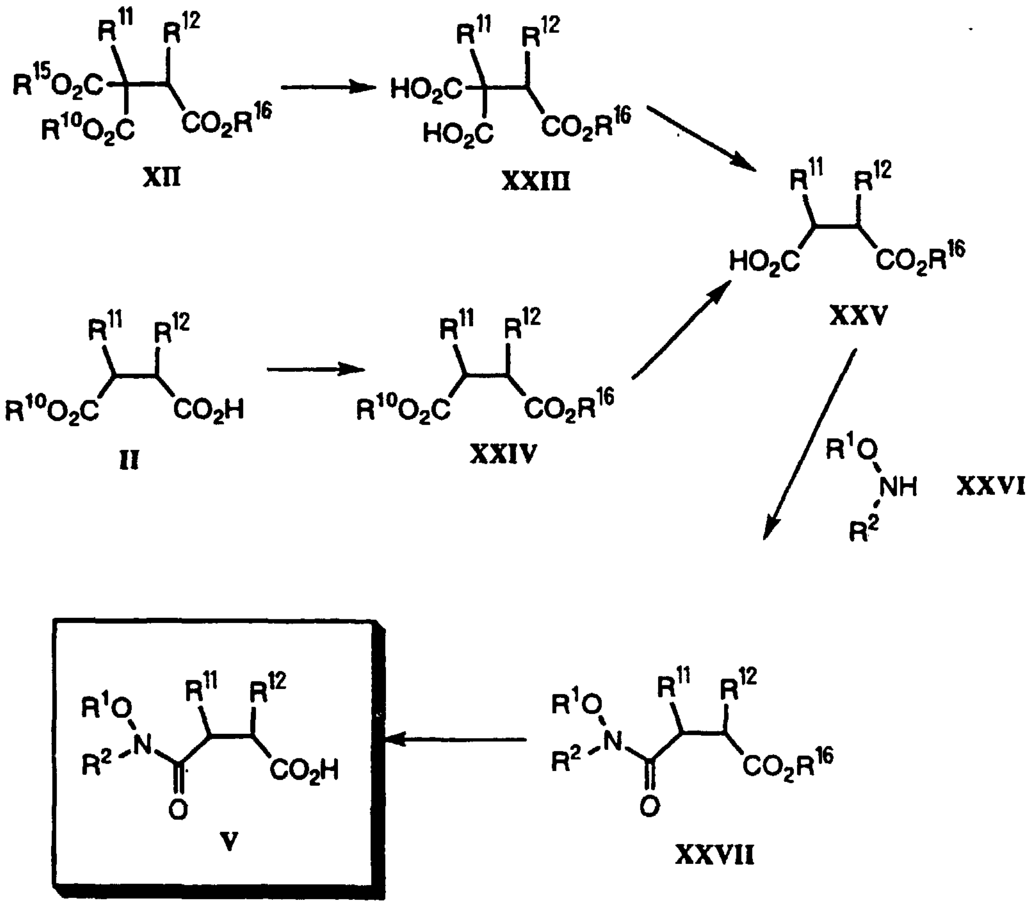
XII XXIII XXV II XXIV XXVI V XXVII

Compounds of the formula (XXIII) may be prepared by de-esterification of a compound of the formula (XII). $R^{10}$, $R^{15}$, and $R^{16}$, all have the meanings as given above, but it is preferable for the production of the compound (XXIII) that $R^{16}$ is 2,2,2-trichloroethyl, and both $R^{10}$ and $R^{15}$ are benzyl. For example, when both $R^{10}$ and $R^{15}$ are benzyl, the de-esterification can be achieved by hydrogenation. Suitable catalysts for the hydrogenation include palladium on carbon, platinum catalysts, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), THF, DMF, DMAc, acetic acid, water, and mixtures thereof. A preferred solvent is THF. The reaction temperature range is ordinarily from 0 to 50°C, and preferably from 10 to 30°C. The reaction time varies depending on the particular starting material, solvent, reaction temperature, and the like employed, but it is ordinarily from 1 to 24 hours, and preferably from 1 to 6 hours.

Compounds of the formula (XXIV) may be prepared by reacting a succinic acid derivative of the formula (II) with an alcohol in the presence of a coupling agent such as N,N'-dicyclohexylcarbodiimide (DCC), and 1-ethyl-3-(3-dimethylaminopropyl)-carbodiimide hydrochloride (EDC • HCl), along with a base such as N,N-dimethylamino-pyridine. Suitable alcohols include, but are not limited to, methanol, ethanol, n-propyl alcohol, iso-propyl alcohol, tert-butyl alcohol, phenol, benzyl alcohol, phenacyl alcohol, 2,2,2-trichloroethanol, and the like (preferably 2,2,2-trichloroethanol). Suitable solvents for carrying out the reaction include inert organic solvents. Illustrative examples of such solvents are saturated hydrocarbons (e.g., n-hexane, etc.), ethers (e.g., THF, etc.), aromatic hydrocarbons (e.g., benzene, toluene, etc.), amides (e.g., DMF, DMAc, etc.), and halogenated hydrocarbons (e.g., dichloromethane, chloroform, etc.). A preferred solvent is dichloromethane. The reaction temperature range is ordinarily from -30 to 30°C, and preferably from -10 to 20°C. The reaction time varies depending on the particular starting material, solvent, reaction temperature, and the like employed, but it is ordinarily from 1 to 24 hours, and preferably from 3 to 15 hours.

Compounds of the formula (XXV) may be prepared by (a) decarboxylating a compound of the formula (XXIII), or partially de-esterifying a compound of the formula (XXIV).

(a) Decarboxylation of the compound (XXIII) may be carried out when heated in an inert solvent. Suitable inert organic solvents include for example saturated hydrocarbons (e.g., n-hexane, etc.), aromatic hydrocarbons (e.g., toluene, benzene, etc.), etc. A preferred solvent is toluene. The reaction temperature range is ordinarily from 70 to 150°C, and preferably from 100 to 120°C. The reaction time varies depending on the particular starting material, solvent, reaction temperature, and the like employed, but it is ordinarily from 1 to 5 hours, and preferably from 2 to 3 hours.

(b) Partial de-esterification of the compound (XXIV) may be achieved for example by hydrogenation when $R^{10}$ is benzyl or by treatment with an acid when $R^{10}$ is tert-butyl. Suitable catalysts for the hydrogenation include palladium on carbon, platinum catalysts, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), ethers (e.g., THF, etc.), amides (e.g., DMF, DMAc, etc.), acetic acid, and water. A preferred solvent is THF. The reaction temperature range is ordinarily from 0 to 50°C, and preferably from 10 to 30°C. The reaction time varies depending on the particular starting material, solvent, reaction temperature, and the like employed, but it is ordinarily from 1 to 24 hours, and preferably from 1 to 6 hours. Suitable acids include trifluoroacetic acid (TFA), solutions containing hydrochloric acid in organic solvents such as ethyl acetate and dioxane, and the like. A preferred acid is TFA which also serves as a solvent. The reaction temperature range is ordinarily from -10 to 50°C, and preferably from 0 to 30°C. The reaction time varies depending on the particular diester compound (XXIV), acid, reaction temperature, and the like employed, but it is ordinarily from 30 minutes to 24 hours, and preferably from 1 to 15 hours.

Compounds of the formula (XXVII) may be prepared by converting a half ester compound of the formula (XXV) into an acid halide thereof, followed by treatment with a protected hydroxyamine compound of the formula (XXVI), but it is preferred for the purpose of selectively removing the protecting group, $R^{16}$, separate from the protecting groups, $R^1$ and $R^2$, that $R^{16}$ is 2,2,2-trichloroethyl. Formation of the acid halide is accomplished by treatment with thionyl chloride, or with dimethylchloroformiminium chloride formed from oxalyl chloride and DMF. Suitable solvents for carrying out the reaction include inert solvents. Illustrative examples of such solvents are aromatic hydrocarbons (e.g., benzene, toluene, etc.), amides (e.g., DMF, DMAc, etc.), ethers (e.g., diethyl ether, etc.), and halogenated hydrocarbons (e.g., dichloromethane, chloroform, etc.), and the like. A preferred solvent is dichloromethane. The reaction temperature range is ordinarily from -20 to 100°C, and preferably from -5 to 80°C. The reaction time is ordinarily from 5 minutes to 24 hours, and preferably from 10 minutes to 8 hours.

The resulting acid halide can be reacted with the protected hydroxyamine compound (XXVI) in the presence of a base to give the target intermediate. Suitable solvents for carrying out the reaction include inert solvents. Illustrative examples of such solvents are aromatic hydrocarbons (e.g., benzene, toluene, etc.), amides (e.g., DMF, DMAc, etc.), ethers (e.g., diethyl ether, etc.), and halogenated hydrocarbons (e.g., dichloromethane, chloroform, etc.), and the like. A preferred solvent is dichloromethane. Suitable bases include tertiary amines such as trialkylamine and pyridine. A preferred base is triethylamine. The reaction temperature range is ordinarily from -20 to 50°C, and preferably from -5 to 30°C. The reaction time is ordinarily from 5 minutes to 24 hours, and preferably from 10 minutes to 5 hours.

Compounds of the formula (V) may be prepared by de-esterification of a compound of the formula (XXVII). For example, when $R^{16}$ is 2,2,2-trichloroethyl, deprotection is accomplished by treatment with zinc in acetic acid which serves as a solvent for carrying out the reaction. The reaction temperature range is ordinarily from 0 to 50°C, and preferably from 5 to 40°C. The reaction time is ordinarily from 30 minutes to 15 hours, and preferably from 1 to 5 hours.

Described below are processes for the preparation of intermediates (IV) and (VI), together with the target compounds (I).

Routes for the production of intermediates (IV) and (VI), and target compounds (I)

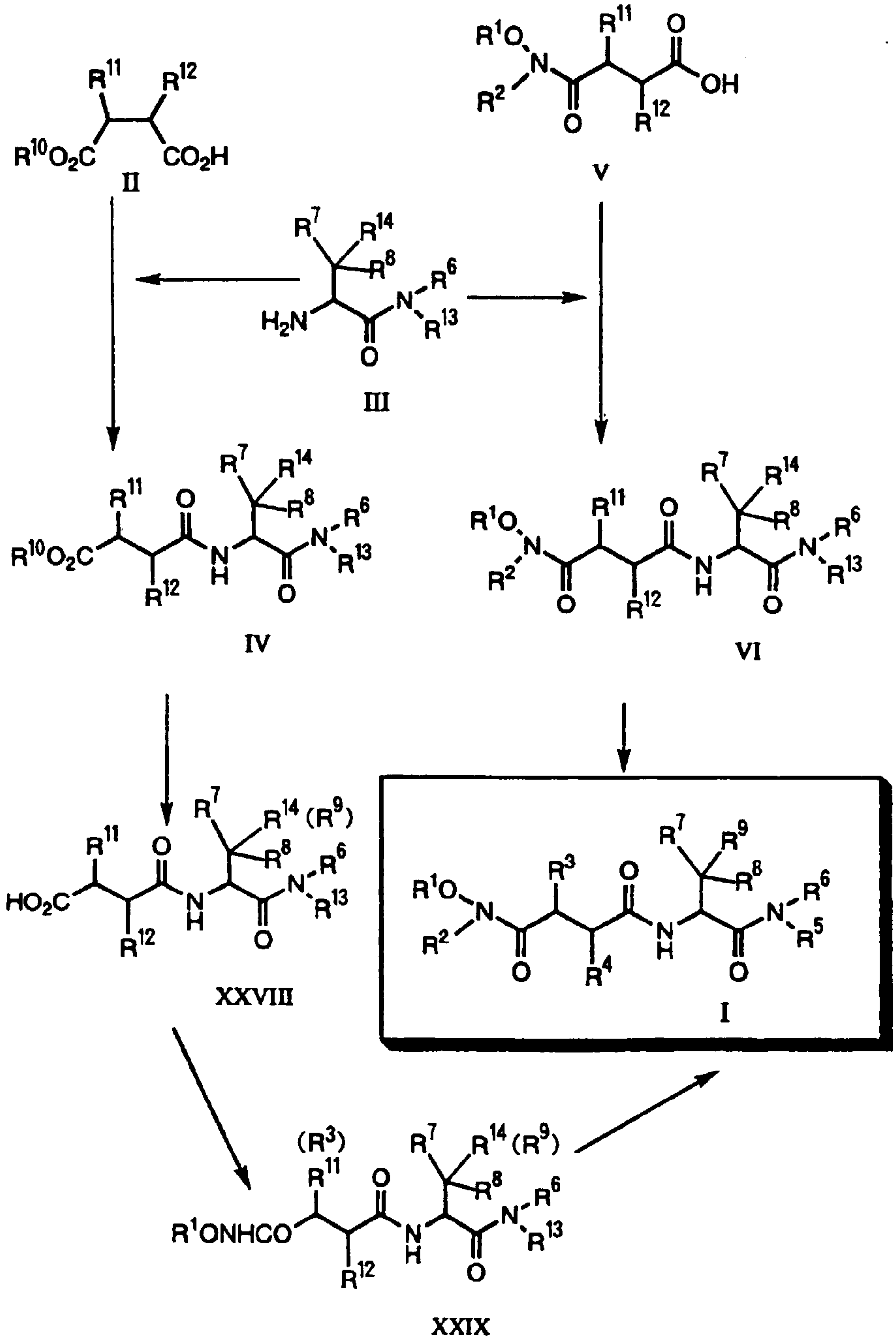

II

V

III

IV

VI

XXVIII

I

XXIX

In the above Scheme, $R^1$ to $R^{14}$, all have the meanings as given above.

Compounds of the formula (IV) may be prepared by reacting the intermediate (II) with the intermediate (III)

according to conventional coupling techniques wherein $R^{10}$ has the meaning as given above, but is a carboxy-protecting group, including for example, unsubstituted or optionally substituted alkyl, unsubstituted or optionally substituted aralkyl, and the like; preferably tert-butyl, benzyl, substituted benzyl, phenacyl, or 2,2,2-trichloroethyl; and more preferably tert-butyl, or benzyl).

Coupling agents used in this reaction include DCC, EDC • HCl, EEDQ, HOBT derivatives, HONB derivatives, HOSu derivatives, carbonate monoalkyl ester derivatives formed by treatment with isobutyloxycarbonyl chloride or ethyloxycarbonyl chloride, DPPA, and the like. A preferred coupling agent is EDC • HCl. Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. The reaction temperature range is ordinarily from -20 to 50°C, and preferably from -15 to 30°C. The reaction time is ordinarily from 1 to 24 hours, and preferably from 2 to 15 hours.

Compounds of the formula (XXVIII) may be prepared by first de-esterification of the compound of the formula (IV) and, as required, then conversion of $R^{14}$ into the target functional group, $R^9$. For example, the de-esterification can be effected by treatment of the tert-butyl ester with a solution which is prepared by dissolving trifluoroacetic acid or hydrogen chloride in ethyl acetate or dioxane. The reaction temperature range is ordinarily from -10 to 20°C, and preferably from -5 to 5°C. The reaction time varies depending on the particular starting material, acid, reaction temperature, and the like employed, but it is ordinarily from 1 to 24 hours, and preferably from 1 to 15 hours.

Following the above process, conversion of $R^{14}$ into $R^9$ or of $R^{11}$ into $R^3$ may be carried out. For example, when $R^{14}$ is a radical containing a protected amino group and $R^9$ is an acetimidoylimino radical, the conversion can be accomplished by first removal of an amino group protection and then treatment with ethyl acetimidate. When the amino-protecting group is Boc, it is removed by treatment with TFA simultaneously with removal of the tert-butyl ester. Alternatively, when it is Z, the deprotection is accomplished by hydrogenolysis.

Suitable catalysts for the hydrogenation are those including palladium on carbon, platinum, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), ethers (e.g., THF, etc.), amides (e.g., DMF, DMAc, etc.), acetic acid, and water, and preferably methanol. The reaction temperature range is ordinarily from 0 to 50°C and preferably from 10 to 30°C. The reaction time varies depending on the particular starting material, solvent, reaction temperature, and the like employed, but it is ordinarily from 1 to 24 hours, and preferably from 1 to 15 hours.

Introduction of an acetimidoyl moiety into the deprotected amino radical may be accomplished by treatment with ethyl acetimidate in the presence of a member selected from customarily utilizable bases (inorganic bases such as sodium carbonate, potassium carbonate, sodium hydroxide and potassium hydroxide, and organic bases such as trialkylamine, N-methylmorpholine, pyridine and N,N-dimethylaminopyridine). Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 50°C, and preferably from -15 to 30°C. The reaction time is ordinarily from 5 minutes to 24 hours, and preferably from 10 minutes to 15 hours.

Compounds of the formula (XXIX) may be prepared by reacting the carboxylic acid compound (XXVIII) with a protected hydroxy-containing hydroxyamine compound according to conventional coupling techniques wherein the hydroxy-protecting group is selected from those known to artisans in the art, but include for example, unsubstituted or substituted benzyl, trialkylsilyl, tert-butyldiphenylsilyl, tetrahydropyranyl, tert-butyl, and the like (preferably benzyl).

Coupling agents used in this reaction include DCC, EDC • HCl, EEDQ, HOBT derivatives, HONB derivatives, HOSu derivatives, carbonate monoalkyl ester derivatives formed by treatment with isobutyloxycarbonyl chloride or ethyloxycarbonyl chloride, DPPA, and the like. A preferred coupling agent is EDC • HCl. Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 20°C, and preferably from -15 to 0°C. The reaction time is ordinarily from 1 to 72 hours, and preferably from 2 to 48 hours.

Compounds of the formula (VI) may be prepared by reacting the intermediate (III) with the intermediate (V) according to conventional coupling techniques wherein both $R^1$ and $R^2$ have the meanings as given above, but preferably $R^1$ is tert-butyl, benzyl, substituted benzyl, or Boc, more preferably benzyl, and $R^2$ is Boc or Z.

Coupling agents used in this reaction include DCC, EDC • HCl, EEDQ, HOBT derivatives, HONB derivatives, HOSu derivatives, carbonate monoalkyl ester derivatives formed by treatment with isobutyloxycarbonyl chloride or ethyloxycarbonyl chloride, DPPA, and the like. A preferred coupling agent is EDC • HCl. Suitable solvents for carrying out the reaction are not limited as long as they do not inhibit the process of reactions but are capable of dissolving starting

materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. The reaction temperature range is ordinarily from -20 to 20°C, and preferably from -15 to 0°C. The reaction time is ordinarily from 1 to 24 hours, and preferably from 2 to 15 hours.

The compounds of the formula (I) may be prepared by deprotection of the hydroxy- and/or amino-protecting group(s) on the compound (VI) or (XXIX), and, as required, optional conversion of $R^{11}$ into $R^3$, $R^{12}$ into $R^4$, $R^{13}$ into $R^5$, and/or $R^{14}$ into $R^9$.

When the hydroxy-protecting group is benzyl and the amino-protecting group is Z or (Cl-Z), the protecting groups are deprotected by hydrogenolysis simultaneously. For removal of benzyl by hydrogenolysis, suitable catalysts for the hydrogenation are those including palladium on carbon, platinum, etc., and preferably palladium on carbon. Suitable solvents for carrying out the reaction include inert organic solvents and the like as long as they do not poison the catalyst. Illustrative examples of such solvents are alcohols (e.g., methanol, ethanol, etc.), amides (e.g., DMF, DMAc, etc.), acetic acid, and water. A preferred solvent is methanol or acetic acid. The reaction temperature range is ordinarily from 0 to 100°C, and preferably from 10 to 50°C. The reaction time varies depending on the particular starting material, solvent, reaction temperature, and the like employed, but it is ordinarily from 1 to 24 hours, and preferably from 1 to 15 hours.

Following the above process, conversion of $R^{14}$ into $R^9$ may be carried out. For example, when $R^{14}$ is a radical containing a protected amino group and $R^9$ is an acetimidoylimino radical, the conversion can be accomplished by first removal of an amino group protection and then treatment with ethyl acetimidate.

Introduction of an acetimidoyl moiety into the deprotected amino radical may be accomplished by treatment with ethyl acetimidate in the presence of a member selected from customarily utilizable bases (inorganic bases such as sodium carbonate, potassium carbonate, sodium hydroxide and potassium hydroxide, and organic bases such as trialkylamine, N-methylmorpholine, pyridine and N,N-dimethylaminopyridine). Suitable solvents for carrying out the reaction are not limited as long as they do not inhibit the process of reactions but are capable of dissolving starting materials. Illustrative examples of such solvents include amides (e.g., DMF, DMAc, etc.), esters (e.g., ethyl acetate, etc.), ethers (e.g., diethyl ether, dioxane, etc.), halogenated hydrocarbons (e.g., dichloromethane, chloroform, etc.), and the like. A preferred solvent is DMF. The reaction temperature range is ordinarily from -20 to 50°C, and preferably from -5 to 30°C. The reaction time is ordinarily from 5 minutes to 24 hours, and preferably from 10 minutes to 15 hours.

The reaction products so prepared may be subjected to ordinarily separation and purification processes after completion of the reactions. The products can be readily isolated by methods including for example extraction with water or an organic solvent, concentration, neutralization, distillation, column chromatography and recrystallization. The resulting compounds may be in the form of solvates or salts (including acid addition salts). Further, the inventive compounds may include those salts derived from medicinally, pharmaceutically or physiologically acceptable acids and bases. These salts are not limited to, but include: those of inorganic acids such as hydrochloric acid, hydrobromic acid, hydroiodic acid, sulfuric acid, nitric acid, phosphoric acid, and perchloric acid; occasionally, those of organic acids such as acetic acid, propionic acid, oxalic acid, succinic acid, citric acid, ascorbic acid, lactic acid, p-toluenesulfonic acid, methanesulfonic acid, fumaric acid, tartaric acid, and maleic acid; those of inorganic bases including alkali or alkaline earth metals such as sodium, potassium, calcium, and magnesium, and ammonium, and those of organic bases including for example dialkylamines such as dimethylamine, and diethylamine, trialkylamines, dibenzylamine, ethanolamine, triethanolamine, morpholine, N-methylmorpholine, piperidine and the like.

These compounds (I) may be converted into salts of pharmaceutically or physiologically acceptable acids or bases by conventional methods. Examples of such salts are those of inorganic acids, including hydrochloride, sulfate, and nitrate; depending on the particular inventive compound, those of organic acids, including acetate, oxalate, succinate, and maleate; those of alkali metals, including a sodium salt, and a potassium salt; those of alkaline earth metals, including a a calcium salt; and the like.

The diseases and/or disorders, associated with tissue degradation, which are targets for application of the compounds prepared according to the present invention include Alzheimer's disease, Parkinson's disease, pancreatitis, ulcerative colitis, aphthous ulcer, autoimmune diseases (including chronic rheumatoid arthritis, Crohn's disease, and anemia associated with autoimmune diseases), osteoarthritis, periodontal diseases and disorders, corneal ulcer, uveitis, a variety of bullae (including congenital epidermolysis bullosa, acquired epidermolysis bullosa, porphyria cutanea tarda (PCT), pemphigoid, and pemphigus vulgaris), refractory dermal ulcers (including bedsore, dermal ulcer in radiotherapeutic patients, dermal ulcer in diabetic patients, and dermal ulcer in patients suffering from arteriosclerosis obliterans), osteoporosis, Behcet's disease, aberrant angiogenesis (accompanying tumor growth, and including lymphoma, ovary cancer, and tumor metastasis and invasion), cachexia, various infectious diseases (including malaria, hepatitis C, HIV infection, tuberculosis, and septicemia), multiple sclerosis, psoriasis, diabates, schizophrenia, depression, etc.

The so produced compounds (I) and salts thereof are low-toxic and well absorbed via oral routes. The compounds (I) and salts thereof intensively inhibit the actions of vertebrate matrix metalloproteinases (MMPs) and/or tumor necrosis factor-$\alpha$-converting enzymes (TNF-$\alpha$ convertases), and serve as useful inhibitors of MMPs and/or TNF-$\alpha$ con-

vertases, in tissues of animals, especially mammals (e.g., human, canine, rabbit, rat, etc.) and the like.

Thus, the compounds (I) of the present invention and salts thereof are promising agents for prophylactically and/or therapeutically treating diseases and/or disorders in which vertebrate MMPs and/or TNF-α convertases have been implicated, for example, those associated with tissue degradation, including Alzheimer's disease, Parkinson's disease, pancreatitis, ulcerative colitis, aphthous ulcer, autoimmune diseases (including chronic rheumatoid arthritis, Crohn's disease, and anemia associated with autoimmune diseases), osteoarthritis, periodontal diseases and disorders, corneal ulcer, uveitis, a variety of bullae (including congenital epidermolysis bullosa, acquired epidermolysis bullosa, porphyria cutanea tarda (PCT), pemphigoid, and pemphigus vulgaris), refractory dermal ulcers (including bedsore, dermal ulcer in radiotherapeutic patients, dermal ulcer in diabetic patients, and dermal ulcer in patients suffering from arteriosclerosis obliterans), osteoporosis, Behcet's disease, aberrant angiogenesis (accompanying tumor growth, and including lymphoma, ovary cancer, and tumor metastasis and invasion), cachexia, various infectious diseases (including malaria, hepatitis C, HIV infection, tuberculosis, and septicemia), multiple sclerosis, psoriasis, diabates, schizophrenia, depression, etc. It should be noted that the compounds (I) of the present invention and salts thereof have not only potent inhibitory activity on MMPs and/or TNF-α convertases but also excellent bioavailability via oral administration routes and other routes (e.g., oral adsorbability, etc.), whereby they may be readily applied not only for the prophylaxis and/or treatment of diseases and/or disorders associated with the degradation of tissues but also for preventing the deterioration of such diseases and/or disorders and may be used as advantageous drugs.

In embodiments of the present invention, the compounds produced according to the present invention, i.e.,

$N^a$-[4-(Hydroxyamino)-2(R)-isobutyl-3(RS)-methylsuccinyl]-$N^e$-acetimidoyl-L-lysine N-methylamide • 1 acetate

$N^a$-[4-(Hydroxyamino)-2(R)-isobutyl-3(RS)-methylsuccinyl]-4'-aminomethyl-L-phenylalanine N-methylamide • 1 acetate

$N^a$-[4-(Hydroxyamino)-2(R),3(R or S)-diisobutylsuccinyl]-L-arginine N-methylamide • 1 acetate

$N^a$-[4-(Hydroxyamino)-2(R)-isobutyl-3(RS)-methylsuccinyl]-L-arginine N-methylamide • 1 acetate

$N^4$-[3-Guanidino-1(S)-methylcarbamoylpropyl]-$N^1$-hydroxy-3(R)-isobutyl-2-(RS)-(3-phenylpropyl)succinamide • 1 acetate

$N^4$-[3-Guanidino-1(S)-methylcarbamoylpropyl]-$N^1$-hydroxy-3(R),2(RS)-diisobutylsuccinamide • 1 acetate

$N^4$-[3-Guanidino-1(S)-methylcarbamoylpropyl]-$N^1$-hydroxy-3(R)-isobutyl-2(R or S)-methylsuccinamide • 1 acetate

$N^a$-[4-(Hydroxyamino)-2(R)-isobutyl-3(RS)-methylsuccinyl]-L-ornithine N-methylamide • 1 acetate

$N^a$-[4-(Hydroxyamino)-2(R)-isobutyl-3(RS)-(3-phenylpropyl)-succinyl]-$N^e$-acetimidoyl-L-lysine N-methylamide • 1 acetate

$N^a$-[4-(Hydroxyamino)-2(R)-isobutyl-3(R or S)-methylsuccinyl]-4'-[tetramethyl bis(phosphono)methylimino]-L-phenylalanine N-methylamide

$N^a$-(4-(Hydroxyamino)-2(R)-isobutyl-3(R or S)-methylsuccinyl]-4'-[trimethyl bis(phosphono)methylimino]-L-phenylalanine N-methylamide • 1 sodium salt

$N^a$-[4-(Hydroxyamino)-2(R)-isobutyl-3(RS)-methylsuccinyl]-4'-[tetraethyl bis(phosphono)methylimino]-L-phenylalanine N-methylamide

$N^a$-[4-(Hydroxyamino)-2(R)-isobutyl-3(RS)-methylsuccinyl]-4'-[triethyl bis(phosphono)methylimino]-L-phenylalanine N-methylamide • 1 sodium salt

$N^a$-[4-(Hydroxyamino)-2(R),3(RS)-diisobutylsuccinyl]-4'-aminomethyl-L-phenylalanine N-(2-hydroxyethyl)amide • 1 acetate

$N^a$-[4-(Hydroxyamino)-2(R)-isobutyl-3(R or S)-(3-phenylpropyl)-succinyl]-$N^e$-acetimidoyl-L-lysine N-(2-hydroxyethyl)amide • 1 acetate

$N^a$-[4-(Hydroxyamino)-2(R)-isobutyl-3(R or S)-methylsucciny]-L-ornithine N-cyclopropylamide • 1 acetate

$N^a$-(4-(Hydroxyamino)-2(R)-isobutyl-3(RS)-methylsuccinyl]-L-lysine N-methylamide • 1 acetate

$N^a$-[4-(Hydroxyamino)-2(R)-isobutyl-3(RS)-methylsuccinyl]-$N^e$-acetimidoyl-L-lysine N-[2-hydroxy-1(RS)-methylethyl]amide • 1 acetate

$N^a$-[4-(Hydroxyamino)-2(R)-isobutyl-3(RS)-methylsuccinyl]-$N^e$-acetimidoyl-L-lysine N-(piperidin-1-yl)amide • 1 acetate

$N^a$-[4-(Hydroxyamino)-2(R)-isobutyl-3(R or S)-methylsuccinyl]-4'-acetimidoyliminomethyl-L-phenylalanine N-methylamide • 1 acetate

$N^a$-[4-(Hydroxyamino)-2(R)-isobutyl-3(R or S)-methylsuccinyl]-4'-acetimidoyliminomethyl-L-phenylalanine N-(morpholin-4-yl)-amide • 1 acetate

$N^a$-[4-(Hydroxyamino)-2(R)-isobutyl-3(S)-hydroxysuccinyl]-4'-acetimidoyliminomethyl-L-phenylalanine N-methylamide • 1 acetate

$N^a$-[4-(Hydroxyamino)-2(R)-isobutyl-3(RS)-(3-phenylpropyl)-succinyl]-$N^e$-acetimidoyl-L-lysine N-(2-N',N'-dimethylaminoethyl)-amide • 2 acetate

$N^a$-[4-(Hydroxyamino)-2(R)-isobutyl-3(RS)-methylsuccinyl]-$N^e$-propionimidoyl-L-lysine N-methylamide • 1 acetate

$N^a$-[4-(Hydroxyamino)-2(R)-isobutyl-3(RS)-[3-(4'-guanidinophenyl)propyl]succinyl]-L-ornithine-N methylamide • 2 acetate

$N^a$-[4-(Hydroxyamino)-2(R)-isobutyl-3(R or S)-(3-phenylpropyl)-succinyl]-4'-aminomethyl-L-phenylalanine N-methylamide • 1 acetate

$N^a$-[4-(Hydroxyamino)-2(R)-isobutyl-3(R or S)-(p-aminomethylbenzyl)succinyl]-L-ornithine N-methylamide • 2 acetate

$N^a$-[4-(Hydroxyamino)-2(R)-isobutyl-3(R or S)-(p-aminomethylbenzyl)succinyl]-4'-aminomethyl-L-phenylalanine N-methylamide • 2 acetate

$N^a$-[4-(Hydroxyamino)-2(R)-isobutyl-3(R or S)-[p-(p-toluenesulfonamidomethyl)benzyl]succinyl]-L-arginine N-methylamide • 1 acetate

$N^a$-[4-(Hydroxyamino)-2(R)-isobutyl-3(R or S)-(p-phthalimidomethylbenzyl)succinyl]-L-arginine N-methylamide • 1 acetate

$N^a$-[4-(Hydroxyamino)-2(R)-isobutyl-3(R or S)-(p-methanesulfonamidomethylbenzyl)succinyl]-L-arginine N-methylamide • 1 acetate

$N^a$-[4-(Hydroxyamino)-2(R)-isobutyl-3(R or S)-(m-aminomethylbenzyl)succinyl]-L-alanine N-methylamide • 1 acetate

$N^a$-[4-(Hydroxyamino)-2(R)-isobutyl-3(R or S)-(2-phenoxyethyl)succinyl]-L-arginine N-methylamide • 1 acetate

$N^a$-[4-(Hydroxyamino)-2(R)-isobutyl-3(R or S)-(3-cyclohexylpropyl)succinyl]-L-arginine N-methylamide • 1 acetate

$N^a$-[4-(Hydroxyamino)-2(R)-(2-naphthylmethyl)-3(R or S)-(3-phenylpropyl)succinyl]-L-arginine N-methylamide • 1 acetate

$N^a$-[4-(Hydroxyamino)-2(R)-isobutyl-3(R or S)-(o-aminomethylbenzyl)succinyl]-L-alanine N-methylamide • 1 acetate

$N^a$-[4-(Hydroxyamino)-2(R)-isobutyl-3(RS)-(3-(p-aminomethylphenyl)propyl]succinyl]-L-alanine N-methylamide • 1 acetate

$N^a$-[4-(Hydroxyamino)-2(R)-isobutyl-3(R or S)-(m-isopropyliminomethylbenzyl)succinyl]-L-alanine N-methylamide • 1 acetate

$N^a$-[4-(Hydroxyamino)-2(R)-isobutyl-3(R or S)-[3-(o-aminomethylphenyl)propyl]succinyl]-L-alanine N-methylamide • 1 acetate

$N^a$-[4-(Hydroxyamino)-2(R)-isobutyl-3(R or S)-[3-(p-aminomethylphenyl)propyl]succinyl]-L-lysine N-methylamide • 2 acetate

$N^a$-[4-(Hydroxyamino)-2(R)-isobutyl-3(R or S)-[5-(acetimidoylimino)pentyl]succinyl]-L-alanine N-methylamide

$N^a$-[4-(Hydroxyamino)-2(R)-isobutyl-3(R or S)-[5-(isopropylimino)pentyl]succinyl]-L-alanine N-methylamide

$N^a$-[4-(Hydroxyamino)-2(R)-isobutyl-3(R or S)-[5-(pyridin-4-ylmethylimino)pentyl]succinyl]-L-alanine N-methylamide • 1 acetate

$N^a$-[4-(Hydroxyamino)-2(R)-isobutyl-3(R or S)-[3-(p-methoxycarbonylphenyl)propyl]succinyl]-L-lysine N-methylamide • 1 acetate

$N^a$-[4-(Hydroxyamino)-2(R)-isobutyl-3(R or S)-[2-(2-ethoxyethoxy)ethyl]succinyl]-L-lysine N-methylamide

$N^a$-[4-(Hydroxyamino)-2(R)-isobutyl-3(R or S)-[3-(p-carboxyphenyl)propyl]succinyl]-L-lysine N-methylamide

$N^a$-[4-(Hydroxyamino)-2(R)-isobutyl-3(R or S)-(8-hydroxyoctyl) • succinyl]-L-lysine N-methylamide • 1 acetate

$N^a$-[4-(Hydroxyamino)-2(R)-isobutyl-3(R or S)-(2-n-butyloxyethyl)succinyl]-L-lysine N-methylamide • 1 acetate

$N^a$-[4-(Hydroxyamino)-2(R)-isobutyl-3(R or S)-(2-isobutyloxyethyl)succinyl]-L-lysine N-methylamide • 1 acetate

$N^a$-[4-(Hydroxyamino)-2(R)-isobutyl-3(R or S)-[3-(m-methoxycarbonylphenyl)propyl]succinyl]-L-lysine N-methylamide • 1 acetate

$N^a$-[4-(Hydroxyamino)-2(R)-isobutyl-3(R or S)-[3-(p-hydroxyphenyl)propyl]succinyl]-L-lysine N-methylamide • 1 acetate

$N^a$-[4-(Hydroxyamino)-2(R)-isobutyl-3(R or S)-[3-(morpholin-4-yl)propyl]succinyl]-L-lysine N-methylamide • 1 acetate

$N^a$-[4-(Hydroxyamino)-2(R)-isopropyl-3(R or S)-(3-phenylpropyl)succinyl]-L-lysine N-methylamide • 1 acetate

$N^a$-[4-(Hydroxyamino)-2(R)-isobutyl-3(R or S)-[3-(m-carboxyphenyl)propyl]succinyl]-L-lysine N-methylamide • 1 acetate

$N^a$-[4-(Hydroxyamino)-2(R)-isobutyl-3(R or S)-[3-(piperidin-1-yl)propyl]succinyl]-L-lysine N-methylamide • 2 acetate

$N^a$-[4-(Hydroxyamino)-2(R)-isobutyl-3(R or S)-methylsuccinyl]-$N^e$-benzimidoyl-L-lysine N-methylamide • 1 acetate

$N^4$-[3-Amino-1(S)-methylcarbamoylpropyl]-$N^1$-hydroxy-2(R or S)-[3-(p-aminomethylphenyl)propyl]-3(R)-isobutylsuccinamide • 2 acetate

$N^a$-[4-(Hydroxyamino)-2(R)-isobutyl-3(R or S)-[3-(p-aminomethylphenyl)propyl]succinyl]-L-tert-leucine N-methylamide • 1 acetate

$N^a$-[4-(Hydroxyamino)-2(R)-isobutyl-3(R or S)-[3-(p-aminomethylphenyl)propyl]succinyl]-L-ornithine N-methylamide • 1 acetate

$N^a$-[4-(Hydroxyamino)-2(R)-isobutyl-3(R or S)-[3-(p-guanidomethylphenyl]propyl]succinyl]-L-ornithine N-methylamide • 2 acetate

$N^a$-[4-(Hydroxyamino)-2(R)-isobutyl-3(R or S)-[3-(3,4,4-trimethyl-2,5-dioxo-imidazolidin-1-yl)propyl]succinyl]-L-lysine N-methylamide

$N^a$-[4-(Hydroxyamino)-3(S)-isobutyl-2(R or S)-(8-hydroxyoctyl)-succinyl]-L-lysine N-methylamide • 1 acetate

$N^a$-[4-(Hydroxyamino)-2(R),3(R or S)-diisobutylsuccinyl]-4'-aminomethyl-L-phenylalanine N-(2-carboxyethyl)amide • 1 acetate

$N^a$-[4-(Hydroxyamino)-2(R),3(R or S)-diisobutylsuccinyl]-4'-acetimidoyliminomethyl-L-phenylalanine N-(2-carboxyethyl)amide • 1 acetate

$N^4$-[2-Amino-2-methyl-1(RS)-methylcarbamoylpropyl]-$N^1$-hydroxy-2(R or S)-[3-(p-aminomethylphenyl)propyl]-3(R)-isopropylsuccinamide • 2 acetate

$N^a$-[4-(Hydroxyamino)-2(R)-isobutyl-3(R or S)-methylsuccinyl]-4'-aminomethyl-L-phenylalanine N-(morpholin-4-yl)amide • 1 acetate

$N^a$-[4-(Hydroxyamino)-2(R)-isobutyl-3(S)-hydroxysuccinyl]-4'-aminomethyl-L-phenylalanine N-methylamide • 1 acetate

$N^a$-[4-(Hydroxyamino)-2(R),3(R or S)-diisobutylsuccinyl]-4'-aminomethyl-L-phenylalanine N-(2-N',N'-dimethylaminoethyl)amide • 2 acetate

$N^a$-[4-(Hydroxyamino)-2(R),3(R or S)-diisobutylsuccinyl]-4'-acetimidoyliminomethyl-L-phenylalanine N-(2-N',N'-dimethylaminoethyl)amide • 2 acetate

$N^a$-[4-(Hydroxyamino)-2(R),3(RS)-diisobutylsuccinyl]-4'-acetimidoyliminomethyl-L-phenylalanine N-(2-hydroxyethyl)amide • 2 acetate

$N^a$-[4-(Hydroxyamino)-2(R)-isobutyl-3(R or S)-[3-(p-aminophenyl)propyl]succinyl]-$N^e$-acetimidoyl-L-lysine N-(2-N',N'-dimethylaminoethyl)amide • 3 acetate

$N^a$-[4-(Hydroxyamino)-2(R),3(RS)-diisobutylsuccinyl]-4'-acetimidoyliminomethyl-L-phenylalanine N-(2-hydroxy-1,1-dimethylethyl)amide • 1 acetate

$N^4$-[3-Amino-2,2-dimethyl-1(RS)-methylcarbamoylpropyl]-$N^1$-hydroxy-2(R or S)-[3-(p-aminomethylphenyl)propyl]-3(R)-isopropyl-succinamide • 2 acetate

$N^4$-[2-Amino-1(S)-methylcarbamoylpropyl]-$N^1$-hydroxy-2(R or S)-[3-(p-aminomethylphenyl)propyl]-3(R)-isopropyl succinamide • 2 acetate

$N^a$-[4-(Hydroxyamino)-2(R)-isopropyl-3(R or S)-(3-(p-aminomethylphenyl)propyl]succinyl]-O-(2,3,4,6-tetra-O-acetyl-β -D-glucopyranosyl)-L-serine N-methylamide • 1 acetate

$N^a$-[4-(Hydroxyamino)-2(R)-isopropyl-3(R or S)-[3-(p-aminomethylphenyl)propyl]succinyl]-O-(β -D-glucopyranosyl)-L-serine N-methylamide • 1 acetate

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-aminomethylphenyl)propyl]-3(R)-isopropylsuccinamide • 1 acetate

$N^4$-[2-Amino-2-methyl-1(RS)-methylcarbamoylpropyl]-$N^1$-hydroxy-2(R or S)-[3-(p-guanidomethylphenyl)propyl]-3(R)-isopropylsuccinamide • 2 acetate

$N^4$-[3-Amino-2,2-dimethyl-1(RS)-methylcarbamoylpropyl]-$N^1$-hydroxy-2(R or S)-[3-(p-guanidomethylphenyl)propyl]-3(R)-isopropylsuccinamide • 2 acetate

$N^4$-[2-Amino-1(S)-methylcarbamoylpropyl]-$N^1$-hydroxy-2(R or S)-[3-(p-guanidomethylphenyl)propyl]-3(R)-isopropylsuccinamide • 2 acetate

$N^a$-[4-(Hydroxyamino)-2(R)-isopropyl-3(R or S)-[3-(p-guanidomethylphenyl)propyl]succinyl]-O-(2,3,4,6-tetra-O-acetyl-β -D-glucopyranosyl)-L-serine N-methylamide • 1 acetate

$N^a$-[4-(Hydroxyamino)-2(R)-isopropyl-3(R or S)-[3-(p-guanidomethylphenyl)propyl]succinyl]-O-(β -D-glucopyranosyl)-L-serine N-methylamide • 1 acetate

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-guanidomethylphenyl)propyl]-3(R)-isopropylsuccinamide • 1 acetate

$N^a$-[4-(Hydroxyamino)-2(R)-isobutyl-3(R or S)-[3-(p-carbamoylphenyl)propyl]succinyl]-L-lysine N-methylamide • 1 acetate

$N^a$-[4-(Hydroxyamino)-2(R)-isopropyl-3(RS)-[3-(p-aminomethylphenyl)propyl]succinyl]-L-ornithine N-methylamide • 2 acetate

$N^4$-[2-Amino-1(S)-methylcarbamoylethyl]-$N^1$-hydroxy-2(R or S)-[3-(p-aminomethylphenyl)propyl]-3(R)-isopropylsuccinamide • 2 acetate, and the like,

have superior inhibitory actions on MMPs and/or TNF-α convertases and remarkably improved bioavailability per oral administration, in comparison with prior art compounds, whereby they are readily applicable to diseases and/or disorders associated with the degradation of tissues and useful with a lot of promise in view of prophylactic and/or therapeutic treatments.

Among the compounds produced according to the present invention, those which have an unprotected or optionally protected phosphono moiety as their functional substituent are expected to have inhibitory activity on the destruction of bones, whereby they are believed to be promising agents for the prophylactic and/or therapeutic treatment of osteoporosis and other diseases and also expected to be applied for the prophylactic and/or therapeutic treatment of diseases and/or disorders associated with the degradation of tissues.

Described below are assays for biological activities of the compounds according to the present invention, formulations, dosage forms and dosage levels thereof, and miscellaneous matters. The efficacy of the compounds represented by the formula (I) according to the present invention as inhibitors of human fibroblast collagenase (metalloproteinase involved in the breakdown of tissues) is determined by a procedure based on the method of Y. Murawaki et al., Journal of Hepatology, 18, p328-334 (1993). The efficacy of the compounds (I) of this invention as inhibitors of human fibroblast stromelysin is determined by a procedure based on the method of S. S. Twining, Anal. Biochem., 143, p30 (1984). The assay results obtained are shown hereinbelow, together with biological examples which illustrate embodiments of the assay protocols.

When employed as pharmaceutical agents, the compounds (I) and salts thereof may be administered in the form of a convenient pharmaceutical composition or formulation suitable for oral, topical, parenteral application, or the like. Any of dosage forms (including those for inhalation and rectal administration) may be selected depending on purpose. Suitable dosage forms of the pharmaceutical compositions or formulations may include powders, granules, tablets, pills, capsules, injections, syrups, emulsions, elixirs, suspensions, solutions, conditioned gels, etc. The pharmaceutical composition or formulation may comprise at least one of said compounds (active components) of the present invention or a salt thereof alone or in admixture with a pharmaceutically acceptable carrier, adjuvant, vehicle, excipient, diluent, etc.

The pharmaceutical compositions can be formulated in accordance with conventional techniques.

The formulations suitable for oral application include powders, granules, tablets, pills, capsules, etc. as mentioned above. In such dosage forms, the active compound may be admixed for example with at least one member selected from the group consisting of sucrose, lactose, cellulose sugar, mannitol, maltitol, dextran, starches, agar, alginates, chitins, chitosans, pectins, gum tragacanth, acacia (gum arabic), gelatins, collagens, casein, albumin, synthetic or semi-synthetic polymers and glycerides. Such dosage forms may also contain any of various pharmaceutically acceptable additives in addition to the above ingredient as customarily conducted. Examples of such additives are inert diluents, lubricants such as magnesium stearate, preservatives such as parabens and sorbic acid, antioxidizing agents such as ascorbic acid, $\alpha$ -tocopherol and cysteine, disintegrating agents, binders, thickening agents, buffering agents, sweetening agents, flavoring agents, perfuming agents, and the like. The tablets and pills can also be prepared further by enteric coating.

Representatives of such formulations are tablets and capsules, each of which is in the form of a dose unit suitable for a single administration and may be manufactured by ordinary techniques wherein customarily acceptable additives as listed below are contained. The tablet may be coated by customarily known techniques for conventional pharmaceutical practices.

(1) ordinary vehicles which serve as binders, including syrup, acacia, gelatin, sorbitol, gum tragacanth, or polyvinylpyrrolidone;
(2) fillers, including lactose, sucrose, corn starch, calcium phosphate, sorbitol, and glycine;
(3) tablet lubricants, including for example magnesium stearate, talc, polyethylene glycol, and silica; and
(4) disintegrants such as potato starch or acceptable wetting agents such as sodium lauryl sulfate.

The fluid formulations suitable for oral application may contain an inert diluent ordinarily used in the art, such as water. Representatives of the oral fluid formulations may be prepared in the form of a suspension, solution, emulsion, syrup, or elixir, wherein water or oil is contained as a component, or provided in the form of a dry product for reconstitution into a liquid drug by addition of water or a suitable vehicle just prior to use.

Such fluid formulations may also contain any of customary additives, including for example suspending agents such as sorbitol, syrup, methylcellulose, glucose syrup, gelatin and hydrogenated dietary oils; emulsifiers such as lecithin, sorbitan monooleate and acacia; non-aqueous vehicles (including dietary oils) such as, for example, almond oil, fractionated cocoanut oil, glycerin, and oily esters of propylene glycol and ethyl alcohol; preservatives including for example ethyl p-hydroxybenzoate, propyl p-hydroxybenzoate, and sorbic acid; and, as required, ordinary flavoring agents, and/or coloring agents.

An oral dose suitable for a single administration may contain from about 1 mg to 10 g, preferably from about 10 mg to 1 g of the compound (I). Actual dosage levels of the active ingredients in the pharmaceutical compositions of the present invention may be varied depending on the disease being treated, the severity of the symptom being treated, the general condition and prior medical history of a particular patient being treated, the route and cycle of administration, etc. A suitable daily dose will vary depending on the condition of the patient, but general dosage levels of about 0.01 to 500 mg, more preferably of about 0.1 to 300 mg of the compound (I) per kilogram of body weight per day are suitably administered to a mammalian patient, for example an adult person.

Specific dose levels and administration cycles for any particular patient will be employed depending upon a variety of factors including the sex, age, body weight, general health, diet, time of administration, route of administration, rate of excretion, drug combination, the severity of the particular disease undergoing therapy, and others.

The pharmaceutical drugs for topical application (including painting) to skin can be prepared in the form of a solution or suspension utilizing suitably sterilized water or non-aqueous vehicles. The additives used include buffering agents such as sodium bisulfite and disodium edetate; preservatives including antiseptic, antimicrobial and antifungal agents such as acetic acid, phenylmercuric nitrate, benzalkonium chloride and chlorhexidine; and thickeners such as hypromellose. The selected dosage levels for topical administration will vary depending on the size of the diseased site being treated, but a single dose (per eye) for ophthalmic administration ranges from 0.01 to 100mg of the compound (I).

The active component can be administered parenterally using a sterilized medium. As used herein, the term "parenteral" administration refers to modes of administration which include subcutaneous, intravenous, intramuscular, and intraperitoneal injection, instillation, and the like. Injectable formulations including for example sterile aqueous or oily suspensions for injection, etc. may be prepared using suitable dispersing agents, moistening agents, suspending agents, etc. by known techniques in the art. The sterile formulations for injection may also include sterile injectable solutions or suspensions, such as aqueous solutions, formed in admixture with parenterally-administrable non-toxic diluents or solvents. Utilizable vehicles or acceptable solvents include water, Ringer's solution, isotonic saline, etc. In addition, sterile non-volatile oils may also be used as solvents or suspending media, For these formulations, any non-volatile oils and fatty acids can be employed. Such vehicles and solvents also include natural, synthetic or semi-synthetic fatty oils or acids, and natural, synthetic or semi-synthetic mono-, di-, or triglycerides.

The suppositories suitable for rectal administration can be prepared by admixing the drug with suitable non-irritative excipients. Examples of the excipients are those which are solid at room temperature but liquid at rectal temperature wherein such substances melt in the rectum to deliver a drug, such as cacao butter and polyethylene glycols. The compound, depending on the vehicle and concentration used, can be either suspended or dissolved in the vehicle. Adjuvants such as a local anesthetic, preservative and buffering agent can be dissolved in the vehicle. For application in the treatment of arthritides such as osteoarthritis, and chronic rheumatoid arthritis, the active compounds of the present invention can be administered orally or injected intra-articularly to a diseased joint. In general, a daily dose for application to a mammal weighing 70 kg in the treatment ranges from 0.001 mg to 6 g of the compound (I).

The present invention further relates to pharmaceutically-acceptable packs (and/or containers or packages) and kits comprising one or more containers filled with one or more of the ingredients of the aforementioned compositions of the invention. Associated with such a single or plural containers can be a notice (attached document) in the form prescribed by a governmental agency regulating the manufacture, use or sale of pharmaceuticals or biological products, reflecting approval by the agency of the manufacture, use or sale of the product for human administration.

## EXAMPLES

Described below are examples, i.e., preparation examples, biological assay examples and formulation examples, of the present invention which are provided only for illustrative purposes, and not to limit the scope of the present invention. All the examples were or can be practiced using standard techniques well or conventionally known to those of ordinary skill in the art unless otherwise specified. In the examples (including the preparation examples, etc.) below as well as elsewhere in the specification, the following abbreviations are intended to have the meanings set forth below.

| | |
|---|---|
| DMF; | N,N-dimethylformamide |
| EDC; | 1-(3-dimethylaminopropyl)-3-ethylcarbodiimide hydrochloride |
| HOBT; | 1-hydroxybenzotriazole |
| TEA; | triethylamine |
| THF; | tetrahydrofuran |
| Et; | ethyl |
| Boc; | tert-butyloxycarbonyl |
| Bn; | benzyl |
| Me; | methyl |
| Cl-Z; | 2-chlorobenzyloxycarbonyl |
| Z; | benzyloxycarbonyl |
| tBu; | tert-butyl |
| Tce; | 2,2,2-trichloroethyl |

Na; sodium

Preparation Example 1

$N^a$-tert-Butyloxycarbonyl-$N^e$-benzyloxycarbonyl-L-lysine N-methylamide (Compound No. 1)

To a solution of $N^a$-tert-butyloxycarbonyl-$N^e$-benzyloxycarbonyl-L-lysine (10.0 g, 26.3 mmol) in DMF (50 ml) was added aqueous methylamine hydrochloride (2.13 g, 31.5 mmol/ 5 ml), HOBT (3.55 g, 26.3 mmol), EDC (6.04 g, 31.5 mmol), and TEA (4.39 ml, 31.5 mmol) sequentially with stirring at -15°C. The mixture was stirred for 1 hour at -15°C and for another 15 hours at room temperature, and evaporated under reduced pressure. AcOEt (200 ml) was added to the residue and the mixture was partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure to give the title compound as a white solid (8.20 g, 20.8 mmol, yield 80%).

$^1$H-NMR ($CDCl_3$)δ ppm; 1.3-1.9 (15H, s + m, C$\underline{(CH_3)_3}$ + CH$\underline{(CH_2)_3}$), 2.73 (3H, d, J=4.7Hz, NH-$\underline{CH_3}$), 3.30 (2H, m, -OC(=O)NH-$\underline{CH_2}$), 4.12 (1H, m, NH-$\underline{CH}$-CO), 5.08 (2H, s, O-$\underline{CH_2}$-Ph), 5.60 (1H, m, NH), 6.61 (1H, m, NH), 7.2-7.5 (5H, m, aromatic-H).

Preparation Example 2

$N^a$-tert-Butyloxycarbonyl-L-4'-cyanophenylalanine N-(morpholin-4-yl)amide (Compound No. 2)

The procedure of Preparation Example 1 except omitting the partition and washing with 1N hydrochloric acid was repeated using $N^a$-tert-butyloxycarbony-L-4'-cyanophenylalanine and 4-aminomorpholine to provide the title compound as a white solid (yield 50%), m.p.; 181-185°C, Rf value; 0.25 (chloroform:methanol = 20:1).

$^1$H-NMR ($CDCl_3$)δ ppm; 1.4 (9H, s, C$\underline{(CH_3)_3}$), 2.6-2.7 (4H, m, N-$\underline{CH_2}$ x 2), 2.9-3.2 (2H, m, $C_6H_4$-$\underline{CH_2}$), 3.4-4.0 (4H, m, O-$\underline{CH_2}$ x 2), 4.21 (1H, m, NH-$\underline{CH}$-CO), 5.09 and 5.19 (2H, m, NH x 2), 7.3-7.6 (4H, m, aromatic-H).

Preparation Example 3

$N^a$-Benzyloxycarbonyl-$N^e$-tert-butyloxycarbonyl-L-lysine N-(2-N',N'-dimethylaminoethyl)amide (Compound No. 3)

The procedure of Preparation Example 2 was repeated using $N^a$-benzyloxycarbonyl-$N^e$-tert-butyloxycarbonyl-L-lysine and N,N-dimethylethylenediamine to provide the title compound as a white solid (yield 50%), m.p.; 61°C, Rf value; 0.26 (chloroform:methanol= 10:1).

$^1$H-NMR ($CDCl_3$)δ ppm; 1.3-1.9 (15H, s + m, C$\underline{(CH_3)_3}$ + CH$\underline{(CH_2)_3}$), 2.21 (6H, s x 2, N-$\underline{CH_3}$ x 2), 2.40 (2H, m, $\underline{CH_2}$-N$(CH_3)_2$), 3.09 (2H, m, NH-$\underline{CH_2}$-$CH_2$-N), 3.32 (2H, m, -OC(=O)NH-$\underline{CH_2}$-), 4.13 (1H, m, NH-$\underline{CH}$-CO), 4.66 (1H, m, NH), 5.10 (2H, s, O-$\underline{CH_2}$-Ph), 5.60 (1H, m, NH), 6.61 (1H, m, NH), 7.3-7.4 (5H, m, aromatic-H).

Preparation Example 4

$N^a$-Benzyloxycarbonyl-$N^e$-tert-butyloxycarbonyl-L-lysine N-(piperidin-1-yl)amide (Compound No. 4)

The procedure of Preparation Example 2 was repeated using $N^a$-benzyloxycarbonyl-$N^e$-tert-butyloxycarbonyl-L-lysine and 1-aminopiperidine to provide the title compound as a white solid (yield 83%), m.p.; 128-131°C, Rf value; 0.17 (chloroform:methanol= 20:1).

$^1$H-NMR ($CDCl_3$)δ ppm; 1.2-1.8 (21H, s + m, C$\underline{(CH_3)_3}$ + CH$\underline{(CH_2)_3}$ + -$\underline{(CH_2)_3}$- of piperidine), 2.7-3.1 (6H, m, N-$\underline{CH_2}$ + N-$\underline{CH_2}$ x 2), 3.98 (1H, m, NH-$\underline{CH}$-CO), 4.62 and 4.86 (2H, m, NH x 2), 5.10 (2H, s, O-$\underline{CH_2}$-Ph), 6.27 (1H, m, NH), 7.3-7.4 (5H, m, aromatic-H).

Preparation Example 5

$N^a$-tert-Butyloxycarbonyl-L-4'-cyanophenylalanine N-methylamide (Compound No. 5)

The procedure of Preparation Example 1 was repeated using $N^a$-tert-butyloxycarbonyl-L-4'-cyanophenylalanine and methylamine hydrochloride to provide the title compound as a white solid (yield 84%), m.p.; 165-167°C, Rf value; 0.55 (chloroform: methanol= 10:1).

$^1$H-NMR ($CDCl_3$)δ ppm; 1.38 (9H, s, $C(\underline{CH_3})_3$), 2.75 (3H, d, J=4.8Hz, NH-$\underline{CH_3}$), 3.02 and 3.18 (1H each, m, $C_6H_4$-$\underline{CH_2}$), 4.34 (1H, m, NH-$\underline{CH}$-CO), 5.08 (1H, m, NH), 6.08 (1H, m, NH), 7.2-7.6 (4H, m, aromatic-H).

Preparation Example 6

$N^a$-Benzyloxycarbonyl-$N^e$-tert-butyloxycarbonyl-L-lysine N-(2-hydroxy-1(RS)-methylethyl)amide (Compound No. 6)

The procedure of Preparation Example 1 was repeated using $N^a$-benzyloxycarbonyl-$N^e$-tert-butyloxycarbonyl-L-lysine and DL-2-amino-1-propanol to provide the title compound as a white solid (yield 87%), m.p.; 115-116°C, Rf value; 0.096 (chloroform:methanol= 20:1).

$^1$H-NMR ($CDCl_3$)δ ppm; 1.14 (3H, d, J=6.6Hz, CH-$\underline{CH_3}$), 1.30-1.90 (15H, s + m, $C(\underline{CH_3})_3$ + $CH(\underline{CH_2})_3$), 3.08 (2H, m, NH-$\underline{CH_2}$), 3.25 (1H, m, $\underline{CH}$-$CH_3$), 3.47 and 3.64 (1H each, m, $\underline{CH_2}$-OH), 4.11 (1H, m, NH-$\underline{CH}$-CO), 4.73 (1H, m, OH), 5.08 (2H, s, O-$\underline{CH_2}$-Ph), 5.78 (1H, m, NH), 6.42 and 6.56 (2H, m, NH x 2), 7.35 (5H, m, aromatic-H).

Preparation Example 7

$N^a$ tert-Butyloxycarbonyl-$N^d$-benzyloxycarbonyl-L-ornithine N-methylamide (Compound No. 7)

The procedure of Preparation Example 1 was repeated using $N^a$-tert-butyloxycarbonyl-$N^d$-benzyloxycarbonyl-L-ornithine and methylamine hydrochloride to provide the title compound as a white solid (yield 92%), m.p.; 141-143°C, Rf value; 0.22 (chloroform:methanol= 20:1).

$^1$H-NMR ($CDCl_3$)δ ppm; 1.41 (9H, s, $C(\underline{CH_3})_3$), 1.57 (2H, m, $CH_2$-$\underline{CH_2}$-$CH_2$), 1.77 (2H, m, CH-$\underline{CH_2}$-), 2.75 (3H, d, J=4.7 Hz, NH-$\underline{CH_3}$), 3.17 and 3.35 (1H each , m, NH-$\underline{CH_2}$), 4.18 (1H, m, NH-$\underline{CH}$-CO), 4.9-5.3 (4H, m, O-$\underline{CH_2}$-Ph + NH x 2), 6.42 (1H, m, NH), 7.2-7.5 (5H, m, aromatic-H).

Preparation Example 8

$N^a$-tert-Butyloxycarbonyl-$N^d$-benzyloxycarbonyl-L-ornithine N-cyclopropylamide (Compound No. 8)

The procedure of Preparation Example 1 was repeated using $N^a$-tert-butyloxycarbonyl-$N^d$-benzyloxycarbonyl-L-ornithine and cyclopropylamine to provide the title compound as a white solid (yield 95%), m.p.; 146-147°C, Rf value; 0.27 (chloroform:methanol= 20:1).

$^1$H-NMR ($CDCl_3$)δ ppm; 0.48 (2H, m, $\underline{CH_2}$ of cyclopropyl), 0.74 (2H, m, $\underline{CH_2}$ of cyclopropyl), 1.43 (9H, s, $C(\underline{CH_3})_3$), 1.56(4H, m, CH-$(\underline{CH_2})_2$-), 2.69 (1H, m, CH of cyclopropyl), 3.16 and 3.37 (1H each, m, NH-$\underline{CH_2}$) 4.14 (1H, m, NH-$\underline{CH}$-CO), 5.09 (2H, m, O-$\underline{CH_2}$-Ph), 5.02 and 5.22 (1H each, m, NH x 2), 6.60 (1H, m, NH), 7.2-7.5 (5H, m, aromatic-H).

Preparation Example 9

$N^a$-tert-Butyloxycarbonyl-$N^g$,$N^g$-dibenzyloxycarbonyl-L-arginine N-methylamide (Compound No. 9)

The procedure of Preparation Example 1 was repeated using $N^a$-tert-butyloxycarbonyl-$N^g$,$N^g$-dibenzyloxycarbonyl-L-arginine and methylamine to provide the title compound as a white solid (yield 96%), m.p.; 154-156°C, Rf value; 0.35 (chloroform: methanol= 20:1).

$^1$H-NMR ($CDCl_3$)δ ppm; 1.44 (9H, s, $C(\underline{CH_3})_3$), 1.71 (4H, m, CH-$(\underline{CH_2})_2$-), 2.44 (3H, d, J=4.8Hz, NH-$\underline{CH_3}$), 3.80 and 4.10 (2H, m, NH-$\underline{CH_2}$), 4.20 (1H, m, NH-$\underline{CH}$-CO), 5.1-5.3 (4H, m, O-$\underline{CH_2}$-Ph x 2), 5.72 (1H, m, NH), 6.55 (1H, m,

NH), 7.3-7.5 (10H, m, aromatic-H), 9.33 and 9.43 (2H, m, NH x 2).

Preparation Example 10

2(S)-tert-Butyloxycarbonylamino-4-$N^1$,$N^2$-dibenzyloxycarbonylguanidinobutanoic acid N-methylamide (Compound No. 10)

To a solution of 4-amino-2(S)-tert-butyloxycarbonylaminobutanoic acid N-methylamide (15.2 g, 65.7 mmol) in DMF (300 ml) was added TEA (9.16 ml, 65.7 mmol) and 1H-pyrazole-N,N'-bis(benzyloxycarbonyl)carboxamidine (29.8 g, 78.8 mmol), and the mixture was stirred for 15 hours at room temperature. AcOEt (1000 ml) was added to the reaction mixture which was then partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure. The resultant reaction mixture was purified by column chromatography (silica gel; 800 g, eluted with a mixture of benzene:AcOEt= 5:1 to 3:2) to give the title compound as a white solid (17.2 g, 48%), m.p.; 128°C, Rf value; 0.68 (chloroform:methanol= 10:1).

$^1$H-NMR ($CDCl_3$)δ ppm; 1.40 (9H, s, C$\underline{(CH_3)_3}$), 1.8-2.0 (2H, m, CH-$\underline{(CH_2)}$-), 2.60 (3H, d, J = 4.8 Hz, NH-$\underline{CH_3}$), 3.17 and 3.82 (1H each, m, NH-$\underline{CH_2}$), 4.13 (1H, m, NH-$\underline{CH}$-CO), 5.09 and 5.19 (2H each, m, O-$\underline{CH_2}$-Ph x 2), 5.64 (1H, m, NH), 6.55 (1H, m, NH), 7.2-7.4 (11H, m, NH + aromatic-H), 8.24 and 8.60 (2H, m, NH x 2).

Preparation Example 11

$N^a$-Benzyloxycarbonyl-L-4'-[tetraethyl bis(phosphonyl)methylimino]phenylalanine N-methylamide (Compound No. 11)

A mixture of $N^a$-benzyloxycarbonyl-L-4'-aminophenylalanine N-methylamide (3.00 g, 9.16 mmol), ethyl orthoformate (1.83 ml, 11.0 mmol) and diethyl phosphite (4.72 ml, 36.6 mmol) was stirred for 2 hours at 140 to 160°C. The unreacted diethyl phosphite and EtOH which produced during the reaction were removed by evaporation under reduced pressure and the reaction mixture was purified by column chromatography (silica gel; 500 g, eluted with a mixture of $CH_2Cl_2$:MeOH = 40:1 to 20:1) to give the title compound as a pale yellow solid (3.97 g, 71% ), m.p.; 126-128°C, Rf value; 0.44 (chloroform:methanol= 10:1).

$^1$H-NMR ($CDCl_3$)δ ppm; 1.0-1.4 (12H, m, $CH_2$-$\underline{CH_3}$ x 4), 2.70 (3H, d, J=4.8Hz, NH-$\underline{CH_3}$), 2.92 (2H, brd, J=6.8Hz, $C_6H_4$-$\underline{CH_2}$), 3.8-4.5 (10H, m, $\underline{CH_2}$-$CH_3$ x 4 + NH-$\underline{CH}$-CO + P-$\underline{CH}$-P), 5.07 (2H, s, O-$\underline{CH_2}$-Ph), 5.40 (1H, m, NH), 5.90 (1H, m, NH), 6.5-7.2 (4H, m, $\underline{C_6H_4}$), 7.2-7.6 (10H, m, aromatic-H + NH).

Preparation Example 12

$N^a$-Benzyloxycarbonyl-L-4'-[tetramethyl bis(phosphonyl)methylimino]phenylalanine N-methylamide (Compound No. 12)

The procedure of Preparation Example 11 was repeated using $N^a$-benzyloxycarbonyl-L-4'-aminophenylalanine N-methylamide (115 g, 350 mmol), ethyl orthoformate (78 g, 530 mmol) and dimethyl phosphite (154 g, 1.40 mol) to provide the title compound as a pale yellow solid (157 g, 80% ), m.p.; 150-153°C, Rf value; 0.41 (chloroform:methanol= 10:1).

$^1$H-NMR ($CD_3OD$)δ ppm;2.67 (3H, s, NH-$\underline{CH_3}$), 2.73 and 2.98 (1H each, m, $C_6H_4$-$\underline{CH_2}$), 3.73-3.78 (12H, m, O-$\underline{CH_3}$ x 4), 4.25 (1H, m, NH-$\underline{CH}$-CO), 4.6-4.7 (1H, m, P-$\underline{CH}$-P), 5.02 (2H, s, O-$\underline{CH_2}$-Ph), 6.7-7.1 (4H, m, $\underline{C_6H_4}$), 7.2-7.3 (5H, m, aromatic-H).

Preparation Example 13

$N^a$-tert-Butyloxycarbonyl-$N^e$-2-chlorobenzyloxycarbonyl-L-lysine N-methylamide (Compound No. 13)

The procedure of Preparation Example 1 was repeated using $N^a$-tert-butyloxycarbonyl-$N^e$-2-chlorobenzyloxycarbonyl-L-lysine and methylamine hydrochloride to provide the title compound as a white solid (yield 94%), m.p.; 109-110°C, Rf value; 0.48 (chloroform:methanol= 10:1).

$^1$H-NMR ($CDCl_3$)δ ppm; 1.3-1.9 (15H, s + m, C$\underline{(CH_3)_3}$ + CH-$\underline{(CH_2)_3}$-2.80 (3H, d, J=4.8Hz, NH-$\underline{CH_3}$), 3.20 (2H, m,

NH-$\underline{CH_2}$), 4.04 (1H, m, NH-$\underline{CH}$-CO), 4.95 and 5.14 (1H each, m, NH x 2), 5.21 (2H, s, O-$\underline{CH_2}$-$C_6H_4$), 6.21 (1H, m, NH), 7.2-7.5 (4H, m, aromatic-H).

Preparation Example 14

$N^a$-tert-Butyloxycarbonyl-L-4'-cyanophenylalanine N-(2-benzyloxyethyl)amide (Compound No. 14)

The procedure of Preparation Example 1 was repeated using $N^a$-tert-butyloxycarbonyl-L-4'-cyanophenylalanine and O-benzylaminoethanol to provide the title compound as a white solid (yield 90%), m.p.; 99-102°C, Rf value; 0.55 (chloroform: methanol= 20:1).

$^1$H-NMR ($CDCl_3$)δ ppm; 1.39 (9H, s, C$\underline{(CH_3)_3}$), 3.03 and 3.16 (1H each, m, $C_6H_4$-$\underline{CH_2}$), 3.4-3.5 (4H, m, NH-$\underline{CH_2}$-$\underline{CH_2}$-O), 4.33 (1H, m, NH-$\underline{CH}$-CO), 4.46 (2H, s, O-$\underline{CH_2}$-Ph), 5.01 (1H, m, NH), 6.22 (1H, m, NH), 7.2-7.6 (9H, m, aromatic-H).

Preparation Example 15

$N^a$-tert-Butyloxycarbonyl-$N^e$-2-chlorobenzyloxycarbonyl-L-lysine N-(2-benzyloxyethyl)amide (Compound No. 15)

The procedure of Preparation Example 1 was repeated using $N^a$-tert-butyloxycarbonyl-$N^e$-2-chlorobenzyloxycarbonyl-L-lysine and O-benzylaminoethanol to provide the title compound as a white solid (yield 99%), m.p.; 69-71°C, Rf value; 0.60 (chloroform:methanol= 10:1).

$^1$H-NMR ($CDCl_3$)δ ppm; 1.2-1.9 (15H, s + m, C$\underline{(CH_3)_3}$ + CH-$\underline{(CH_2)_3}$-), 3.18 (2H, m, NH-$\underline{CH_2}$), 3.4-3.6 (4H, m, NH-$\underline{CH_2}$-$\underline{CH_2}$-O), 4.04 (1H, m, NH-$\underline{CH}$-CO), 4.51 (2H, 5, O-$\underline{CH_2}$-Ph), 4.93 and 5.12 (1H each, m, NH x 2), 5.21 (2H, s, C(=O)O-$\underline{CH_2}$-$C_6H_4$), 6.44 (1H, m, NH), 7.2-7.5 (9H, m, aromatic-H).

Preparation Example 16

3(RS)-tert-Butyloxycarbonyl-6-phenyl-2(R)-isobutylhexanoic acid (Compound No. 16)

a) Benzyl 2(R)-bromo-4-methylpentanoate (Compound No. 16-a)

To a solution of 2(R)-bromo-4-methylpentanoic acid (28.5 g, 146 mmol), benzyl alcohol (18.1 ml, 175 mmol), and 4-dimethylaminopyridine (1.90 g, 14.6 mmol) in dichloromethane (140 ml) was added EDC (35.6 g, 175 mmol) with stirring while ice cooling. The mixture was stirred for 1 hour while ice cooling, and further overnight at room temperature. The resultant mixture was partitioned and washed successively with water, saturated aqueous sodium bicarbonate, and saturated aqueous sodium chloride (twice for each). The organic layer was dried over anhydrous magnesium sulfate, evaporated under reduced pressure, and purified by column chromatography (silica gel; 700 g, eluted with a mixture of n-hexane:AcOEt = 40:1) to give the title compound as a colorless oil (32.0 g, 77%), specific rotation $[\alpha]_D$ = +31.8° (c=1.0, MeOH), Rf value; 0.48 (AcOEt:n-hexane= 1:5).

$^1$H-NMR ($CDCl_3$)δ ppm; 0.9 (6H, 2 x d, J= 6.5Hz, CH$\underline{(CH_3)_2}$), 1.67 (1H, m, $(CH_3)_2$$\underline{CH}$), 1.90 (2H, m, $(CH_3)_2$CH-$\underline{CH_2}$), 4.30 (1H, t, J= 7Hz, -$\underline{CH}$-Br), 5.2 (2H, 5, $\underline{CH_2}$-Ph), 7.32 (5H, s, aromatic-H).

b) Dibenzyl 3(RS)-tert-butyloxycarbonyl-2(R)-isobutylsuccinate (Compound No. 16-b)

To a solution of benzyl tert-butylmalonate (24.9 g, 99.6 mmol) in DMF (60 ml) was added potassium tert-butoxide (13.4 g, 120 mmol) portionwise with stirring at 0°C. The mixture was stirred for 1 hour at room temperature and re-cooled to 0°C. A solution of Compound No. 16-a (28.4 g, 99.6 mmol) in DMF (60 ml) was added dropwise to the cooled mixture over a period of 1 hour. After stirring for 15 hours at 5°C, AcOEt (2 L) was added to the reaction mixture which was then partitioned and washed successively with saturated aqueous sodium chloride, 1N hydrochloric acid, saturated aqueous sodium bicarbonate, and saturated aqueous sodium chloride (twice for each). The organic layer was dried over anhydrous magnesium sulfate, evaporated under reduced pressure, and purified by column chromatography (silica gel; 750 g, eluted with a mixture of n-hexane:AcOEt= 20:1) to give the title compound as a colorless oil (40.0 g, 89%), specific rotation $[\alpha]_D$ = +16.7° (c=1.0, MeOH), Rf value; 0.56 (AcOEt:n-hexane= 1:5).

$^1$H-NMR ($CDCl_3$)δ ppm; 0.82 (6H, 2 x d, J= 10Hz, CH$\underline{(CH_3)_2}$), 1.15-1.8 (12H, 2 x s + m, $(CH_3)_2$$\underline{CH\text{-}CH_2}$, +

$C(\underline{CH_3})_3$), 3.2 (1H, m, $CH_2$-$\underline{CH}$-CO), 3.7 (1H, m, CO-$\underline{CH}$-CO), 5.1 (4H, m, $\underline{CH_2}$-Ph x 2), 7.32 (10H, s, aromatic-H).

c) Dibenzyl 3(RS)-tert-butyloxycarbonyl-3-cinnamyl-2(R)-isobutylsuccinate (Compound No. 16-c)

To a solution of Compound No. 16-b (9.49 g, 20.9 mmol) in DMF (100 ml) was added 60% sodium hydride (1.0 g, 25.1 mmol) portionwise with stirring at room temperature. The mixture was stirred for 2 hours at room temperature and cooled to 0°C. Cinnamyl bromide (5.35 g, 27.2 mmol) was added portionwise to the cooled mixture which was then stirred for 15 hours at 5°C. The solvent was evaporated under reduced pressure, and AcOEt (500 ml) was added to the residue. The mixture was partitioned and washed successively with saturated aqueous sodium chloride, 1N hydrochloric acid, saturated aqueous sodium bicarbonate, and saturated aqueous sodium chloride (twice for each). The organic layer was dried over anhydrous magnesium sulfate, evaporated under reduced pressure, and purified by column chromatography (silica gel; 700 g, eluted with a mixture of n-hexane:AcOEt= 20:1) to give the title compound as a colorless oil (10.9 g, 91%), Rf value; 0.34 (AcOEt:n-hexane = 1:9).

$^1$H-NMR ($CDCl_3$)δ ppm; 0.7-1.0 (6H, m, $CH(\underline{CH_3})_2$), 1.1-2.1 (12H, m, $(CH_3)_2\underline{CH}$-$\underline{CH_2}$, + $C(\underline{CH_3})_3$), 2.8 (2H, bd , J= 5.4Hz, $\underline{CH_2}$-CH=CH), 3.0-3.3 (1H, m, $CH_2$-$\underline{CH}$-CO), 5.0-5.2 (4H, m, $\underline{CH_2}$-O x 2), 6.1-6.4 (28, m, $CH_2$-$\underline{CH=CH}$), 7.1-7.5 (15H, m, aromatic-H).

d) 3(RS)-tert-Butyloxycarbonyl-6-phenyl-2(R)-isobutylhexanoic acid (Compound No. 16)

To a solution of Compound No. 16-c (4.2 g, 7.36 mmol) in ethanol (35 ml) was added 10% palladium on carbon (50% wet catalyst, 1.3 g), and the mixture was vigorously stirred under hydrogen atmosphere for 7 hours at room temperature. The catalyst was filtered off and then ethanol was evaporated under reduced pressure. To the residue was added N-methylmorpholine (0.81 ml, 7.36 mmol) and toluene (50 ml) and the mixture was refluxed for 2 hours. The reaction mixture was partitioned and washed successively with 1N hydrochloric acid, and saturated aqueous sodium chloride (twice for each), dried over anhydrous magnesium sulfate, evaporated under reduced pressure, and purified by column chromatography (silica gel; 150 g, eluted with a mixture of chloroform:methanol= 200:1) to give the title compound as a colorless oil (1.1 g, 43%), Rf value; 0.60 (chloroform:methanol:acetic acid= 95:5:3).

$^1$H-NMR ($CDCl_3$)δ ppm; 0.88 (6H, bd, J= 5.7Hz, $CH(\underline{CH_3})_2$), 1.0-2.0 (16H, m, $(CH_3)_2\underline{CH}$-$\underline{CH_2}$, + $C(\underline{CH_3})_3$ + $\underline{CH_2}$-$\underline{CH_2}$-$CH_2$-Ph), 2.4-2.8 (4H, m, $\underline{CH}$-CO x 2 + $\underline{CH_2}$-Ph), 7.0-7.4 (5H, m, aromatic-H).

Preparation Example 17

3(RS)-tert-Butyloxycarbonyl-5-methyl-2(R)-isobutylhexanoic acid (Compound No. 17)

The procedures of Preparation Examples 16-c and d were repeated using Compound No. 16-b and methallyl iodide to provide the title compound as a pale yellow oil (overall yield 52%), Rf value; 0.23 (AcOEt:n-hexane= 1:4).

$^1$H-NMR ($CDCl_3$)δ ppm; 0.90 (12H, m, $CH(\underline{CH_3})_2$ x 2), 1.0-1.3 (28, m, $\underline{CH}(CH_3)_2$ x 2), 1.4-1.8 (13H,S + m, $\underline{CH_2}$-$CH(CH_3)_2$ x 2 + $C(\underline{CH_3})_3$), 2.5-2.7 (2H, m, $\underline{CH}$-CO x 2).

Preparation Example 18

2(R)-[1(RS)-[tert-Butyloxycarbonyl)ethyl]-4-methylpentanoic acid (Compound No. 18)

The procedures of Preparation Examples 16-c and d were repeated using Compound No. 16-b and methyl iodide to provide the title compound as a pale yellow oil (overall yield 79%), Rf value; 0.28 (chloroform:methanol= 20:1).

$^1$H-NMR ($CDCl_3$)δ ppm; 0.91 (6H, m, $CH(\underline{CH_3})_2$), 1.1-1.3 (4H, m, $\underline{CH}(CH_3)_2$ + CO-CH-$\underline{CH_3}$), 1.4-1.8 (11H,S + m, $\underline{CH_2}$-$CH(CH_3)_2$ + $C(\underline{CH_3})_3$), 2.59 and 2.73 (2H, m, $\underline{CH}$-CO x 2).

Preparation Example 19

3(R)-Carboxy-5-methyl-2(RS)-(3-phenylpropyl)hexanoic acid-N-benzyloxy-N-benzyloxycarbonylamide (Compound No. 19)

a) 5-Methyl-2(RS)-(3-phenylpropyl)-3(R)-2,2,2-trichloroethyloxycarbonyl-hexanoic acid-N-benzyloxy-N-benzyloxycarbonylamide (Compound No. 19-a)

5-Methyl-2(RS)-(3-phenylpropyl)-3(R)-2,2,2-trichloroethyloxycarbonyl-hexanoic acid (1.00 g, 2.27 mmol) was dissolved in $CH_2Cl_2$ (20 ml), and cooled to 0°C under nitrogen atmosphere. To the cooled solution was added DMF (5 drops), and oxalyl chloride (217 µl, 2.49 mmol) with a syringe and the mixture was stirred for 20 minutes at 0°C. The resultant solution was added to a solution of O-benzyl-N-benzyloxycarbonylhydroxylamine (640 mg, 2.49 mmol) and TEA (1.04 ml, 7.47 mmol) in $CH_2Cl_2$ (20 ml) dropwise under nitrogen atmosphere maintaining the temperature at 0°C. After stirring for 15 hours at room temperature, the reaction solution was partitioned and washed successively with 1N hydrochloric acid, saturated aqueous sodium bicarbonate, and saturated aqueous sodium chloride (twice for each), and dried over anhydrous magnesium sulfate. The solvent was removed by evaporation under reduced pressure, and the residue was purified by column chromatography (silica gel; 40 g, eluted with a mixture of n-hexane:AcOEt= 10:1) to give the title compound as a colorless oil (1.1 g, 43%).

$^1$H-NMR ($CDCl_3$)δ ppm; 0.8-0.9 (6H, m, CH$\underline{(CH_3)_2}$), 1.1-1.3 (1H, m, $\underline{CH}(CH_3)_2$), 1.4-1.9 (6H, m, $\underline{CH_2}$-$CH(CH_3)_2$ + $\underline{CH_2}$-$\underline{CH_2}$-$CH_2$-Ph), 2.53 (2H, m, $CH_2$-$\underline{CH_2}$-Ph), 2.84 (1H, m, $\underline{CH}$-CO), 3.83 (1H, m, $\underline{CH}$-CO), 4.66 (2H, s, $\underline{CH_2}$-$CCl_3$), 4.88 (2H, s, O-$\underline{CH_2}$-Ph), 5.26 (2H, s, C(=O)O-$\underline{CH_2}$-Ph), 7.0-7.4 (15H, m, aromatic-H).

b) 3(R)-Carboxy-5-methyl-2(RS)-(3-phenylpropyl)hexanoic acid-N-benzyloxy-N-benzyloxycarbonylamide (Compound No. 19)

To a solution of Compound No. 19-a (800 mg, 1.21 mmol) in acetic acid (30 ml) was added Zn powders (2.80 g, 36.0mmol), and the mixture was stirred for 2.5 hours at room temperature. Zn powders were filtered off and then acetic acid was evaporated, followed by addition of AcOEt. The AcOEt solution was partitioned and washed successively with saturated aqueous sodium chloride, 1N hydrochloric acid, and saturated aqueous sodium chloride (twice for each), dried over anhydrous magnesium sulfate, evaporated under reduced pressure, and purified by column chromatography (silica gel; 50 g, eluted with a mixture of n-hexane:AcOEt= 5:1) to give the title compound as a colorless oil (430 mg, 68%), Rf value; 0.37 (AcOEt:n-hexane = 1 :2).

$^1$H-NMR ($CDCl_3$)δ ppm; 0.8-1.0 (6H, m, CH$\underline{(CH_3)_2}$), 1.1-1.3 (1H, m, $\underline{CH}(CH_3)_2$), 1.4-1.9 (6H, m, $\underline{CH_2}$-$CH(CH_3)_2$ + $\underline{CH_2}$-$\underline{CH_2}$-$CH_2$-Ph), 2.55 (2H, m, $CH_2$-$\underline{CH_2}$-Ph), 2.92 (1H, m, $\underline{CH}$-CO), 3.91 (1H, m, $\underline{CH}$-CO), 4.87 (2H, s, O-$\underline{CH_2}$-Ph), 5.26 (2H, s, C(=O)O-$\underline{CH_2}$-Ph), 7.0-7.4 (15H, m, aromatic-H).

Preparation Example 20

3(RS)-tert-Butoxycarbonyl-6-[4'-($N^1$,$N^2$-dibenzyloxycarbonylguanido)phenyl]-2(R)-isobutylhexanoic acid (Compound No. 20)

a) Dibenzyl 3(RS)-tert-butoxycarbonyl-3-(4'-nitro-cinnamyl)-2(R)-isobutylsuccinate (Compound No. 20-a)

The procedure of Preparation Example 16-c was repeated using Compound No. 16-b and 4-nitro-cinnamyl bromide to provide the title compound as a yellow oil (yield 71%).

$^1$H-NMR ($CDCl_3$)δ ppm; 0.8-1.0 (6H, m, CH$\underline{(CH_3)_2}$), 1.1-1.5 (12H, s + m, $(CH_3)_2\underline{CH\text{-}CH_2}$ + C$\underline{(CH_3)_3}$), 2.90 (2H, m, $\underline{CH_2}$-CH=CH), 3.21 (1H, m, $\underline{CH}$-CO), 5.03 and 5.14 (2H each, s x 2, O-$\underline{CH_2}$-Ph x 2), 6.3-6.5 (2H, m, $CH_2$-$\underline{CH=CH}$), 7.2-7.4 (12H, m, aromatic-H), 8.1 (2H, m, aromatic-H).

b) 3(RS)-tert-Butoxycarbonyl-3-[3-(4'-aminophenyl)propyl]-2(R)-isobutylsuccinic acid (Compound No. 20-b)

To a solution of Compound No. 20-a (4.50 g, 7.31 mmol) in ethanol (100 ml) was added 5% palladium on carbon (50% wet catalyst, 2.5 g), and the mixture was vigorously stirred under hydrogen atmosphere for 2 hours at room temperature. The catalyst was filtered off and then ethanol was evaporated under reduced pressure to yield the title compound as a colorless oil (quantitative yield).

$^1$H-NMR ($CD_3OD$)δ ppm; 0.8-1.0 (6H, m, CH($CH_3$)$_2$), 1.1-2.0 (16H, m, ($CH_3$)$_2$CH-$CH_2$ + C($CH_3$)$_3$ + $CH_2$-$CH_2$-$CH_2$-$C_6H_4$), 2.49 (2H, m, $CH_2$-$C_6H_4$), 2.7-3.5 (1H, m, CH-CO), 6.7-7.1 (4H, m, aromatic-H).

c) 3(RS)-tert-Butoxycarbonyl-6-[4'-($N^1$,$N^2$-dibenzyloxycarbonylguanido)phenyl]-2(R)-isobutylhexanoic acid (Compound No. 20)

To a solution of compound No. 20-b (2.98 g, 7.31 mmol) in DMF (30 ml) was added TEA (3.10 ml, 21.9 mmol) and 1H-pyrazole-N,N'-bis(benzyloxycarbonyl)carboxamidine (3.32 g, 8.77 mmol) and the mixture was stirred for 15 hours at 40°C. The reaction mixture was concentrated under reduced pressure, then dissolved in AcOEt (100 ml), partitioned and washed successively with 1N hydrochloric acid, and saturated aqueous sodium chloride (twice for each), dried over anhydrous magnesium sulfate, evaporated under reduced pressure, and purified by column chromatography (silica gel; 400 g, eluted with a mixture of $CHCl_3$:MeOH = 200:1) to give the title compound as a colorless oil (1.03 g, 21%).

$^1$H-NMR ($CDCl_3$)δ ppm; 0.8-1.0 (6H, m, CH($CH_3$)$_2$), 1.0-1.8 (16H, m, ($CH_3$)$_2$CH-$CH_2$ + C($CH_3$)$_3$ + $CH_2$-$CH_2$-$CH_2$-$C_6H_4$, 2.4-2.7 (4H, m, $CH_2$-$C_6H_4$ + CH-CO x 2), 5.1-5.3 (4H, m, O-$CH_2$-Ph x 2), 7.10 (2H, m, NH x 2), 7.2-7.5 (14H, m, aromatic-H).

Preparation Example 21

Methyl 3(R)-carboxy-2(S)-hydroxy-5-methylhexanoate (Compound No. 21)

Anhydrous trifluoroacetic acid (4 ml) was added to 3(R)-carboxy-2(S)-hydroxy-5-methylhexanoic acid (440 mg, 2.31 mmol) and the mixture was stirred for 4 hours at 0°C. The solvent was evaporated and methanol (4 ml) was added to the residue. The mixture was stirred for 2 hours at 0°C. After methanol was evaporated, the residue was purified by column chromatography (silica gel; 35 g, eluted with a mixture of chloroform:methanol= 20:1) to give the title compound as a colorless oil (344 mg, 73%).

$^1$H-NMR ($CDCl_3$)δ ppm; 0.94 (6H, d, J=5.0Hz, CH($CH_3$)$_2$), 1.3-2.0 (16H, m, ($CH_3$)$_2$CH-$CH_2$), 2.8-3.2 (1H, m, CH-$CO_2$H), 3.82 (3H, s, $OCH_3$), 4.29 (1H, d, J=3.5Hz, HO-CH), 6.6 (2H, brm, OH + $CO_2$H).

Preparation Example 22

$N^a$-[4-(Hydroxyamino)-2(R)-isobutyl-3(RS)-methylsuccinyl]-$N^e$-acetimidoyl-L-lysine N-methylamide • 1 acetate (Compound No. 22)

a) $N^e$-Benzyloxycarbonyl-L-lysine N-methylamide • 1 hydrochloride (Compound No. 22-a)

Compound No. 1 (8.20 g, 20.8 mmol) was dissolved in 4N HCl (AcOEt solution, 100 ml) under ice-cooling and the mixture was stirred for 45 minutes at the same temperature, and concentrated under reduced pressure. $Et_2O$ (100 ml) was added to the residue to yield precipitated crystals which were collected by filtration and dried under reduced pressure, giving the title compound as white crystals (quantitative yield).

$^1$H-NMR ($CD_3OD$)δ ppm; 1.3-1.8 (6H, m, CH($CH_2$)$_3$), 2.70 (3H, s, NH-$CH_3$), 3.27 (2H, m, -OC(=O)NH-$CH_2$), 4.25 (1H, m, NH-CH-CO), 5.05 (2H, s, O-$CH_2$-Ph), 7.2-7.5 (5H, m, aromatic-H).

b) $N^a$-[4-tert-Butoxy-2(R)-isobutyl-3(RS)-methylsuccinyl]-$N^e$-benzyloxycarbonyl-L-lysine N-methylamide (Compound No. 22-b)

To a solution of Compound No. 22-a (6.86 g, 20.8 mmol) in DMF (26 ml)-$CH_2Cl_2$ (60 ml) was added Compound No. 18 (4.60 g, 18.8 mmol), HOBT (3.40 g, 24.9 mmol), EDC (4.77 g, 24.9 mmol) and TEA (2.89 ml, 20.8 mmol) successively with stirring at -15°C, and the mixture was stirred for 1 hour at -15°C and for another 15 hours at room temperature. The solvent was evaporated under reduced pressure, and AcOEt (200 ml) was added to the residue. The mixture was partitioned and washed successively with saturated aqueous sodium chloride, 1N hydrochloric acid, saturated aqueous $NaHCO_3$, and saturated aqueous sodium chloride (twice for each). The organic layer was dried over anhydrous $MgSO_4$, and evaporated under reduced pressure. The resulting reaction mixture was purified by column chromatography (silica gel; 200 g, eluted with a mixture of chloroform:methanol= 40:1) to give the title compound as a white solid (6.87 g, 70%), Rf value; 0.55 (chloroform:methanol = 10:1).

$^1$H-NMR ($CDCl_3$)δ ppm; 0.8-0.9 (6H, m, CH(CH$_3$)$_2$), 1.0-1.2 (4H, m, CO-CH-CH$_3$ + CH(CH$_3$)$_2$), 1.3-1.9 (17H, s + m, CH$_2$-CH(CH$_3$)$_2$ + (CH$_2$)$_3$-CH$_2$-NH + C(CH$_3$)$_3$), 2.4-2.7 (5H, d + m, J=4.7Hz, NH-CH$_3$ + CH-CO x 2), 3.2-3.4 (2H, m, CH$_2$-NH), 4.25 (1H, m, NH-CH-CO), 5.10 (2H, s, O-CH$_2$-Ph), 6.5 (1H, m, NH), 7.0-7.2 (2H, m, NH x 2), 7.2-7.5 (5H, m, aromatic-H).

c) N$^a$-[4-Hydroxy-2(R)-isobutyl-3(RS)-methylsuccinyl]-N$^e$-benzyloxycarbonyl-L-lysine N-methylamide • 1 acetate (Compound No. 22-c)

To Compound No. 22-b (3.4 g, 6.54 mmol) was added ice-cooled 95% trifluoroacetic acid (containing 5% water, 20 ml) and the mixture was stirred for 4 hours at 5°C. The reaction mixture was concentrated under reduced pressure, and $Et_2O$ was added to the residue. The mixture was stirred for 1 hour at room temperature to precipitate solid products which were collected by filtration, and dried, yielding the title compound as colorless glassy materials (3.0 g, 99%).

$^1$H-NMR ($CDCl_3$)δ ppm; 0.8-1.0 (6H, m, CH(CH$_3$)$_2$), 1.0-1.3 (4H, m, CO-CH-CH$_3$ + CH(CH$_3$)$_2$), 1.3-1.8 (8H, m, CH$_2$-CH(CH$_3$)$_2$ + CH-(CH$_2$)$_3$), 2.49 (1H, m, CH-CO), 2.75 (4H, m, NH-CH$_3$ + CH-CO), 2.94 (2H, m, CH$_2$-NH), 4.30 (1H, m, NH-CH-CO), 5.12 (2H, s, O-CH$_2$-Ph), 6.5 (1H, m, NH), 7.0-7.1 (2H, m, NH x 2), 7.2-7.5 (5H, m, aromatic-H).

d) N$^a$-[4-(N-Benzyloxyamino)-2(R)-isobutyl-3(RS)-methylsuccinyl]-N$^e$-acetimidoyl-L-lysine N-methylamide (Compound No. 22-d)

To a solution of Compound No. 22-c (2.0 g, 4.31 mmol) in methanol (40 ml) was added 5% palladium on carbon (50% wet catalyst, 1.0 g), and the mixture was vigorously stirred under hydrogen atmosphere for 3.5 hours at room temperature. The catalyst was filtered off and then the solvent was evaporated under reduced pressure. The resulting residue was dissolved in DMF (40 ml), and cooled to 0°C, followed by addition of TEA (1.93 ml, 13.8 mmol) and ethyl acetimidate hydrochloride (577 mg, 4.53 mmol).

The mixture was stirred for 15 hours at room temperature, and then cooled to 0°C, followed by successive addition of HOBT (612 mg, 24.9 mmol), O-benzylhydroxylamine hydrochloride (1.38 g, 8.62 mmol), EDC (1.65 g, 8.62 mmol) and TEA (1.20 ml, 8.62 mmol). The mixture was stirred for 15 hours at room temperature, and evaporated under reduced pressure. The reaction mixture was purified by DIAION HP-20 (Mitsubishi Chemical Corporation, Japan; 200 ml, eluted with 0-70% aqueous methanol) and column chromatography (silica gel; 70 g, eluted with a gradient mixture of chloroform:methanol:acetic acid= 10:2:1 to 5:2:1). The pertinent fractions were pooled, followed by addition of water (30 ml). Lyophilization of the resultant mixture yielded the title compound as amorphous white powders (1.0 g, 49%).

$^1$H-NMR ($CDCl_3$ + $CD_3Cl$)δ ppm; 0.7-0.9 (6H, m, CH(CH$_3$)$_2$), 0.9-1.1 (4H m, CO-CH-CH$_3$ + CH(CH$_3$)$_2$), 1.2-1.9 (8H, m, CH$_2$-CH(CH$_3$)$_2$ + CH-(CH$_2$)$_3$), 1.94 (3H, s, CH$_3$CO$_2$H), 2.18 (3H, s, C-CH$_3$), 2.55 (1H, m, CH-CO), 2.73 (4H, m, NH-CH$_3$ + CH-CO), 3.0-3.4 (2H, m, CH$_2$-NH), 4.36 (1H, m, NH-CH-CO), 4.89 (2H, s, O-CH$_2$-Ph), 7.2-7.4 (5H, m, aromatic-H).

e) N$^a$-[4-(N-Hydroxyamino)-2(R)-isobutyl-3(RS)-methylsuccinyl]-N$^e$-acetimidoyl-L-lysine N-methylamide • 1 acetate (Compound No. 22)

To a solution of Compound No. 22-d (1.0 g, 2.10 mmol) in acetic acid (10 ml) was added 5% palladium on carbon (50% wet catalyst, 1.0 g), and the mixture was vigorously stirred under hydrogen atmosphere for 2.0 hours at room temperature. The catalyst was filtered off and then acetic acid was evaporated under reduced pressure. $Et_2O$ (20 ml) was added to the resulting residue to precipitate crystals which were collected by filtration, yielding the title compound as white crystals (889 mg, 95%), m.p.; 161-165°C, Rf value; 0.15 (chloroform:methanol:acetic acid= 5:2:1), 0.43 (n-BuOH:AcOH: water= 4:1:1), FABMS ($M^+$ +1): 386.

$^1$H-NMR ($CD_3OD$)δ ppm; 0.8-0.9 (6H, m, CH(CH$_3$)$_2$), 1.0-1.1 (4H, m, CO-CH-CH$_3$ + CH(CH$_3$)$_2$), 1.3-1.9 (8H, m, CH$_2$-CH(CH$_3$)$_2$ + (CH$_2$)$_3$-CH$_2$-NH), 1.94 (3H, s, CH$_3$CO$_2$H), 2.24 (4H, s + m, C-CH$_3$ + CH-CO), 2.55 (1H, m, CH-CO), 2.72 (3H, m, NH-CH$_3$), 3.22 (2H, m, CH$_2$-NH), 4.28 (1H, m, NH-CH-CO).

Preparation Example 23

$N^a$-[4-(Hydroxyamino)-2(R)-isobutyl-3(RS)-methylsuccinyl]-4'-aminomethyl-L-phenylalanine N-methylamide • 1 acetate (Compound No. 23)

a) $N^a$-[4-tert-Butoxy-2(R)-isobutyl-3(RS)-methylsuccinyl]-L-4'-cyanophenylalanine N-methylamide (Compound No. 23-a)

The procedures of Preparation Examples 22-a and b were repeated using Compound No. 5 and Compound No. 18 to provide the title compound as a white solid (yield 80%), m.p.; 160-164°C, Rf value; 0.60 (chloroform:methanol= 10:1).

$^1$H-NMR ($CDCl_3$)δ ppm; 0.7-0.9 (9H, m, CH$\underline{(CH_3)_2}$ + CO-CH-$\underline{CH_3}$), 1.2-1.5 (12H, m, $\underline{CH_2}$-$\underline{CH}$($CH_3)_2$ + C$\underline{(CH_3)_3}$), 2.41 (2H, m, $\underline{CH}$-CO x 2), 2.73 (3H, m, NH-$\underline{CH_3}$), 3.0-3.4 (2H, m, $C_6H_4$-$\underline{CH_2}$), 4.12 (1H, m, NH-$\underline{CH}$-CO), 6.27 and 6.58 (1H each, m, NH x 2), 7.33-7.59 (4H, m, aromatic-H).

b) $N^a$-[4-(N-Benzyloxyamino)-2(R)-isobutyl-3(RS)-methylsuccinyl]-L-4'-cyanophenylalanine N-methylamide (23-b)

To Compound No. 23-a (36.9 g, 85.9 mmol) was added ice-cooled 95% trifluoroacetic acid (containing 5% water, 200 ml) and the mixture was stirred for 2 hours at 5°C. The reaction mixture was concentrated under reduced pressure, and $Et_2O$ was added thereto. The mixture was stirred for 1 hour at room temperature to precipitate solid products which were collected by filtration, and dried.

The resultant solid materials were dissolved in DMF (300 ml), and cooled to -15°C, followed by successive addition of HOBT (13.9 g, 103 mmol), O-benzylhydroxylamine hydrochloride (20.6 g, 129 mmol), EDC (19.7 g, 103 mmol) and TEA (18.0 ml, 129 mmol). After stirred for 15 hours at room temperature, the reaction mixture was added dropwise to water (2 L) to precipitate crystals which were collected by filtration. The resultant crystals were washed successively with 1N hydrochloric acid, 10% aqueous $Na_2CO_3$, water, and $Et_2O$, and dried under reduced pressure to give the title compound as white crystals (39.2 g, 95%), Rf value; 0.42 and 0.47 (chloroform:methanol= 10:1).

$^1$H-NMR ($CDCl_3$ + $CD_3OD$)δ ppm; 0.5-1.0 (10H, m, $\underline{CH(CH_3)_2}$ + CO-CH-$\underline{CH_3}$), 1.2-1.5 (2H, m, $\underline{CH_2}$-CH($CH_3)_2$), 2.0 and 2.34 (1H each, m, $\underline{CH}$-CO x 2), 2.73 (3H, m, NH-$\underline{CH_3}$), 2.98 and 3.19 (1H each, m, $C_6H_4$-$\underline{CH_2}$), 3.59 (1H, m, NH-$\underline{CH}$-CO ), 4.63 (1H, m, NH), 4.86 (2H, m, O-$\underline{CH_2}$-Ph), 7.13 (1H, m, NH), 7.3-7.6 (9H, m, aromatic-H).

c) $N^a$-[4-(Hydroxyamino)-2(R)-isobutyl-3(RS)-methylsuccinyl]-4'-aminomethyl-L-phenylalanine N-methylamide • 1 acetate (Compound No. 23)

To a solution of Compound No. 23-b (10.0 g, 20.8 mmol) in acetic acid (90 ml) was added 5% palladium on carbon (50% wet catalyst, 5 g), and the mixture was vigorously stirred under hydrogen atmosphere for 16 hours at room temperature. The catalyst was filtered off and then acetic acid was evaporated under reduced pressure. The resultant residue was dissolved in water (80 ml), and then lyophilized. The resulting crude product was purified by column reversed phase chromatography (500 g of Chromatorex ODS-1020T, Fuji Silysia Chemical, Japan; eluted with a gradient of 0% to 4% methanol/0.1% aqueous acetic acid), and then lyophilized to give the title compound as a white solid (6.7 g, 71%), m.p.; 223-226°C, Rf value; 0.14 (chloroform:methanol:acetic acid = 5:2:1), 0.52 and 0.60 (n-BuOH:AcOH:water= 4:1:1).

$^1$H-NMR ($CD_3OD$)δ ppm; 0.68 (3H, m, CO-CH-$\underline{CH_3}$), 0.7-0.9 (6H, m, CH$\underline{(CH_3)_2}$), 1.2-1.6 (4H, m, $\underline{CH_2}$-$\underline{CH}$($CH_3)_2$), 1.93 (3H, s, $\underline{CH_3}CO_2H$), 2.57 (2H, m, $\underline{CH}$-CO x 2), 2.66 (3H, m, NH-$\underline{CH_3}$), 2.89-3.30 (2H, m, $C_6H_4$-$\underline{CH_2}$), 4.04 (2H, m, $\underline{CH_2}$-$NH_2$), 4.67 (1H, m, NH-$\underline{CH}$-CO), 7.35 (4H, m, aromatic-H).

Preparation Example 24

$N^a$-[4-(Hydroxyamino)-2(R),3(R or S)-diisobutylsuccinyl]-L-arginine N-methylamide • 1 acetate (Compound No. 24)

a) $N^a$-[4-tert-Butoxy-2(R),3(RS)-diisobutylsuccinyl]-$N^g$,$N^g$-di-benzyloxycarbonyl-L-arginine N-methylamide (Compound No. 24-a)

The procedures of Preparation Examples 22-a and b were repeated using Compound No. 9 and Compound No. 17 to provide the title compound as a white solid (yield 66%).

$^1$H-NMR ($CDCl_3$)δ ppm; 0.8-0.9 (12H, m, CH($CH_3$)$_2$ x 2), 1.1-1.3 (2H, m, CH($CH_3$)$_2$ x 2), 1.4-2.0 (17H, s + m, $CH_2$-CH($CH_3$)$_2$ x 2 + ($CH_2$)$_2$-$CH_2$-NH + C($CH_3$)$_3$), 2.4-2.6 (2H, m, CH-CO x 2), 2.72 (3H, d, J=4.8Hz, NH-$CH_3$), 3.6-3.9 (2H, m, $CH_2$-NH), 4.32 (1H, m, NH-CH-CO), 5.0-5.3 (4H, s x 2, O-$CH_2$-Ph x2), 7.0 (1H, m, NH), 7.2-7.5 (11H, m, aromatic-H + NH), 8.3 and 8.5 (2H, m, NH x 2).

b) $N^a$-[4-(Hydroxyamino)-2(R),3(R or S)-diisobutylsuccinyl]-L-arginine N-methylamide • 1 acetate (Compound No. 24)

The procedures of Preparation Examples 23-b and c were repeated using Compound No. 24-a to provide the title compound as a white solid (overall yield 60%).

$^1$H-NMR ($CD_3OD$)δ ppm; 0.6-0.9 (12H, m, CH($CH_3$)$_2$ x 2), 1.0-1.2 (2H, m, CH($CH_3$)$_2$ x 2), 1.3-1.9 (8H, m, $CH_2$-CH($CH_3$)$_2$ x 2 + ($CH_2$)$_2$-$CH_2$-NH), 1.96 (3H, s, $CH_3CO_2H$), 2.4-2.6 (2H, m, CH-CO x 2), 2.72 (3H, s, NH-$CH_3$), 3.6-3.9 (2H, m, $CH_2$-NH), 4.27 (1H, m, NH-CH-CO).

Preparation Example 25

$N^a$-[4-(Hydroxyamino)-2(R)-isobutyl-3(RS)-methylsuccinyl]-L-arginine N-methylamide • 1 acetate (Compound No. 25)

a) $N^a$-[4-tert-Butoxy-2(R)-isobutyl-3(RS)-methylsuccinyl]-$N^g$,$N^g$-dibenzyloxycarbonyl-L-arginine N-methylamide (Compound No. 25-a)

The procedures of Preparation Examples 22-a and b were repeated using Compound No. 9 and Compound No. 18 to provide the title compound as a white solid (yield 57%).

$^1$H-NMR ($CDCl_3$)δ ppm; 0.7-1.0 (6H, m, CH($CH_3$)$_2$), 1.0-1.3 (4H, m, CH($CH_3$)$_2$ + CO-CH-$CH_3$), 1.3-2.0 (15H, s + m, $CH_2$-CH($CH_3$)$_2$ + ($CH_2$)$_2$-$CH_2$-NH + C($CH_3$)$_3$), 2.2-2.7 (5H, d + m, J=4.8Hz, NH-$CH_3$ + CH-CO x 2), 3.6-4.6 (3H, m, $CH_2$-NH + NH-CH-CO), 5.14 and 5.23 (4H, s x 2, O-$CH_2$-Ph x2), 6.5-7.1 (3H, m, NH x 3), 7.2-7.5 (10H, m, aromatic-H), 9.5 (1H, m, NH).

b) $N^a$-[4-(Hydroxyamino)-2(R)-isobutyl-3(RS)-methylsuccinyl]-L-arginine N-methylamide • 1 acetate (Compound No. 25)

The procedures of Preparation Examples 23-b and c were repeated using Compound No. 25-a to provide the title compound as amorphous white powders (overall yield 53%), Rf value; 0.15 (chloroform:methanol:acetic acid= 5:2:1), 0.47 (n-BuOH:AcOH:water= 4:1:1), FABMS ($M^+$ +1): 373.

$^1$H-NMR ($CD_3OD$)δ ppm; 0.8-0.9 (6H, m, CH($CH_3$)$_2$), 1.0-1.2 (4H, m, CH($CH_3$)$_2$ + CO-CH-$CH_3$), 1.3-1.9 (6H, m, $CH_2$-CH($CH_3$)$_2$ + ($CH_2$)$_2$-$CH_2$-NH), 1.94 (3H, s, $CH_3CO_2H$), 2.2-2.6 (2H, m, CH-CO x 2), 2.73 (3H, s, NH-$CH_3$), 3.6-3.8 (2H, m, $CH_2$-NH), 4.26 (1H, m, NH-CH-CO).

Preparation Example 26

$N^4$-[3-Guanidino-1(S)-methylcarbamoylpropyl]-$N^1$-hydroxy-3(R)-isobutyl-2-(RS)-(3-phenylpropyl)succinamide • 1 acetate (Compound No. 26)

a) [4-tert-Butoxy-2(R)-isobutyl-3(RS)-(3-phenylpropyl)]-succinic acid N-[3-$N^1$,$N^2$-dibenzyloxycarbonylguanidino-1(S)-methylcarbamoyl-n-propyl]amide (Compound No. 26-a)

The procedures of Preparation Examples 22-a and b were repeated using Compound No. 10 and Compound No. 16 to provide the title compound as a white solid (yield 55%).

$^1$H-NMR ($CDCl_3$)δ ppm; 0.87 (6H, m, CH($CH_3$)$_2$), 1.2-1.9 (18H, m, $CH_2$-CH($CH_3$)$_2$ + $CH_2$-$CH_2$-NH + ($CH_2$)$_2$-$CH_2$-Ph + C($CH_3$)$_3$), 2.1-2.8 (7H, d + m, J=4.8Hz, NH-$CH_3$ + CH-CO x 2 + $CH_2$-Ph), 3.6-3.8 (2H, m, $CH_2$-NH), 4.45 (1H, m, + NH-CH-CO), 5.0-5.3 (4H, m, O-$CH_2$-Ph x 2), 7.0 (1H, m, NH), 7.2-7.5 (16H, m, aromatic-H + NH), 8.4 and 8.6 (2H, m, NH x 2).

b) $N^4$-[3-Guanidino-1(S)-methylcarbamoylpropyl]-$N^1$-hydroxy-3(R)-isobutyl-2-(RS)-(3-phenylpropyl)succinamide • 1 acetate (Compound No. 26)

The procedures of Preparation Examples 23-b and c were repeated using Compound No. 26-a to provide the title compound as a white solid (overall yield 51%), m.p.; 143-149°C, Rf value; 0.38 (chloroform:methanol:acetic acid= 5:2:1), 0.62 (n-BuOH:AcOH:water= 4:1:1), FABMS ($M^+$ +2): 464.

$^1$H-NMR ($CD_3OD$)δ ppm; 0.87 (6H, m, CH($CH_3$)$_2$), 1.1-1.9 (9H, m, $CH_2$-CH($CH_3$)$_2$ + $CH_2$-$CH_2$-NH + ($CH_2$)$_2$-$CH_2$-Ph), 2.2-2.8 (7H, s + m, NH-$CH_3$ + CH-CO x 2 + $CH_2$-Ph), 3.6-3.8 (2H, m, $CH_2$-NH), 4.25 (1H, m, + NH-CH-CO), 7.1-7.3 (5H, m, aromatic-H).

Preparation Example 27

$N^4$-[3-Guanidino-1(S)-methylcarbamoylpropyl]-$N^1$-hydroxy-3(R),2(RS)-diisobutylsuccinamide • 1 acetate (Compound No. 27)

a) [4-tert-Butoxy-2(R)-isobutyl-3(RS)-isobutyl]succinic acid-N-[3-$N^1$,$N^2$-dibenzyloxycarbonylguanidino-1(S)-methylcarbamoylpropyl]amide (Compound No. 27-a)

The procedures of Preparation Examples 22-a and b were repeated using Compound No. 10 and Compound No. 17 to provide the title compound as a white solid (yield 22%).

$^1$H-NMR ($CDCl_3$)δ ppm; 0.8-0.9 (12H, m, CH($CH_3$)$_2$ x 2), 1.1-1.3 (2H, m, CH($CH_3$)$_2$ x 2), 1.4-1.9 (15H, s + m, $CH_2$-CH($CH_3$)$_2$ x 2 + $CH_2$-$CH_2$-NH + C($CH_3$)$_3$), 2.4-2.6 (2H, m, CH-CO x 2), 2.71 (3H, d, J=4.8Hz, NH-$CH_3$), 3.6-3.8 (2H, m, $CH_2$-NH), 4.40 (1H, m, NH-CH-CO), 5.0-5.3 (4H, m, O-$CH_2$-Ph x2), 7.1 (1H, m, NH), 7.2-7.5 (11H, m, aromatic-H + NH), 8.4 and 8.6 (2H, m, NH x 2).

b) $N^4$-[3-Guanidino-1(S)-methylcarbamoylpropy]-$N^1$-hydroxy-3(R),2(RS)-diisobutylsuccinamide • 1 acetate (Compound No. 27)

The procedures of Preparation Examples 23-b and c were repeated using Compound No. 27-a to provide the title compound as a white solid (overall yield 18%), m.p.; 165-167°C, Rf value; 0.27 (chloroform:methanol:acetic acid= 5:2:1), 0.66 (n-BuOH:AcOH:water= 4:1:1), FABMS ($M^+$ +1): 401.

$^1$H-NMR ($CD_3OD$)δ ppm; 0.6-0.75 and 0.8-0.9 (13H, m, CH($CH_3$)$_2$ x 2 + CH($CH_3$)$_2$), 1.06 (1H, m, CH($CH_3$)$_2$), 1.3-1.9 (6H, m, $CH_2$-CH($CH_3$)$_2$ x 2 + $CH_2$-$CH_2$-NH), 1.95 (3H, s, $CH_3CO_2H$), 2.4-2.6 (2H, m, CH-CO x 2), 2.73 (3H, s, NH-$CH_3$), 3.6-3.8 (2H, m, $CH_2$-NH), 4.25 (1H, m, NH-CH-CO).

Preparation Example 28

$N^4$-[3-Guanidino-1(S)-methylcarbamoylpropyl]-$N^1$-hydroxy-3(R)-isobutyl-2(R or S)-methylsuccinamide • 1 acetate (Compound No. 28)

a) [4-tert-Butoxy-2(R)-isobutyl-3(R or S)-methyllsuccinic acid N-[3-$N^1$,$N^2$-dibenzyloxycarbonylguanidino-1(S)-methylcarbamoylpropyl]amide (Compound No. 28-a)

The procedures of Preparation Examples 22-a and b were repeated using Compound No. 10 and Compound No. 18 to provide the title compound as a white solid (yield 25%).

$^1$H-NMR ($CDCl_3$)δ ppm; 0.7-1.0 (6H, m, CH($CH_3$)$_2$), 1.0-1.2 (4H, m, CH($CH_3$)$_2$ + CO-CH-$CH_3$), 1.3-2.1 (13H, s + m, $CH_2$-CH($CH_3$)$_2$ + $CH_2$-$CH_2$-NH + C($CH_3$)$_3$), 2.1-2.8 (5H, d + m, J=4.8Hz, CH-CO x 2 + NH-$CH_3$), 3.2 and 3.6 (1H each, m, $CH_2$-NH), 4.45 (1H, m, NH-CH-CO), 5.0-5.3 (4H, m, O-$CH_2$-Ph x2), 7.1 (1H, m, NH), 7.2-7.5 (11H, m, aromatic-H + NH), 8.4 and 8.6 (2H, m, NH x 2).

b) $N^4$-[3-Guanidino-1(S)-methylcarbamoylpropyl]-$N^1$-hydroxy-3(R)-isobutyl-2(R or S)-methylsuccinamide • 1 acetate (Compound No. 28)

The procedures of Preparation Examples 23-b and c were repeated using Compound No. 28-a to provide

the title compound as a white solid (overall yield 65%), m.p.; 135-139°C, Rf value; 0.15 (chloroform:methanol:acetic acid= 5:2:1), 0.49 (n-BuOH:AcOH:water= 4:1:1), FABMS ($M^+$ +1): 359.

$^1$H-NMR (CD,OD)δ ppm; 0.8-0.9 (6H, m, CH($CH_3$)$_2$), 1.0-1.2 (4H, m, CH($CH_3$)$_2$ + CO-CH-$CH_3$), 1.3-1.9 (4H, m, $CH_2$-CH($CH_3$)$_2$ + $CH_2$-$CH_2$-NH), 1.95 (3H, s, $CH_3CO_2H$), 2.2-2.6 (2H, m, CH-CO x 2), 2.72 (3H, s, NH-$CH_3$), 3.6-3.8 (2H, m, $CH_2$-NH), 4.27 (1H, m, NH-CH-CO).

Preparation Example 29

$N^a$-[4-(Hydroxyamino)-2(R)-isobutyl-3(RS)-methylsuccinyl]-L-ornithine N-methylamide • 1 acetate (Compound No. 29)

a) $N^a$-[4-tert-Butoxy-2(R)-isobutyl-3(RS)-methylsuccinyl]-$N^d$-benzyloxycarbonyl-L-ornithine N-methylamide (Compound No. 29-a)

The procedures of Preparation Examples 22-a and b were repeated using Compound No. 7 and Compound No. 18 to provide the title compound as a colorless oil (yield 72%).

$^1$H-NMR ($CDCl_3$)δ ppm; 0.8-1.0 (6H, m, CH($CH_3$)$_2$), 1.0-1.3 (4H, m, CH($CH_3$)$_2$ + CO-CH-$CH_3$), 1.3-1.9 (15H, s + m, $CH_2$-CH($CH_3$)$_2$ + ($CH_2$)$_2$-$CH_2$-NH + C($CH_3$)$_3$), 2.5-2.7 (2H, m, CH-CO x 2), 2.72 (3H, d, J=4.8Hz, NH-$CH_3$), 3.2-3.4 (2H, m, $CH_2$-NH), 4.30 (1H, m, NH-CH-CO), 5.09 (2H, s, O-$CH_2$-Ph), 6.4-6.7 (1H, m, NH), 7.0-7.2 (2H, m, NH x 2), 7.2-7.5 (5H, m, aromatic-H).

b) $N^a$-[4-(Hydroxyamino)-2(R)-isobutyl-3(RS)-methylsuccinyl]-L-ornithine N-methylamide • 1 acetate (Compound No. 29)

The procedures of Preparation Examples 23-b and c were repeated using Compound No. 29-a to provide the title compound as amorphous white powders (overall yield 28%).

$^1$H-NMR ($CD_3OD$)δ ppm; 0.8-0.9 (6H, m, CH($CH_3$)$_2$), 1.0-1.2 (4H, m, CH($CH_3$)$_2$ + CO-CH-$CH_3$), 1.3-1.7 (6H, m, $CH_2$-CH($CH_3$)$_2$ + ($CH_2$)$_2$-$CH_2$-NH), 1.88 (3H, s, $CH_3CO_2H$), 2.4-2.7 (5H, m, CH-CO x 2 + NH-$CH_3$), 2.85 (2H, m, $CH_2$-NH), 4.27 (1H, m, NH-CH-CO).

Preparation Example 30

$N^a$-[4-(Hydroxyamino)-2(R)-isobutyl-3(RS)-(3-phenylpropyl)-succinyl]-$N^e$-acetimidoyl-L-lysine N-methylamide • 1 acetate (Compound No. 30)

a) $N^a$-[4-tert-Butoxy-2(R)-isobutyl-3(RS)-(3-phenylpropyl)-succinyl]-$N^e$-2-chlorobenzyloxycarbonyl-L-lysine N-methylamide (Compound No. 30-a)

The procedures of Preparation Examples 22-a and b were repeated using compound No. 13 and compound No. 16 to provide the title compound as a colorless oil (yield 83%).

$^1$H-NMR ($CDCl_3$)δ ppm; 0.8-0.9 (6H, m, CH($CH_3$)$_2$), 1.08 (1H, m, CH($CH_3$)$_2$), 1.2-1.9 (21H, s + m, ($CH_2$)$_3$-$CH_2$-NH + ($CH_2$)$_2$-$CH_2$-Ph + C($CH_3$)$_3$ + $CH_2$CH($CH_3$)$_2$), 2.4-2.6 (4H, m, CH-CO x 2 + $CH_2$-Ph), 2.77 (3H, m, NH-$CH_3$), 3.18 (2H, m, $CH_2$-NH), 4.32 (1H, m,+ NH-CH-CO), 4.91 (1H, m, NH), 5.22 (2H, m, O-$CH_2$-$C_6H_4$), 6.28 and 6.4 (2H, m, NH x 2), 7.1-7.4 (9H, m, aromatic-H).

b) $N^4$-Benzyloxy-$N^4$-benzyloxycarbonyl-$N^1$-[5-benzyloxycarbonylamino-1(S)-methylcarbamoylpentyl]-2(R)-isobutyl-3(RS)-(3-phenylpropyl)succinamide (Compound No. 30-b)

The procedure of Preparation Example 22-a was repeated using Compound No. 1 and Compound No. 19 to provide the title compound as a white solid (yield 52%).

$^1$H-NMR ($CDCl_3$)δ ppm; 0.7-1.0 (6H, m, CH($CH_3$)$_2$), 1.0-1.2 (1H, m, CH($CH_3$)$_2$), 1.2-1.9 (12H, m, CH-($CH_2$)$_3$ + ($CH_2$)$_2$-$CH_2$-Ph + $CH_2$CH($CH_3$)$_2$), 2.4-2.6 (3H, m, CH-CO + $CH_2$-Ph ), 2.73 (3H, d, J=4.8Hz, NH-$CH_3$), 3.1-3.3 (2H, m, $CH_2$-NH), 3.90 (1H, m, CH-CO), 4.08 (1H, m, NH-CH-CO), 4.80 (2H, m, O-$CH_2$-Ph), 5.09 and 5.26 (4H, s x 2, C(=O)O-$CH_2$-Ph x 2, 6.5 (1H, m, NH), 7.0-7.2 (2H, m, NH x 2), 7.0-7.4 (20H, m, aromatic-H).

c) $N^a$-[4-(Hydroxyamino)-2(R)-isobutyl-3(RS)-(3-phenylpropyl)-succinyl]-L-lysine N-methylamide (Compound No. 30-c)

The procedures of Preparation Examples 23-b and c except using MeOH for catalytic hydrogenation were repeated using Compound No. 30-a to provide the title compound as a white solid (overall yield 70%), m.p.; 181-182°C, Rf value; 0.46 (chloroform:methanol:acetic acid= 5:2:1), 0.56 (n-BuOH:AcOH: water= 4:1:1).

$^1$H-NMR (DMSO-$d_6$)δ ppm; 0.7-1.0 (7H, m, <u>CH(CH$_3$)$_2$</u>), 1.1-1.7 (12H, m, <u>CH$_2$</u>-CH(CH$_3$)$_2$ + <u>(CH$_2$)$_3$</u>-CH$_2$-NH$_2$ + <u>(CH$_2$)$_2$</u>-CH$_2$-Ph), 2.3-2.7 (m, NH-<u>CH$_3$</u> + <u>CH</u>-CO x 2 + <u>CH$_2$</u>-Ph + <u>CH$_2$</u>-NH$_2$), 4.18 (1H, m, NH-<u>CH</u>-CO), 7.1-7.3 (5H, m, aromatic-H).

Similarly, the procedure of Preparation Example 23-c except using MeOH for catalytic hydrogenation was repeated using Compound No. 30-b to provide the title compound (yield 82%).

d) $N^a$-[4-(Hydroxyamino)-2(R)-isobutyl-3(RS)-(3-phenylpropyl)-succinyl]-$N^e$-acetimidoyl-L-lysine N-methylamide • 1 acetate (Compound No. 30)

Compound No. 30-c (3.78 g, 8.42 mmol) was dissolved in DMF (100 ml), and cooled to 0°C, followed by addition of TEA (2.41 ml, 17.3 mmol) and ethyl acetimidate hydrochloride (1.13 g, 8.84 mmol). The mixture was stirred for 30 minutes at 0°C, and then for another 1 hour at room temperature. DMF was evaporated under reduced pressure and the residue was purified by column reversed phase chromatography (200 g of Chromatorex ODS-1020T, Fuji Silysia Chemical, Japan; eluted with a gradient of 4% to 20% methanol/0.1% aqueous acetic acid), and then lyophilized to give the title compound as a white solid (2.50 g, 54%), m.p.; 119-124°C, Rf value; 0.29 (chloroform:methanol: acetic acid= 5:2:1), 0.60 (n-BuOH:AcOH:water= 4:1:1),
FABMS ($M^+$ +2): 492.

$^1$H-NMR (CD$_3$OD)δ ppm; 0.8-0.9 (6H, m, CH<u>(CH$_3$)$_2$</u>), 1.0-1.1 (1H, m, <u>CH</u>(CH$_3$)$_2$), 1.2-1.9 (12H, m, <u>(CH$_2$)$_3$</u>-CH$_2$-NH + <u>(CH$_2$)$_2$</u>-CH$_2$-Ph + <u>CH$_2$</u>CH(CH$_3$)$_2$), 1.92 (3H, s, <u>CH$_3$</u>CO$_2$H), 2.19 (3H, s, C-<u>CH$_3$</u>), 2.5-2.5 (4H, m, <u>CH</u>-CO x 2 + <u>CH$_2$</u>-Ph ), 2.72 (3H, s, NH-<u>CH$_3$</u>), 3.20 (2H, m, <u>CH$_2$</u>-NH), 4.27 (1H, m, NH-<u>CH</u>-CO), 7.1-7.3 (5H, m, aromatic-H).

<u>Preparation Example 31</u>

$N^a$-[4-(Hydroxyamino)-2(R)-isobutyl-3(R or S)-methylsuccinyl]-4'-[tetramethyl bis(phosphono)methyliminol-L-phenylalanine N-methylamide (Compound No. 31)

a) $N^a$-[4-tert-Butoxy-2(R)-isobutyl-3(RS)-methylsuccinyl]-4'-[tetramethyl bis(phosphono)methylimino]-L-phenylalanine N-methylamide (Compound No. 31-a)

To a solution of Compound No. 12 (3.10 g, 5.56 mmol) in MeOH (100 ml) was added 10% palladium on carbon (50% wet catalyst, 3.1 g), and the mixture was vigorously stirred under hydrogen atmosphere for 1.5 hours at room temperature. The catalyst was filtered off and then the solvent was evaporated under reduced pressure. The resultant oily material was dissolved in DMF (60 ml), to which was added Compound No. 18 (1.36 g, 5.56 mmol), HOBT (900 mg, 6.67 mmol), EDC (1.28 g, 6.67 mmol) and TEA (775 μl, 5.58 mmol) successively with stirring at -15°C. The mixture was stirred for 1 hour at -15°C, and further for another 15 hours at room temperature, and evaporated under reduced pressure, followed by addition of AcOEt (200 ml). The resultant mixture was partitioned and washed successively with saturated aqueous sodium chloride, 1N hydrochloric acid, saturated aqueous NaHCO$_3$, and saturated aqueous sodium chloride (twice for each). The organic layer was dried over anhydrous MgSO$_4$, and evaporated under reduced pressure, and the residue was purified column chromatography (silica gel; 130 g, eluted with a mixture of chloroform:methanol= 20:1) to give the title compound as a pale yellow oil (1.49 g, 41%).

$^1$H-NMR (CDCl$_3$)δ ppm; 0.7-1.2 (9H, m, CH<u>(CH$_3$)$_2$</u> + CO-CH-<u>CH$_3$</u>), 1.3 (1H, m, <u>CH</u>(CH$_3$)$_2$), 1.4-1.8 (11H, m, <u>CH$_2$</u>-CH(CH$_3$)$_2$ + C<u>(CH$_3$)$_3$</u>), 2.3-2.5 (2H, m, <u>CH</u>-CO x 2), 2.72 (3H, m, NH-<u>CH$_3$</u>), 2.95 (2H, m, C$_6$H$_4$-<u>CH$_2$</u>), 3.80 (12H, m, O-<u>CH$_3$</u> x 4), 4.12 (1H, m, NH-<u>CH</u>-CO), 4.58 (1H, m, P-<u>CH</u>-P), 5.90 , 6.17 and 6.36 (3H, m, NH x 3), 6.61-7.07 (4H, m, aromatic-H).

b) $N^a$-[4-(Hydroxyamino)-2(R)-isobutyl-3(R or S)-methylsuccinyl]-4'-[tetramethyl bis(phosphono)methylimino)-L-phenylalanine N-methylamide (Compound No. 31)

The procedures of Preparation Examples 23-b and c were repeated using Compound No. 31-a to provide

the title compound as a white solid (overall yield 42%), m.p.; 204-208°C, Rf value; 0.52 (chloroform:methanol:acetic acid= 5:2:1), 0.63 (n-BuOH:AcOH:water= 4:1:1), FABMS ($M^+$ +2): 610.

$^1$H-NMR ($CD_3OD$)δ ppm; 0.6 (3H, d, J = 6.9Hz, CO-CH-$\underline{CH_3}$), 0.72 and 0.79 (6H, d each, J=6.6 and 6.3Hz, CH$\underline{(CH_3)_2}$), 0.9 and 1.1-1.5 (3H, m, $\underline{CH_2}$-$\underline{CH}$($CH_3$)$_2$), 1.98 and 2.38 (1H each, m, $\underline{CH}$-CO x 2), 2.57 (3H, s, NH-$\underline{CH_3}$), 2.6-2.9 (2H, m, $C_6H_4$-$\underline{CH_2}$), 3.67 (12H, m, O-$\underline{CH_3}$ x 4), 4.46 (2H, m, NH-$\underline{CH}$-CO + P-$\underline{CH}$-P), 6.6-7.0 (4H, m, aromatic-H).

Preparation Example 32

$N^a$-[4-(Hydroxyamino)-2(R)-isobutyl-3(R or S)-methylsuccinyl]-4'-[trimethyl bis(phosphono)methylimino]-L-phenylalanine N-methylamide • 1 sodium salt (Compound No. 32)

Compound No. 31-b (1.18 g, 1.94 mmol) was dissolved in MeOH (15 ml), and cooled to 0°C, followed by dropwise addition of 2N aqueous sodium hydroxide (9.7 ml). The mixture was stirred for 15 hours at room temperature, and then re-cooled to 0°C. The cooled reaction mixture was adjusted with 6N hydrochloric acid to pH 7, and evaporated. The resultant residue was purified by column reversed phase chromatography (50 g of Chromatorex ODS-1020T, Fuji Silysia Chemical, Japan; eluted with a gradient of 1 to 10% aqueous methanol), and then lyophilized to give the title compound as a white solid (263 mg, 22%), m.p.; 235-240°C, Rf value; 0.14 (chloroform: methanol:acetic acid= 5:2:1), 0.46 (n-BuOH:AcOH:water= 4:1:1).

$^1$H-NMR ($CD_3OD$)δ ppm; 0.71 (3H, d, J=6.6Hz, CO-CH-$\underline{CH_3}$), 0.81 and 0.88 (6H, d each, J=6.6 and 6.3Hz, CH$\underline{(CH_3)_2}$), 1.02 and 1.2-1.6 (3H, m, $\underline{CH_2}$-$\underline{CH}$($CH_3$)$_2$), 2.08 and 2.48 (1H each, m, $\underline{CH}$-CO x 2), 2.66 (3H, s, NH-$\underline{CH_3}$), 2.7-3.0 (2H, m, $C_6H_4$-$\underline{CH_2}$), 3.48 (3H, m, O-$\underline{CH_3}$ x 4), 3.70 and 3.75 (6H, m, O-$\underline{CH_3}$ x 2), 4.07 (1H, m, NH-$\underline{CH}$-CO), 4.54 (1H, m, P-$\underline{CH}$-P), 6.6-7.1 (4H, m, aromatic-H).

Preparation Example 33

$N^a$-[4-(Hydroxyamino)-2(R)-isobutyl-3(RS)-methylsuccinyl]-4'-[tetraethyl bis(phosphono)methylimino]-L-phenylalanine N-methylamide (Compound No. 33)

a) $N^a$-[4-tert-Butoxy-2(R)-isobutyl-3(RS)-methylsuccinyl]-4'-[tetraethyl bis(phosphono)methylimino]-L-phenylalanine N-methylamide (Compound No. 33-a)

The procedure of Preparation Example 31-a was repeated using Compound No. 11 and Compound No. 18 to provide the title compound as a yellow oil (yield 89%).

$^1$H-NMR ($CDCl_3$)δ ppm; 0.7-0.9 (6H, m, CH$\underline{(CH_3)_2}$), 0.9-1.1 (4H, m, $\underline{CH}$($CH_3$)$_2$ + CO-CH-$\underline{CH_3}$), 1.2-1.35 (12H, m, $CH_2$-$\underline{CH_3}$ x 4), 1.43 (9H, s, C$\underline{(CH_3)_3}$), 1.6-1.9 (2H, m, $\underline{CH_2}$-CH($CH_3$)$_2$), 2.3-2.5 (2H, m, $\underline{CH}$-CO x 2), 2.69 (3H, m, NH-$\underline{CH_3}$), 2.95-3.1 (2H, m, $C_6H_4$-$\underline{CH_2}$), 4.0-4.3 (9H, m, $\underline{CH_2}$-$CH_3$ x 4 + NH-$\underline{CH}$-CO), 4.55 (1H, m, P-$\underline{CH}$-P), 5.77 , 6.19 and 6.41 (1H each, m, NH x 3), 6.6-7.1 (4H, m, aromatic-H).

b) $N^a$-[4-(Hydroxyamino)-2(R)-isobutyl-3(RS)-methylsuccinyl]-4'-[tetraethyl bis(phosphono)methylimino]-L-phenylalanine N-methylamide

The procedures of Preparation Examples 23-b and c were repeated using Compound No. 33-a to provide the title compound as a white solid (overall yield 16%), m.p.; 185-189°C, Rf value; 0.68 (chloroform:methanol:acetic acid= 5:2:1), 0.83 (n-BuOH:AcOH:water= 4:1:1), FABMS ($M^+$ +2): 666.

$^1$H-NMR ($CD_3OD$)δ ppm; 0.62 (3H, d, J = 6.9Hz, CO-CH-$\underline{CH_3}$), 0.72 and 0.79 (6H, d each, J=6.6 and 6.3Hz, CH$\underline{(CH_3)_2}$), 0.9 (1H, m, $\underline{CH}$($CH_3$)$_2$), 1.0-1.2 (12H, m, $CH_2$-$\underline{CH_3}$ x 4), 1.2-1.5 (2H, m, $\underline{CH_2}$-CH($CH_3$)$_2$), 2.0 (1H, m, $\underline{CH}$-CO), 2.38 (1H, m, $\underline{CH}$-CO), 2.59 (3H, m, NH-$\underline{CH_3}$), 2.6-2.9 (2H, m, $C_6H_4$-$\underline{CH_2}$), 3.9-4.1 (8H, m, $\underline{CH_2}$-$CH_3$ x 4), 4.45 (2H, m, NH-$\underline{CH}$-CO + P-$\underline{CH}$-P), 6.6-7.0 (4H, m, aromatic-H).

Preparation Example 34

$N^a$-[4-(Hydroxyamino)-2(R)-isobutyl-3(RS)-methylsuccinyl]-4'-[triethyl bis(phosphono)methyliminol]-L-phenylalanine N-methylamide • 1 sodium salt (Compound No. 34)

The procedure of Preparation Example 32 was repeated using Compound No. 33-b (1.18 g, 1.94 mmol) to provide the title compound as a white solid (overall yield 32%), m.p.; 215-220°C, Rf value; 0.31 (chloroform:methanol:acetic acid= 5:2:1), 0.60 (n-BuOH:AcOH:water= 4:1:1).

$^1$H-NMR ($CD_3OD$)δ ppm; 0.75 (3H, d, J = 6.6Hz, CO-CH-$\underline{CH_3}$), 0.82 and 0.88 (6H, d x 2, J = 6.6 and 6.3Hz, CH$\underline{(CH_3)_2}$), 0.9-1.6 (12H, m, $\underline{CH_2}$-$\underline{CH}$($CH_3$)$_2$ + $CH_2$-$\underline{CH_3}$ x 3), 2.11 and 2.49 (1H, m, $\underline{CH}$-CO x 2), 2.65 (3H, s, NH-$\underline{CH_3}$), 2.7-3.0 (2H, m, $C_6H_4$-$\underline{CH_2}$), 3.84 and 4.0-4.2 (6H, m, $\underline{CH_2}$-$CH_3$ x 3), 4.53 (2H, m, NH-$\underline{CH}$-CO + P-$\underline{CH}$-P), 6.6-7.1 (4H, m, aromatic-H).

Preparation Example 35

$N^a$-[4-(Hydroxyamino)-2(R),3(RS)-diisobutylsuccinyl]-4'-aminomethyl-L-phenylalanine N-(2-hydroxyethyl)amide • 1 acetate (Compound No. 35)

a) $N^a$-[4-tert-Butoxy-2(R),3(RS)-diisobutylsuccinyl]-L-4'-cyanophenylalanine N-(2-benzyloxyethyl)amide (Compound No. 35-a)

The procedure of Preparation Example 22-a was repeated using Compound No. 14 and Compound No. 17 to provide the title compound as a colorless glassy material (yield 58%), m.p.; 64-67°C, Rf value; 0.38 (chloroform:methanol= 20:1).

$^1$H-NMR ($CDCl_3$)δ ppm; 0.7-0.9 (12H, m, CH$\underline{(CH_3)_2}$ x 2), 0.9-1.1 (2H, m, $\underline{CH}$($CH_3$)$_2$ x 2), 1.2-1.6 (13H, m, C$\underline{(CH_3)_3}$ + $\underline{CH_2}$-CH($CH_3$)$_2$ x 2), 2.32 and 2.48 (1H each, m, $\underline{CH}$-CO x 2), 3.10 (2H, d, J=7.2Hz, $C_6H_4$-$\underline{CH_2}$), 3.3-3.6 (4H, m, NH-$\underline{CH_2}$-$\underline{CH_2}$-O), 4.45 (2H, s, O-$\underline{CH_2}$-Ph), 4.66 (1H, m, NH-$\underline{CH}$-CO), 6.12 and 6.33 (1H each, m, NH x 2), 7.2-7.6 (9H, m, aromatic-H).

b) $N^a$-[4-(Hydroxyamino)-2(R),3(RS)-diisobutylsuccinyl]-4'-aminomethyl-L-phenylalanine N-(2-hydroxyethyl)amide • 1 acetate (Compound No. 35)

The procedures of Preparation Examples 23-b and c were repeated using Compound No. 35-a to provide the title compound as a white solid (overall yield 46%), m.p.; 196-198°C, Rf value; 0.45 (chloroform:methanol:acetic acid= 5:2:1), 0.49 (n-BuOH:AcOH:water= 4:1:1), FABMS ($M^+$ +1): 465.

$^1$H-NMR ($CD_3OD$)δ ppm; 0.6-0.8 (6H, m, CH$\underline{(CH_3)_2}$), 0.8-0.9 (6H, m, CH$\underline{(CH_3)_2}$), 0.9-1.1 (2H, m, $\underline{CH}$($CH_3$)$_2$ x 2), 1.3-1.7 (4H, m, $\underline{CH_2}$-CH($CH_3$)$_2$ x 2), 1.93 (3H, s, $CH_3CO_2H$), 2.16 and 2.47 (1H each, m, $\underline{CH}$-CO x 2), 2.98 and 3.10 (1H each, m, $C_6H_4$-$\underline{CH_2}$), 3.23 and 3.50 (m, NH-$\underline{CH_2}$-$\underline{CH_2}$-O), 4.03 (2H, s, $\underline{CH_2}$-$NH_2$), 4.67 (1H, m, NH-$\underline{CH}$-CO), 7.38 (4H, m, aromatic-H).

Preparation Example 36

$N^a$-[4-(Hydroxyamino)-2(R)-isobutyl-3(R or S)-(3-phenylpropyl)-succinyl]-$N^e$-acetimidoyl-L-lysine N-(2-hydroxyethyl)amide • 1 acetate (Compound No. 36)

a) $N^a$-[4-tert-Butoxy-2(R)-isobutyl-3(RS)-(3-phenylpropyl)-succinyl]-$N^e$-2-chlorobenzyloxycarbonyl-L-lysine N-(2-benzyloxyethyl)amide (Compound No. 36-a)

The procedure of Preparation Example 22-a was repeated using Compound No. 15 and Compound No. 16 to provide the title compound as a colorless waxy material (yield 88%).

$^1$H-NMR ($CDCl_3$)δ ppm; 0.84 (6H, m, CH$\underline{(CH_3)_2}$), 1.2-1.9 (22H, m, $\underline{(CH_2)_3}$-$CH_2$-NH + $\underline{(CH_2)_2}$-$CH_2$-Ph + $\underline{C(CH_3)_3}$ + $\underline{CH_2CH}$($CH_3$)$_2$), 2.3-2.7 (4H, m, $\underline{CH}$-CO x 2 + $\underline{CH_2}$-Ph), 3.15 (2H, m, $\underline{CH_2}$-NH), 3.4-3.6 (4H, m, NH-$\underline{CH_2}$-$\underline{CH_2}$-O), 4.36 (1H, m, NH-$\underline{CH}$-CO), 4.51 (2H, m, O-$\underline{CH_2}$-Ph), 4.91 (1H, m, NH), 5.21 (3H, s + m, -C(=O)O-$\underline{CH_2}$-$C_6H_4$), 6.44 (1H, m, NH), 7.0-7.5 (14H, m, aromatic-H).

b) $N^a$-[4-(Benzyloxyamino)-2(R)-isobutyl-3(RS)-(3-phenylpropyl)-succinyl]-$N^e$-2-chlorobenzyloxycarbonyl-L-lysine N-(2-benzyloxyethyl)amide (Compound No. 36-b)

The procedure of Preparation Example 23-b was repeated using Compound No. 36-a (3.83 g, 4.92 mmol) to provide the title compound as a white solid (yield 69%), m.p.; 158-165°C, Rf value; 0.67 (chloroform:methanol= 10:1).

$^1$H-NMR ($CDCl_3$)δ ppm; 0.7-0.9 (6H, m, CH($\underline{CH_3}$)$_2$), 1.09 (1H, m, $\underline{CH}$($CH_3$)$_2$), 1.2-1.9 (12H, m, $\underline{(CH_2)_3}$-$CH_2$-NH + $\underline{(CH_2)_2}$-$CH_2$-Ph + $\underline{CH_2}$CH($CH_3$)$_2$), 2.22 (1H, m, $\underline{CH}$-CO), 2.4-2.7 (3H, m, $\underline{CH}$-CO + $\underline{CH_2}$-Ph), 3.12 (2H, m, $\underline{CH_2}$-NH), 3.2-3.6 (4H, m, NH-$\underline{CH_2}$-$\underline{CH_2}$-O), 4.4-4.5 (3H, m, $CH_2$-O-$\underline{CH_2}$-Ph + NH-$\underline{CH}$-CO), 4.8-4.9 (2H, m, NH-O-$\underline{CH_2}$-Ph), 5.1-5.2 (3H, m, NH + -C(=O)O-$\underline{CH_2}$-$C_6H_4$), 6.9-7.3 (22H, m, aromatic-H + NH x 3).

c) $N^a$-[4-(Hydroxyamino)-2(R)-isobutyl-3(RS)-(3-phenylpropyl)-succinyl]-L-lysine N-2-benzyloxyethylamide • 1 acetate (Compound No. 36-c)

To a solution of Compound No. 36-b (2.60 g, 3.10 mmol) in acetic acid (30 ml) was added 5% palladium on carbon (50% wet catalyst, 1.5 g), and the mixture was vigorously stirred under hydrogen atmosphere for 2.5 hours at room temperature. The catalyst was filtered off and then acetic acid was evaporated under reduced pressure. $Et_2O$ (50 ml) was added to the resulting residue to precipitate crystals which were collected by filtration, yielding the title compound as white crystals (1.44 g, 86%).

$^1$H-NMR ($CDCl_3$ + $CD_3OD$)δ ppm; 0.8-1.0 (6H, m, CH($\underline{CH_3}$)$_2$), 1.0-1.2 (1H, m, $\underline{CH}$($CH_3$)$_2$), 1.2-1.95 (12H, m, $\underline{(CH_2)_3}$-$CH_2$-NH + $\underline{(CH_2)_2}$-$CH_2$-Ph + $\underline{CH_2}$CH($CH_3$)$_2$), 1.98 (3H, s, $\underline{CH_3}$$CO_2$H), 2.18 (1H, m, $\underline{CH}$-CO), 2.57 (3H, m, $\underline{CH}$-CO + $\underline{CH_2}$-Ph), 2.90 (2H, m, $\underline{CH_2}$-NH), 3.2-3.4 and 3.63 (4H, m, NH-$\underline{CH_2}$-$\underline{CH_2}$-O), 4.32 (1H, m, NH-$\underline{CH}$-CO), 7.0-7.3 (5H, m, aromatic-H).

d) $N^a$-[4-(Hydroxyamino)-2(R)-isobutyl-3(R or S)-(3-phenylpropyl)succinyl]-$N^e$-acetimidoyl-L-lysine N-(2-hydroxyethyl)-amide • 1 acetate (Compound No. 36)

Compound No. 36-c (1.31 g, 2.43 mmol) was dissolved in DMF (50 ml), and cooled to 0°C, followed by addition of TEA (1.10 ml, 7.86 mmol) and ethyl acetimidate hydrochloride (360 mg, 2.92 mmol). The reaction mixture was stirred for 30 minutes at 0°C, and then evaporated. This reaction mixture was purified by column reversed phase chromatography (50 g of Chromatorex ODS-1012T, Fuji Silysia Chemical, Japan; eluted with a gradient of 0.1 to 7% methanol/0.1% aqueous acetic acid), and then lyophilized to give the title compound as a white solid (881 mg, 63%), m.p.; 118-121°C, Rf value; 0.22 (chloroform:methanol:acetic acid= 5:2:1), 0.48 (n-BuOH:AcOH: water= 4:1:1), FABMS ($M^+$ +1): 520.

$^1$H-NMR ($CD_3OD$)δ ppm; 0.8-0.9 (6H, m, CH($\underline{CH_3}$)$_2$), 1.05 (1H, m, $\underline{CH}$($CH_3$)$_2$), 1.2-1.8 (12H, m, $\underline{CH_2}$-CH($CH_3$)$_2$ + $\underline{(CH_2)_3}$-$CH_2$-NH + $\underline{CH_2}$-$\underline{CH_2}$-$CH_2$-Ph), 1.89 (3H, s, $\underline{CH_3}$$CO_2$H), 2.23 (3H, s, C-$\underline{CH_3}$), 2.4-2.6 (4H, m, $\underline{CH_2}$-Ph + $\underline{CH}$-CO x 2), 3.1-3.3 (m, NH-$\underline{CH_2}$-$CH_2$-OH + $\underline{CH_2}$-NH), 3.58 (2H, m, $\underline{CH_2}$-OH), 4.30 (1H, m, NH-$\underline{CH}$-CO), 7.1-7.3 (5H, m, aromatic-H).

<u>Preparation Example 37</u>

$N^a$-[4-(Hydroxyamino)-2(R)-isobutyl-3(R or S)-methylsuccinyl]-L-ornithine N-cyclopropylamide • 1 acetate (Compound No. 37)

a) $N^a$-[4-tert-Butoxy-2(R)-isobutyl-3(RS)-methylsuccinyl]-$N^d$-benzyloxycarbonyl-L-ornithine N-methylamide (Compound No. 37-a)

The procedures of Preparation Examples 22-a and b were repeated using Compound No. 8 and Compound No. 18 to provide the title compound as a colorless oil (yield 54%).

$^1$H-NMR ($CDCl_3$)δ ppm; 0.48 (2H, m, $\underline{CH_2}$ of cyclopropyl), 0.7-0.9 (8H, m, $\underline{CH_2}$ of cyclopropyl + CH($\underline{CH_3}$)$_2$), 1.0-1.2 (4H, m, $\underline{CH}$($CH_3$)$_2$ + CO-CH-$\underline{CH_3}$), 1.3-1.9 (15H, m, $\underline{CH_2}$-CH($CH_3$)$_2$ + $\underline{(CH_2)_2}$-$CH_2$-NH + C($\underline{CH_3}$)$_3$), 2.4-2.7 (3H, m, $\underline{CH}$ of cyclopropyl + $\underline{CH}$-CO x 2), 3.2-3.4 (2H, m, $\underline{CH_2}$-NH), 4.31 (1H, m, NH-$\underline{CH}$-CO), 5.07 (2H, s, O-$\underline{CH_2}$-Ph), 6.4-6.7 (1H, m, NH), 7.0-7.2 (2H, m, NH x 2), 7.2-7.5 (5H, m, aromatic-H).

b) $N^a$-[4-(Hydroxyamino)-2(R)-isobutyl-3(R or S)-methylsuccinyl)-L-ornithine N-cyclopropylamide • 1 acetate

The procedures of Preparation Examples 23-b and c were repeated using Compound No. 37-a to provide the title compound as a white solid (overall yield 35%), m.p.; 185-192°C, Rf value; 0.16 (chloroform:methanol:acetic acid= 5:2:1), 0.46 (n-BuOH: AcOH:water= 4:1:1), FABMS ($M^+$ +1): 357.

$^1$H-NMR ($CD_3OD$)δ ppm; 0.45 (2H, m, $\underline{CH_2}$ of cyclopropyl), 0.7-0.9 (8H, m, $\underline{CH_2}$ of cyclopropyl + CH$\underline{(CH_3)_2}$), 1.0-1.2 (4H, m, $\underline{CH}(CH_3)_2$ + CO-CH-$\underline{CH_3}$), 1.3-1.7 (6H, m, $\underline{CH_2}$-$CH(CH_3)_2$ + $\underline{(CH_2)_2}$-$CH_2$-NH), 1.89 (3H, m, $\underline{CH_3}CO_2H$), 2.4-2.7 (3H, m, $\underline{CH}$ of cyclopropyl + $\underline{CH}$-CO x 2), 2.86 (2H, m, $\underline{CH_2}$-NH), 4.26 (1H, m, NH-$\underline{CH}$-CO).

Preparation Example 38

$N^a$-[4-(Hydroxyamino)-2(R)-isobutyl-3(RS)-methylsuccinyl]-L-lysine N-methylamide • 1 acetate (Compound No. 38)

The procedures of Preparation Examples 36-b and c were repeated using Compound No. 22-b to provide the title compound as a white solid (overall yield 48%), m.p.; 173-177°C, Rf value; 0.14 (chloroform:methanol:acetic acid= 5:2:1), 0.42 (n-BuOH: AcOH:water= 4:1:1), FABMS ($M^+$ +1): 345.

$^1$H-NMR ($CD_3OD$)δ ppm; 0.7-0.9 (6H, m, CH$\underline{(CH_3)_2}$), 1.02 (4H, m, $\underline{CH}(CH_3)_2$ + CO-CH-$\underline{CH_3}$), 1.3-1.7 (8H, m; $\underline{CH_2}$-$CH(CH_3)_2$ + $\underline{(CH_2)_3}$-$CH_2$-$NH_2$), 1.87 (3H, m, $\underline{CH_3}CO_2H$), 2.21 and 2.54 (1H each, m, $\underline{CH}$-CO x 2), 2.68 (3H, s, NH-$\underline{CH_3}$), 2.85 (2H, m, $\underline{CH_2}$-$NH_2$), 4.25 (1H, m, NH-$\underline{CH}$-CO).

Preparation Example 39

$N^a$-[4-(Hydroxyamino)-2(R)-isobutyl-3(RS)-methylsuccinyl]-$N^e$-acetimidoyl-L-lysine N-[2-hydroxy-1(RS)-methylethyl]amide • 1 acetate (Compound No. 39)

a) $N^a$-[4-tert-Butoxy-2(R)-isobutyl-3(RS)-methylsuccinyl]-$N^e$-tert-butyloxycarbonyl-L-lysine N-[2-hydroxy-1(RS)-methylethyl]-amide (Compound No. 39-a)

The procedures of Preparation Examples 22-a and b were repeated using Compound No. 6 and Compound No. 18 to provide the title compound as a colorless oil (yield 71%).

$^1$H-NMR ($CDCl_3$)δ ppm; 0.8-1.0 (6H, m, CH$\underline{(CH_3)_2}$), 1.0-1.2 (7H, m, $\underline{CH}(CH_3)_2$ + CH-$\underline{CH_3}$ x 2), 1.3-2.0 (26H, s x 2 + m, C$\underline{(CH_3)_3}$ x 2 + $\underline{CH_2}$-$CH(CH_3)_2$ + $\underline{(CH_2)_3}$-$CH_2$-NH), 2.4-2.6 (2H, m, $\underline{CH}$-CO x 2), 3.08 (2H, m, $\underline{CH_2}$-NH), 3.4-3.6 (3H, m, NH-$\underline{CH_2}$-$\underline{CH_2}$-OH), 4.04 (1H, m, NH-$\underline{CH}$-CO), 4.43 (1H, m, OH), 4.85 (1H, m, NH), 6.8-7.1 (2H, m, NH x 2).

b) $N^a$-[4-(Hydroxyamino)-2(R)-isobutyl-3(RS)-methylsuccinyl]-$N^e$-acetimidoyl-L-lysine N-[2-hydroxy-1(RS)-methylethyl]amide • 1 acetate (Compound No. 39)

To Compound No. 39-a (8.50 g, 16.0 mmol) was added ice-cooled 95% trifluoroacetic acid (containing 5% water, 70 ml) and the mixture was stirred for 30 minutes at 5°C, and for another 1 hour at room temperature, and concentrated under reduced pressure, followed by addition of $Et_2O$. The mixture was stirred for 1 hour at room temperature to precipitate solid products which were collected by filtration, and dried, yielding a white solid carboxylic acid compound (6.01 g, 79%).

This carboxylic acid compound was dissolved in DMF (60 ml), and cooled to 0°C. To the cooled solution was added TEA (6.90 ml, 49.7 mmol) and ethyl acetimidate hydrochloride (2.18 g, 17.6 mmol), and the mixture was stirred for 30 minutes at room temperature, and then re-cooled to 0°C, followed by successive addition of HOBT (2.38 g, 17.6 mmol), O-benzylhydroxylamine hydrochloride (5.38 g, 33.7 mmol), EDC (4.61 g, 24.1 mmol) and TEA (4.70 ml, 33.7 mmol). The mixture was stirred for 15 hours at room temperature, and then evaporated under reduced pressure. The reaction mixture was purified by DIAION HP-20 (Mitsubishi Chemical Corporation, Japan; 500 ml, eluted with 10 to 100% aqueous methanol) and column chromatography (silica gel; 150 g, eluted with a mixture of chloroform:methanol:acetic acid= 5:2:1 and a mixture of methanol:acetic acid= 1:1). The pertinent fractions were pooled, and concentrated under reduced pressure. The resulting residue was dissolved in acetic acid (100 ml), followed by addition of 5% palladium on carbon (50% wet catalyst, 3.0 g). The mixture was vigorously stirred under hydrogen atmosphere for 1.5 hours at room temperature. The catalyst was filtered off and then acetic acid was evaporated under reduced pressure. The resultant residue was dissolved in water, and lyophilized to give the title compound as a white solid (2.55 g, 32%), m.p.; 118-127°C, Rf value; 0.13 (chloroform:methanol:acetic acid= 5:2:1), 0.39 (n-BuOH:AcOH:water= 4:1:1), FABMS

($M^+$ +1): 430.

$^1$H-NMR ($CD_3OD$)δ ppm; 0.8-1.0 (6H, m, CH($CH_3$)$_2$), 1.0-1.2 (7H, m, CH($CH_3$)$_2$ + CH-$CH_3$ x 2), 1.2-2.0 (11H, s + m, $CH_2$-CH($CH_3$)$_2$ + ($CH_2$)$_3$-$CH_2$-NH + $CH_3CO_2H$), 2.20 (3H, s, C-$CH_3$), 2.57 (2H, m, CH-CO x 2), 3.2-3.5 (m, $CH_2$-$NH_2$ + NH-$CH_2$-$CH_2$-OH), 3.91 (1H, m, NH-CH-CO).

Preparation Example 40

$N^a$-[4-(Hydroxyamino)-2(R)-isobutyl-3(RS)-methylsuccinyl]-$N^e$-acetimidoyl-L-lysine N-(piperidin-1-yl)amide • 1 acetate (Compound No. 40)

a) $N^a$-[4-tert-Butoxy-2(R)-isobutyl-3(RS)-methylsuccinyl]-$N^e$-tert-butyloxycarbonyl-L-lysine N-(piperidin-1-yl)amide (Compound No. 40-a)

The procedures of Preparation Examples 22-a and b were repeated using Compound No. 4 and Compound No. 18 to provide the title compound as a pale yellow oil (yield 71%).

$^1$H-NMR ($CDCl_3$)δ ppm; 0.8-1.0 (6H, m, CH($CH_3$)$_2$), 1.0-1.2 (4H, m, CH($CH_3$)$_2$ + CO-CH-$CH_3$), 1.3-1.9 (32H, s x 2 + m, C($CH_3$)$_3$ x 2 + $CH_2$-CH($CH_3$)$_2$ + ($CH_2$)$_3$-$CH_2$-NH + -($CH_2$)$_3$- of piperidine), 2.4-2.6 (2H, m, CH-CO x 2), 2.6-3.1 (6H, m, $CH_2$-N-$CH_2$ + $CH_2$-$NH_2$), 4.28 (1H, m, NH-CH-CO), 5.13 (1H, m, NH), 6.41 and 6.57 (2H, m, NH x 2).

b) $N^a$-[4-(Hydroxyamino)-2(R)-isobutyl-3(RS)-methylsuccinyl]-$N^e$-acetimidoyl-L-lysine N-(piperidin-1-yl)amide • 1 acetate (Compound No. 40)

The procedure of Preparation Example 39-b was repeated using Compound No. 40-a(3.80 g, 6.85 mmol) to provide the title compound as a white solid (overall yield 16%), m.p.; 155-162°C, Rf value; 0.21 (chloroform:methanol:acetic acid= 5:2:1), 0.48 (n-BuOH:AcOH:water= 4:1:1), FABMS ($M^+$ +1): 455.

$^1$H-NMR ($CD_3OD$)δ ppm; 0.8-0.9 (6H, m, CH($CH_3$)$_2$), 1.0-1.1 (4H, m, CH($CH_3$)$_2$ + CO-CH-$CH_3$), 1.3-2.0 (17H, s + m, $CH_2$-CH($CH_3$)$_2$ + ($CH_2$)$_3$-$CH_2$-NH + -($CH_2$)$_3$- of piperidine + $CH_3CO_2H$), 2.0-2.3 (5H, m, CH-CO x 2 + C-$CH_3$), 2.3-2.7 (4H, m, $CH_2$-N-$CH_2$), 3.1-3.3 (2H, m, $CH_2$-NH), 4.21 (1H, m, NH-CH-CO).

Preparation Example 41

$N^a$-[4-(Hydroxyamino)-2(R)-isobutyl-3(R or S)-methylsuccinyl]-4'-acetimidoyliminomethyl-L-phenylalanine N-methylamide • 1 acetate (Compound No. 41)

The procedures of Preparation Examples 22-c, d and e were repeated using Compound No. 23-a to provide the title compound as a white solid (overall yield 10%), m.p.; 144-151°C, Rf value; 0.15 (chloroform:methanol:acetic acid= 5:2:1), 0.46 (n-BuOH: . AcOH:water= 4:1:1), FABMS ($M^+$ +1): 434.

$^1$H-NMR ($CD_3OD$)δ ppm; 0.6-0.9 (9H, m, CH($CH_3$)$_2$ + CO-CH-$CH_3$), 1.0-1.1 (1H, m, CH($CH_3$)$_2$), 1.3-1.6 (2H, m, $CH_2$-CH($CH_3$)$_2$), 1.90 (3H, s, $CH_3CO_2H$), 2.09 (1H, m, CH-CO), 2.25 (3H, s, C-$CH_3$), 2.47 (1H, m, CH-CO), 2.70 (3H, m, NH-$CH_3$), 2.92 and 3.07 (2H, m, $C_6H_4$-$CH_2$), 4.41 (2H, s, $C_6H_4$-$CH_2$-NH), 4.64 (1H, m, NH-CH-CO), 7.2-7.4 (4H, m, aromatic-H).

Preparation Example 42

$N^a$-[4-(Hydroxyamino)-2(R)-isobutyl-3(R or S)-methylsuccinyl]-4'-acetimidoyliminomethyl-L-phenylalanine N-(morpholin-4-yl)-amide • 1 acetate (Compound No. 42)

a) $N^a$-[4-tert-Butoxy-2(R)-isobutyl-3(RS)-methylsuccinyl]-L-4'-cyanophenylalanine N-(morpholin-4-yl)amide (Compound No. 42-a)

The procedures of Preparation Examples 22-a and b were repeated using Compound No. 2 and Compound No. 18 to provide the title compound as a white solid (yield 64%).

$^1$H-NMR ($CDCl_3$)δ ppm; 0.7-0.9 (6H, m, CH($CH_3$)$_2$), 0.9-1.2 (3H, m, CO-CH-$CH_3$), 1.3-1.7 (12H, s + m, C($CH_3$)$_3$ +

$\underline{CH_2}$-$\underline{CH}(CH_3)_2$), 2.3-2.6 (2H, m, $\underline{CH}$-CO x 2), 2.6-2.8 (4H, m, $\underline{CH_2}$-N-$\underline{CH_2}$), 2.9-3.3 (2H, m, $C_6H_4$-$\underline{CH_2}$), 3.77 (4H, m, $\underline{CH_2}$-O-$\underline{CH_2}$), 4.62 (1H, m, NH-$\underline{CH}$-CO), 6.47 (2H, m, NH x 2), 7.3-7.6 (4H, m, aromatic-H).

b) $N^a$-[4-(Hydroxyamino)-2(R)-isobutyl-3(R or S)-methylsuccinyl]-4'-acetimidoyliminomethyl-L-phenylalanine N-(morpholin-4-yl)amide • 1 acetate (Compound No. 42)

The procedures of Preparation Examples 22-c, d and e were repeated using Compound No. 42-a to provide the title compound as amorphous white powders (overall yield 10%).

FABMS ($M^+$ +1): 505
$^1$H-NMR ($CD_3OD$)δ ppm; 0.7-0.9 (6H, m, CH$\underline{(CH_3)_2}$), 0.9-1.2 (4H, m, $\underline{CH}(CH_3)_2$ + CO-CH-$\underline{CH_3}$), 1.3-1.7 (2H, m, $\underline{CH_2}$-$CH(CH_3)_2$), 1.90 (3H, s, $\underline{CH_3}CO_2H$), 2.25 (3H, s, C-$\underline{CH_3}$), 2.5-2.8 (6H, m, $\underline{CH}$-CO x 2 + $\underline{CH_2}$-N-$\underline{CH_2}$), 2.9-3.1 (2H, m, $C_6H_4$-$\underline{CH_2}$), 3.75 (4H, m, $\underline{CH_2}$-O-$\underline{CH_2}$), 4.02 (2H, m, NH-$\underline{CH_2}$), 4.52 (1H, m, NH-$\underline{CH}$-CO), 7.1-7.3 (4H, m, aromatic-H).

Preparation Example 43

$N^a$-[4-(Hydroxyamino)-2(R)-isobutyl-3(S)-hydroxysuccinyl]-4'-acetimidoyliminomethyl-L-phenylalanine N-methylamide • 1 acetate (Compound No. 43)

a) $N^a$-[3(S)-Hydroxy-2(R)-isobutyl-4-methoxysuccinyl]-L-4'-cyanophenylalanine N-methylamide (Compound No. 43-a)

The procedures of Preparation Examples 22-a and b were repeated using Compound No. 5 and Compound No. 21. The resultant product was re-solidified from chloroform-n-hexane to provide the title compound as a white solid (1.36 g, 50%).

$^1$H-NMR ($CDCl_3$)δ ppm; 0.7-1.1 (6H, m, CH$\underline{(CH_3)_2}$), 1.0-1.6 (3H, m, $\underline{CH_2}$-$\underline{CH}(CH_3)_2$), 2.4-3.5 (6H, d + m, J = 4.8Hz, NH-$\underline{CH_3}$ + $\underline{CH}$-CO + $\underline{CH_2}$-$C_6H_4$), 3.80 (3H, s, O$\underline{CH_3}$), 4.24 (1H, m, NH-$\underline{CH}$-CO), 4.4-4.7 (1H, m, HO-$\underline{CH}$-CO), 6.35 (1H, m, NH), 6.76 (1H, m, OH), 7.4-7.6 (5H, m, aromatic-H + NH).

b) $N^a$-[4,3(S)-Dihydroxy-2(R)-isobutylsuccinyl]-L-4'-cyanophenylalanine N-methylamide (Compound No. 43-b)

To a solution of Compound No. 43-a (1.95 g, 5.0 mmol) in methanol (20 ml) was added 2N aqueous sodium hydroxide (10 ml, 20 mmol) with stirring at 0°C, and the mixture was stirred for 2 hours. After methanol was evaporated under reduced pressure, the reaction solution was adjusted with 6N hydrochloric acid to pH 2. The target product was extracted with AcOEt (10 ml x 2), and washed with saturated aqueous sodium chloride. The organic layer was dried over anhydrous $MgSO_4$, and evaporated under reduced pressure. The resulting residue was purified by column chromatography (silica gel; 30 g, eluted with a mixture of chloroform:methanol= 5:1 to 2:1) to give the title compound as a white solid (1.61 g, 86%), m.p.; 172-173°C, Rf value; 0.52 (chloroform:methanol:acetic acid= 5:2:1).

$^1$H-NMR ($CD_3OD$)δ ppm; 0.79 (6H, m, CH$\underline{(CH_3)_2}$), 1.0-1.3 (1H, m, $\underline{CH}(CH_3)_2$), 1.5-1.6 (2H, m, $\underline{CH_2}$-$CH(CH_3)_2$), 2.6-2.8 (4H, s + m, NH-$\underline{CH_3}$ + $\underline{CH}$-CO), 3.01 and 3.33 (m, $C_6H_4$-$\underline{CH_2}$), 4.14 (1H, d, NH-$\underline{CH}$-CO), 4.65 (1H, m, J=5.4Hz, HO-$\underline{CH}$-CO), 7.4-7.6 (4H, m, aromatic-H).

c) $N^a$-[4,3(S)-Dihydroxy-2(R)-isobutylsuccinyl]-4'-aminomethyl-L-phenylalanine N-methylamide • 1 hydrochloride (Compound No. 43-c)

To a solution of Compound No. 43-b (500 mg, 1.33mmol) in a mixture of DMF (10 ml) and conc. hydrochloric acid (550 μl, 6.66 mmol) was added 5% palladium on carbon (50% wet catalyst, 500 mg), and the mixture was vigorously stirred under hydrogen atmosphere for 15 hours at room temperature. The catalyst was filtered off and then DMF was evaporated under reduced pressure. To the residue was added AcOEt to solidify a product which was collected by filtration to give the title compound as a white solid (479 mg, 82%).

$^1$H-NMR ($D_2O$)δ ppm; 0.64 (6H, m, CH$\underline{(CH_3)_2}$), 0.9-1.4 (3H, m, $\underline{CH_2}$-$\underline{CH}(CH_3)_2$), 2.4-2.6 (4H, s + m, NH-$\underline{CH_3}$ + $\underline{CH}$-CO), 2.89 and 3.05 (1H each, m, $C_6H_4$-$\underline{CH_2}$), 4.01 (2H, s, $\underline{CH_2}$-$NH_2$), 4.4-4.7 (2H, m, HO-$\underline{CH}$-CO + NH-$\underline{CH}$-CO), 7.1-7.3 (4H, m, aromatic-H).

d) $N^a$-[4-(Hydroxyamino)-2(R)-isobutyl-3(S)-hydroxysuccinyl]-4'-acetimidoyliminomethyl-L-phenylalanine N-methylamide • 1 acetate (Compound No. 43)

Compound No. 43-c (330 mg, 0.79 mmol) was dissolved in DMF (10 ml), and cooled to 0°C, followed by addition of TEA (342 µl, 2.45 mmol) and ethyl acetimidate hydrochloride (110 mg, 0.87 mmol). The mixture was stirred for 30 minutes at 0°C and for another 30 minutes at room temperature, to which was HOBT (120 mg, 0.87 mmol), O-benzylhydroxylamine hydrochloride (260 mg, 1.66 mmol), EDC (230 mg, 1.19 mmol) and TEA (230 µl, 1.66 mmol) successively at 0°C. The resultant mixture was stirred for 2 hours at 4°C, and then evaporated under reduced pressure. The residue was purified by column chromatography (silica gel; 20 g, eluted with from a mixture of chloroform:methanol:acetic acid= 80:10:5 to a mixture of methanol:acetic acid= 1:1).

The purified product was dissolved in acetic acid (20 ml), followed by addition of 5% palladium on carbon (50% wet catalyst, 300 mg). The mixture was vigorously stirred under hydrogen atmosphere for 2 hours at room temperature. The catalyst was filtered off and then acetic acid was evaporated under reduced pressure. AcOEt (20 ml) was added to the residue, leading to precipitation of solids. The resulting solid was collected by filtration to give the title compound as a white solid (250 mg, 64%), Rf value; 0.16 (chloroform:methanol:acetic acid= 5:2:1), 0.50 (n-BuOH:AcOH: water= 4:1:1), FABMS ($M^+$ +1): 436.

$^1$H-NMR ($CD_3OD$)δ ppm; 0.83 (6H, m, CH$\underline{(CH_3)_2}$), 1.1-1.6 (3H, m, $\underline{CH_2}$-$\underline{CH}(CH_3)_2$), 1.97 (3H, s, $\underline{CH_3}CO_2H$), 2.25 (3H, s, C-$\underline{CH_3}$), 2.6-2.8 (4H, s + m, NH-$\underline{CH_3}$ + $\underline{CH}$-CO), 2.9-3.3 (2H, m, $C_6H_4$-$\underline{CH_2}$), 4.03 (2H, s, $\underline{CH_2}$-NH), 4.4-4.6 (2H, m, NH-$\underline{CH}$-CO + HO-$\underline{CH}$-CO), 7.28 (4H, m, aromatic-H).

Preparation Example 44

$N^a$-[4-(Hydroxyamino)-2(R)-isobutyl-3(RS)-(3-phenylpropyl)-succinyl]-$N^e$-acetimidoyl-L-lysine N-(2-N',N'-dimethylaminoethyl)amide • 2 acetate (Compound No. 44)

a) $N^a$-[4-tert-Butoxy-2(R)-isobutyl-3(RS)-(3-phenylpropyl)succinyl]-$N^e$-tert-butyloxycarbonyl-L-lysine N-(2-N',N'-dimethylaminoethyl)amide (Compound No. 44-a)

The procedure of Preparation Example 31-a was repeated using Compound No. 3 and Compound No. 16 to provide the title compound as a white solid (yield 69%), m.p.; 102-105°C, Rf value; 0.38 (chloroform:methanol= 10:1).

$^1$H-NMR ($CDCl_3$)δ ppm; 0.86 (6H, m, CH$\underline{(CH_3)_2}$), 1.2-1.9 (31H, m, $\underline{(CH_2)_3}$-$CH_2$-NH + $\underline{(CH_2)_2}$-$CH_2$-Ph + C$\underline{(CH_3)_3}$ x 2 + $\underline{CH_2CH}(CH_3)_2$), 2.22 (6H, s x 2, N-$\underline{CH_3}$ x 2), 2.3-2.7 (6H, m, $\underline{CH}$-CO x 2 + $\underline{CH_2}$-Ph + $\underline{CH_2}$-N$(CH_3)_2$), 3.09 (2H, m, NH-$\underline{CH_2}$-$CH_2$-N), 3.32 (2H, m, OC(=O)NH-$\underline{CH_2}$), 4.38 (1H, m, NH-$\underline{CH}$-CO), 4.65 (1H, m, NH), 6.42 and 6.55 (1H each, m, NH x 2), 7.1-7.3 (5H, m, aromatic-H).

b) $N^a$-[4-(Hydroxyamino)-2(R)-isobutyl-3(RS)-(3-phenylpropyl)succinyl]-$N^e$-acetimidoyl-L-lysine N-(2-N',N'-dimethylaminoethyl)amide • 2 acetate (Compound No. 44)

The procedure of Preparation Example 39-b was repeated using Compound No. 44-a (2.21 g, 3.41 mmol) to provide the title compound as amorphous white powders (overall yield 12%), m.p.; 95-101°C, Rf value; 0.082 (chloroform:methanol:acetic acid= 5:2:1), 0.14 (n-BuOH:AcOH:water= 4:1:1), FABMS ($M^+$ +2): 548.

$^1$H-NMR ($CD_3OD$)δ ppm; 0.87 (6H, m, CH$\underline{(CH_3)_2}$), 1.2-1.9 (13H, m, $\underline{(CH_2)_3}$-$CH_2$-NH + $\underline{(CH_2)_2}$-$CH_2$-Ph + $\underline{CH_2CH}(CH_3)_2$), 1.95 (6H, s x 2, $\underline{CH_3}CO_2H$ x 2), 2.21 (3H, s, C-$\underline{CH_3}$), 2.4-2.7 (4H, m, $\underline{CH}$-CO x 2 + $\underline{CH_2}$-Ph), 2.92 and 2.95 (6H, s each, N-$\underline{CH_3}$ x 2), 3.2-3.4 (4H, m, NH-$\underline{CH_2}$-$\underline{CH_2}$-N), 3.5-3.7 (2H, m, NH-$\underline{CH_2}$), 4.15 (1H, m, NH-$\underline{CH}$-CO), 7.1-7.3 (5H, m, aromatic-H).

Preparation Example 45

$N^a$-[4-(Hydroxyamino)-2(R)-isobutyl-3(RS)-methylsuccinyl]-$N^e$-propionimidoyl-L-lysine N-methylamide • 1 acetate (Compound No. 45)

The procedures of Preparation Examples 22-c, d and e except that ethyl acetimidate hydrochloride was replaced with ethyl propionimidate hydrochloride were repeated using Compound No. 22-b to provide the title compound as a white solid (overall yield 42%), m.p.; 113-119°C, Rf value; 0.15 (chloroform:methanol:acetic acid= 5:2:1), 0.47 (n-BuOH:AcOH: water= 4:1:1), FABMS ($M^+$ +1): 400.

$^1$H-NMR ($CD_3OD$)δ ppm; 0.8-0.9 (6H, m, CH($CH_3$)$_2$), 1.0-1.1 (4H, m, CO-CH-$CH_3$ + CH($CH_3$)$_2$), 1.27 (3H, m, $CH_2$-$CH_3$), 1.3-1.9 (8H, m, $CH_2$-CH($CH_3$)$_2$ + ($CH_2$)$_3$-$CH_2$-NH), 1.94 (3H, s, $CH_3CO_2H$), 2.2-2.6 (4H, m, $CH_2$-$CH_3$ + CH-CO x 2), 2.70 (3H, m, NH-$CH_3$), 3.21 (2H, m, $CH_2$-NH), 4.30 (1H, m, NH-CH-CO).

Preparation Example 46

$N^a$-[4-(Hydroxyamino)-2(R)-isobutyl-3(RS)-[3-(4'-guanidinophenyl)propyl]succinyl]-L-ornithine-N-methylamide • 2 acetate (Compound No. 46)

a) $N^a$-[4-tert-Butoxy-2(R)-isobutyl-3(RS)-[3-[p-($N^1$,$N^2$-dibenzyloxycarbonylguanidino)phenyl]propyl]succinyl]-$N^e$-benzyloxycarbonyl-L-lysine N-methylamide (Compound No. 46-a)

The procedures of Preparation Examples 22-a and b were repeated using Compound No. 7 and Compound No. 20 to provide the title compound as a colorless oil (yield 93%).

$^1$H-NMR ($CDCl_3$)δ ppm; 0.86 (6H, m, CH($CH_3$)$_2$), 1.0-1.9 (20H, s + m, ($CH_2$)$_2$-$CH_2$-NH + ($CH_2$)$_2$-$CH_2$-$C_6H_4$ + C($CH_3$)$_3$ + $CH_2$CH($CH_3$)$_2$), 2.2-2.8 (7H, m, $CH_2$-$C_6H_4$ + CH-CO x 2 + NH-$CH_3$), 2.9-3.5 (2H, m, $CH_2$-NH), 4.47 (1H, m, NH-CH-CO), 5.0-5.3 (6H, m, $CH_2$-Ph x 3), 6.4-6.7 (2H, m, NH x 2), 7.0-7.2 (2H, m, NH x 2), 7.2-7.5 (16H, m, aromatic-H + NH).

b) $N^a$-[4-(Hydroxyamino)-2(R)-isobutyl-3(RS)-[3-(4'-guanidinophenyl)propyl]succinyl]-L-ornithine-N-methylamide • 2 acetate (Compound No. 46)

The procedures of Preparation Examples 23-b and c were repeated using Compound No. 46-a to provide the title compound as amorphous white powders (overall yield 25%), Rf value; 0.04 (chloroform:methanol:acetic acid= 5:2:1), 0.34 (n-BuOH:AcOH: water= 4:1:1), FABMS ($M^+$ +1): 492.

$^1$H-NMR ($CD_3OD$)δ ppm; 0.86 (6H, m, CH($CH_3$)$_2$), 1.0-1.8 (11H, m, ($CH_2$)$_2$-$CH_2$-$NH_2$ + ($CH_2$)$_2$-$CH_2$-$C_6H_4$ + $CH_2$CH($CH_3$)$_2$), 1.89 (6H, s x 2, $CH_3CO_2H$), 2.17 (1H, m, CH-CO), 2.5-2.9 (8H, s + m, $CH_2$-$C_6H_4$ + $CH_2$-NH + CH-CO + NH-$CH_3$), 4.28 (1H, m, NH-CH-CO), 7.1-7.3 (4H, m, aromatic-H).

Preparation Example 47

$N^a$-[4-(Hydroxyamino)-2(R)-isobutyl-3(R or S)-(3-phenylpropyl)succinyl]-4'-aminomethyl-L-phenylalanine N-methylamide • 1 acetate

The procedure of Preparation Example 23 was repeated using Compound No. 5 and Compound No. 16 to provide the title compound as a white solid.

FABMS ($M^+$ +1):497.
$^1$H-NMR ($CD_3OD$)δ ppm; 0.8-1.1 (7H, m, CH($CH_3$)$_2$), 1.3-1.6 (6H, m, CH-($CH_2$)$_2$ + $CH_2$-CH($CH_3$)$_2$), 1.89 (3H, s, $CH_3CO_2H$), 2.10 (1H, m, CH-CO), 2.3-2.5 (3H, m, $CH_2$-Ph + CH-CO), 2.63 (3H, s, NH-$CH_3$), 2.8-3.1 (2H, m, $C_6H_4$-$CH_2$), 3.93 (2H, m, $CH_2$-$NH_2$), 4.58 (1H, m, NH-CH-CO), 7.0-7.4 (9H, m, aromatic-H).

Preparation Example 48

$N^a$-[4-(Hydroxyamino)-2(R)-isobutyl-3(R or S)-(p-aminomethylbentyl)succinyl]-L-ornithine N-methylamide • 2 acetate

The procedure of Preparation Example 23 was repeated using 4-(p-benzyloxycarbonylaminomethylphenyl)-3(RS)-tert-butyloxycarbonyl-2(R)-isobutylbutanoic acid and Compound No. 7 to provide the title compound as a white solid.

FABMS ($M^+$ ): 436.
$^1$H-NMR ($CD_3OD$)δ ppm; 0.7-1.0 (6H, m, CH($CH_3$)$_2$), 1.08 (1H, m, CH($CH_3$)$_2$), 1.3-1.9 (6H, m, CH-($CH_2$)$_2$ + $CH_2$-CH($CH_3$)$_2$), 1.84 (6H, s, $CH_3CO_2H$× 2), 2.41 (1H, m, CH-CO), 2.5-3.0 (8H, m, NH-$CH_3$ + $CH_2$-$NH_2$ + CH-CO + $C_6H_4$-$CH_2$), 3.95(2H, m, $C_6H_4$-$CH_2$-$NH_2$), 4.34 (1H, m, NH-CH-CO), 7.1-7.4 (4H, m, aromatic-H).

Preparation Example 49

$N^a$-[4-(Hydroxyamino)-2(R)-isobutyl-3(R or S)-(p-aminomethylbenzyl)succinyl]-4'-aminomethyl-L-phenylalanine N-methylamide • 2 acetate

The procedure of Preparation Example 23 was repeated using 4-(p-benzyloxycarbonylaminomethylphenyl)-3(RS)-tert-butyloxycarbonyl-2(R)-isobutylbutanoic acid and Compound No. 5 to provide the title compound as a white solid.

FABMS ($M^+$ +1): 498.
$^1$H-NMR ($CD_3OD$)δ ppm; 0.7-0.85 (6H, m, CH$\underline{(CH_3)_2}$), 0.90 (1H, m, $\underline{CH}(CH_3)_2$), 1.2-1.4 (2H, m, $\underline{CH_2}$-CH$(CH_3)_2$), 1.84 (6H, s, $\underline{CH_3}CO_2H \times 2$), 2.07 (1H, m, $\underline{CH}$-CO), 2.41 (1H, m, $\underline{CH}$-CO), 2.64 (3H, m, NH-$\underline{CH_3}$), 2.81 and 3.06 (1H each, m, $C_6H_4$-$\underline{CH_2}$), 3.2 (m, $C_6H_4$-$\underline{CH_2}$), 3.93 (2H, m, $\underline{CH_2}$-$NH_2$), 4.77 (1H, m, NH-$\underline{CH}$-CO), 6.84 , 7.05 and 7.1-7.3 (8H, m, aromatic-H).

Preparation Example 50

$N^a$-[4-(Hydroxyamino)-2(R)-isobutyl-3(R or S)-[p-(p-toluenesulfonamidomethyl)benzyl]succinyl]-L-arginine N-methylamide • 1 acetate

The procedure of Preparation Example 23 was repeated using 4-[p-(p-toluenesulfonamidomethyl)phenyl]-3(RS)-tert-butyloxycarbonyl-2(R)-isobutylbutanoic acid and Compound No. 9 to provide the title compound as a white solid.

FABMS ($M^+$ ): 632.
$^1$H-NMR ($CD_3OD$)δ ppm; 0.8-1.0 (6H, m, CH$\underline{(CH_3)_2}$), 1.0-1.1 (1H, m, $\underline{CH}(CH_3)_2$), 1.4-1.8 (6H, m, CH-$\underline{(CH_2)_2}$ + $\underline{CH_2}$-CH$(CH_3)_2$), 1.89 (3H, s, $\underline{CH_3}CO_2H$), 2.39 (1H, m, $\underline{CH}$-CO), 2.43 (3H, s, $C_6H_4$-$\underline{CH_3}$), 2.5-2.8 (6H, m, $\underline{CH}$-CO + NH-$\underline{CH_3}$ + $C_6H_4$-$\underline{CH_2}$), 3.18 (2H, m, $\underline{CH_2}$-NH), 3.93 (2H, s, $C_6H_4$-$\underline{CH_2}$-NH), 4.3B (1H, m, NH-$\underline{CH}$-CO), 7.01 , 7.21 , 7.36 and 7.71 (2H each, m, aromatic-H).

Preparation Example 51

$N^a$-[4-(Hydroxyamino)-2(R)-isobutyl-3(R or S)-(p-phthalimidomethylbenzyl)succinyl]-L-arginine N-methylamide • 1 acetate

The procedure of Preparation Example 23 was repeated using 4-(p-phthalimidomethylphenyl)-3(RS)-tert-butyloxycarbonyl-2(R)-isobutylbutanoic acid and Compound No. 9 to provide the title compound as a white solid.

FABMS ($M^+$ ): 608.
$^1$H-NMR ($CD_3OD$)δ ppm; 0.8-0.9 (6H, m, CH$\underline{(CH_3)_2}$), 1.06 (1H, m, $\underline{CH}(CH_3)_2$), 1.4-1.85 (6H, m, CH-$\underline{(CH_2)_2}$ + $\underline{CH_2}$-CH$(CH_3)_2$), 1.90 (3H, s, $\underline{CH_3}CO_2H$), 2.39 (1H, m, $\underline{CH}$-CO), 2.5-2.8 (6H, m, $\underline{CH}$-CO + NH-$\underline{CH_3}$ + $C_6H_4$-$\underline{CH_2}$), 3.16 (2H, m, $\underline{CH_2}$-NH), 4.39 (1H, m, NH-$\underline{CH}$-CO), 4.75 (2H, s, $C_6H_4$-$\underline{CH_2}$-N), 7.05 and 7.23 (2H each, m, aromatic-H), 7.7-7.9 (4H, m, aromatic-H).

Preparation Example 52

$N^a$-[4-(Hydroxyamino)-2(R)-isobutyl-3(R or S)-(p-methanesulfonamidomethylbenzyl)succinyl]-L-arginine N-methylamide • 1 acetate

The procedure of Preparation Example 23 was repeated using 4-(p-methanesulfonamidomethylphenyl)-3(RS)-tert-butyloxycarbonyl-2(R)-isobutylbutanoic acid and Compound No. 9 to provide the title compound as a white solid.

$^1$H-NMR ($CD_3OD$)δ ppm; 0.8-0.95 (6H, m, CH$\underline{(CH_3)_2}$), 1.08 (1H, m, $\underline{CH}(CH_3)_2$), 1.4-1.85 (6H, m, CH-$\underline{(CH_2)_2}$ + $\underline{CH_2}$-CH$(CH_3)_2$), 1.89 (3H, s, $\underline{CH_3}CO_2H$), 1.96 (3H, s, $\underline{CH_3}$-$SO_2$), 2.41 (1H, m, $\underline{CH}$-CO), 2.5-2.8 (6K, m, $\underline{CH}$-CO + NH-$\underline{CH_3}$ + $C_6H_4$-$\underline{CH_2}$), 3.26 (2H, m, $\underline{CH_2}$-NH), 4.28 (2H, s, $C_6H_4$-$\underline{CH_2}$-NH), 4.39 (1H, m, NH-$\underline{CH}$-CO), 7.05 and 7.14 (2H each, m, aromatic-H).

Preparation Example 53

$N^a$-[4-(Hydroxyamino)-2(R)-isobutyl-3(R or S)-(m-aminomethylbenzyl)succinyl]-L-alanine N-methylamide • 1 acetate

The procedure of Preparation Example 23 was repeated using 4-(m-benzyloxycarbonylaminomethylphenyl)-3(RS)-tert-butyloxycarbonyl-2(R)-isobutylbutanoic acid and $N^a$-tert-butyloxycarbonyl-L-alanine N-methylamide to provide the title compound as a white solid.

FABMS ($M^+$ ): 393.
$^1$H-NMR ($CD_3OD$)δ ppm; 0.85-0.95 (6H, m, CH$\underline{(CH_3)_2}$), 1.08 (1H, m, $\underline{CH}(CH_3)_2$), 1.36 (3H, d, J =7.2Hz, CH-$\underline{CH_3}$), 1.4-1.7 (2H, m, $\underline{CH_2}$-CH($CH_3)_2$), 1.91 (3H, s, $\underline{CH_3}CO_2H$), 2.42 (1H, m, $\underline{CH}$-CO), 2.6-2.9 (6H, m, $\underline{CH}$-CO + NH-$\underline{CH_3}$ + $C_6H_4$-$\underline{CH_2}$), 4.03 (2H, s, $C_6H_4$-$\underline{CH_2}$-$NH_2$), 4.40 (1H, m, NH-$\underline{CH}$-CO), 7.1-7.4 (4H, m, aromatic-H).

Preparation Example 54

$N^a$-[4-(Hydroxyamino)-2(R)-isobutyl-3(R or S)-(2-phenoxyethyl)succinyl]-L-arginine N-methylamide • 1 acetate

The procedure of Preparation Example 23 was repeated using 5-phenoxy-3(RS)-tert-butyloxycarbonyl-2(R)-isobutylpentanoic acid and Compound No. 9 to provide the title compound as a white solid.

FABMS ($M^+$ ): 479.
$^1$H-NMR ($CD_3OD$)δ ppm; 0.8-0.95 (6H, m, CH$\underline{(CH_3)_2}$), 1.10 (1H, m, $\underline{CH}(CH_3)_2$), 1.4-1.85 (8H, m, $\underline{CH_2}$-$CH_2$-O + CH-$\underline{(CH_2)_2}$ + $\underline{CH_2}$-CH($CH_3)_2$), 1.89 (3H, s, $\underline{CH_3}CO_2H$), 2.45 (1H, m, $\underline{CH}$-CO), 2.65 (1H, m, $\underline{CH}$-CO), 2.72 (3H, s, NH-$\underline{CH_3}$), 3.15 (2H, m, $\underline{CH_2}$-NH), 3.8-4.0 (O-$\underline{CH_2}$), 4.34 (1H, m, NH-$\underline{CH}$-CO), 6.88 and 7.23 (2H each, m, aromatic-H).

Preparation Example 55

$N^a$-[4-(Hydroxyamino)-2(R)-isobutyl-3(R or S)-(3-cyclohexylpropyl)succinyl]-L-arginine N-methylamide • 1 acetate

The procedure of Preparation Example 23 was repeated using 6-cyclohexyl-3(RS)-tert-butyloxycarbonyl-2(R)-isobutylhexanoic acid and Compound No. 9 to provide the title compound as a white solid.

FABMS ($M^+$ + 1): 484.
$^1$H-NMR (DMSO-$d_6$)δ ppm; 0.6-1.8 (33H, m, $\underline{CH_2-CH(CH_3)_2}$ + CH-$\underline{(CH_2)_3-C_5H_{11}}$ + CH-$\underline{(CH_2)_2}$ + $\underline{CH_3}CO_2H$), 1.99 (1H, m, $\underline{CH}$-CO), 2.49 (1H, m, $\underline{CH}$-CO), 2.55 (3H, d, J =4.4Hz, NH-$\underline{CH_3}$), 3.00 (2H, m, $\underline{CH_2}$-NH), 4.20 (1H, m, NH-$\underline{CH}$-CO), 8.05 and 8.23 (1H each, m NH× 2).

Preparation Example 56

$N^a$-[4-(Hydroxyamino)-2(R)-(2-naphthylmethyl)-3(R or S)-(3-phenylpropyl)succinyl]-L-arginine N-methylamide • 1 acetate

The procedure of Preparation Example 23 was repeated using 6-phenyl-3(RS)-tert-butyloxycarbonyl-2(R)-(2-naphthylmethyl)hexanoic acid and Compound No. 9 to provide the title compound as a white solid.

FABMS ($M^+$ + 1): 562.
$^1$H-NMR ($CD_3OD$)δ ppm; 1.2-1.8 (8H, m, CH-$\underline{(CH_2)_2}$× 2), 1.90 (3H, s, $\underline{CH_3}CO_2H$), 2.3-3.1 (11H, m, $\underline{CH}$-CO × 2 + $\underline{CH_2}$-Ph + $\underline{CH_2}$-Naphthyl + $\underline{CH_2}$-NH + N-$\underline{CH_3}$), 4.08 (1H, m, NH-$\underline{CH}$-CO), 7.1-7.9 (12H, m, aromatic-H).

Preparation Example 57

$N^a$-[4-(Hydroxyamino)-2(R)-isobutyl-3(R or S)-(o-aminomethylbenzyl)succinyl]-L-alanine N-methylamide • 1 acetate

The procedure of Preparation Example 23 was repeated using 4-(o-benzyloxycarbonylaminomethylphenyl)-3(RS)-tert-butyloxycarbonyl-2(R)-isobutylbutanoic acid and $N^a$-tert-butyloxycarbonyl-L-alanine N-methylamide to provide the title compound as a white solid.

FABMS ($M^+$ ): 393.

$^1$H-NMR ($CD_3OD$)δ ppm; 0.8-1.0 (6H, m, CH($CH_3$)$_2$), 1.09 (1H, m, CH($CH_3$)$_2$), 1.38 (3H, d, J =4.7Hz, CH-$CH_3$), 1.60 (2H, m, $CH_2$-CH($CH_3$)$_2$), 1.89 (3H, s, $CH_3CO_2H$), 2.53 (1H, m, CH-CO), 2.65-2.95 (6H, m, CH-CO + NH-$CH_3$ + $C_6H_4$-$CH_2$), 4.13 (2H, s, $CH_2$-$NH_2$), 4.42 (1H, m, NH-CH-CO), 7.1-7.4 (4H, m, aromatic-H).

Preparation Example 58

$N^a$-[4-(Hydroxyamino)-2(R)-isobutyl-3(RS)-[3-(p-aminomethylphenyl)propyl]succinyl]-L-alanine N-methylamide • 1 acetate

The procedure of Preparation Example 23 was repeated using 6-(p-benzyloxycarbonylaminomethylphenyl)-3(RS)-tert-butyloxycarbonyl-2(R)-isobutylhexanoic acid and $N^a$-tert-butyloxycarbonyl-L-alanine N-methylamide to provide the title compound as a white solid.

FABMS ($M^+$ ): 421.

$^1$H-NMR ($CD_3OD$)δ ppm; 0.7-0.85 (6H, m, CH($CH_3$)$_2$), 0.93 (1H, m, CH($CH_3$)$_2$), 1.17 (3H, d, J =7.0Hz, CH-$CH_3$), 1.2-1.6 (6H, m, CH-($CH_2$)$_2$ + $CH_2$-CH($CH_3$)$_2$), 1.80 (3H, s, $CH_3CO_2H$), 2.06 (1H, m, CH-CO), 2.4-2.55 (3H, m, CH-CO + $C_6H_4$-$CH_2$), 2.62 (3H, s, NH-$CH_3$), 3.92 (2H, s, $CH_2$-$NH_2$), 4.20 (1H, m, NH-CH-CO), 7.11 and 7.21 (2H each, m, aromatic-H).

Preparation Example 59

$N^a$-[4-(Hydroxyamino)-2(R)-isobutyl-3(R or S)-(m-isopropyliminomethylbenzyl)succinyl]-L-alanine N-methylamide • 1 acetate

To a mixture of Compound No. 53 (400 mg, 0.884 mmol), acetone (154 mg, 2.65 mmol) and methanol (8 ml) was added sodium cyanoborohydride (67 mg, 1.07 mmol) while cooling in an ice bath, and the mixture was stirred overnight at room temperature. After methanol was evaporated under reduced pressure, the residue was dissolved in 5% aqueous acetic acid, purified by column reversed phase chromatography (25 g of Chromatorex ODS-1020T, Fuji Silysia Chemical, Japan; eluted with a gradient of 1% to 5% methanol/0.1% aqueous acetic acid), and then lyophilized to give the title compound as a white solid.

FABMS ($M^+$ ): 435.

$^1$H-NMR ($CD_3OD$)δ ppm; 0.8-1.0 (6H, m, CH($CH_3$)$_2$), 1.08 (1H, m, CH($CH_3$)$_2$), 1.36 (9H, m, CH-$CH_3$ + NH-CH($CH_3$)$_2$), 1.55 (2H, m, $CH_2$-CH($CH_3$)$_2$), 1.89 (3H, s, $CH_3CO_2H$), 2.22 (1H, m, CH-CO), 2.6-2.9 (6H, m, CH-CO + NH-$CH_3$ + $C_6H_4$-$CH_2$), 3.37 (1H, m, NH-CH($CH_3$)$_2$), 4.14 (2H, s, $CH_2$-$NH_2$), 4.40 (1H, m, NH-CH-CO), 7.15-7.4 (4H, m, aromatic-H).

Preparation Example 60

$N^a$-[4-(Hydroxyamino)-2(R)-isobutyl-3(R or S)-[3-(o-aminomethylphenyl)propyl]succinyl]-L-alanine N-methylamide • 1 acetate

The procedure of Preparation Example 23 was repeated using 6-(o-benzyloxycarbonylaminomethylphenyl)-3(RS)-tert-butyloxycarbonyl-2(R)-isobutylhexanoic acid and $N^a$-tert-butyloxycarbonyl-L-alanine N-methylamide to give the title compound as a white solid.

FABMS ($M^+$ ): 421.

$^1$H-NMR (DMSO-$d_6$)δ ppm; 0.7-1.0 (7H, m, CH($CH_3$)$_2$), 1.13 (3H, d, J=6.9Hz, CH-$CH_3$), 1.1-1.6 (6H, m, CH-($CH_2$)$_2$ + $CH_2$-CH($CH_3$)$_2$), 1.81 (3H, s, $CH_3CO_2H$), 2.06 (1H, m, CH-CO), 2.4-2.6 (m, CH-CO + $C_6H_4$-$CH_2$ + NH-$CH_3$), 3.70 (2H, s, $CH_2$-$NH_2$), 4.23 (1H, m, NH-CH-CO), 7.0-7.2 and 7.32 (4H, m, aromatic-H), 7.63 and 8.16 (1H each, m, NH× 2).

Preparation Example 61

$N^a$-[4-(Hydroxyamino)-2(R)-isobutyl-3(R or S)-(3-(p-aminomethylphenyl)propyl]succinyl]-L-lysine N-methylamide • 2 acetate

The procedure of Preparation Example 23 was repeated using 6-(p-benzyloxycarbonylaminomethylphenyl)-3(RS)-tert-butyloxycarbonyl-2(R)-isobutylhexanoic acid and Compound No. 1 to give the title compound as a white solid.

FABMS ($M^+$ ): 479.
$^1$H-NMR ($CD_3OD$)δ ppm; 0.8-1.0 (6H, m, CH($\underline{CH_3}$)$_2$), 1.06 (1H, m, $\underline{CH}$($CH_3$)$_2$), 1.2-1.9 (12H, m, CH-$\underline{(CH_2)_3}$ + CH-$\underline{(CH_2)_2}$ + $\underline{CH_2}$-CH($CH_3$)$_2$), 1.92 (6H, s, $\underline{CH_3}CO_2H$× 2), 2.19 (1H, m, $\underline{CH}$-CO), 2.45-2.7 (3H, m, $\underline{CH}$-CO + $C_6H_4$-$\underline{CH_2}$), 2.72(3H, s, NH-$\underline{CH_3}$), 2.91 (2H, m, $\underline{CH_2}$-$NH_2$), 4.06 (2H, s, $C_6H_4$-$\underline{CH_2}$-$NH_2$), 4.28 (1H, m, NR-$\underline{CH}$-CO), 7.22 and 7.35 (2H each, m, aromatic-H).

Preparation Example 62

$N^a$-[4-(Hydroxyamino)-2(R)-isobutyl-3(R or S)-[5-(acetimidoylimino)pentyl]succinyl]-L-alanine N-methylamide

$N^a$-[4-(Hydroxyamino)-2(R)-isobutyl-3(R or S)-(5-aminopentyl)succinyl]-L-alanine N-methylamide • 1 acetate (200 mg, 0.48 mmol) was dissolved in DMF (6 ml), and then cooled to 0°C, followed by addition of TEA (200 ml, 1.44 mmol) and ethyl acetimidate hydrochloride (124 mg, 1.01 mmol). The mixture was stirred for 2 hours. DMF was evaporated under reduced pressure and the residue was dissolved in 5% aqueous acetic acid, purified by column reversed phase chromatography (25 g of Chromatorex ODS-1020T, Fuji Silysia Chemical, Japan; eluted with distilled water and 0.1% aqueous acetic acid), and then lyophilized to give the title compound as a white solid.

FABMS ($M^+$ ): 400.
$^1$H-NMR ($CD_3OD$)δ ppm; 0.8-0.95 (6H, m, CH($\underline{CH_3}$)$_2$), 1.04 (1H, m, $\underline{CH}$($CH_3$)$_2$), 1.2-1.7 (13H, m, CH-$\underline{(CH_2)_4}$ + CH-$\underline{CH_3}$ + $\underline{CH_2}$-CH($CH_3$)$_2$), 2.13 (1H, m, $\underline{CH}$-CO), 2.20 (3H, s, C-$\underline{CH_3}$), 2.54 (1H, m, $\underline{CH}$-CO), 2.72 (3H, s, NH-$\underline{CH_3}$), 3.19 (2H, m, $\underline{CH_2}$-NH), 4.34 (1H, m, NH-$\underline{CH}$-CO).

Preparation Example 63

$N^a$-[4-(Hydroxyamino)-2(R)-isobutyl-3(R or S)-[5-(isopropylimino)pentyl]succinyl]-L-alanine N-methylamide

The procedure of Preparation Example 59 was repeated using $N^a$-[4-(hydroxyamino)-2(R)-isobutyl-3(R or S)-(5-aminopentyl)succinyl]-L-alanine N-methylamide • 1 acetate to give the title compound as a white solid.

FABMS ($M^+$ ): 401.
$^1$H-NMR ($CD_3OD$)δ ppm; 0.8-0.95 (6H, m, CH($\underline{CH_3}$)$_2$), 1.06 (1H, m, $\underline{CH}$($CH_3$)$_2$), 1.25-1.8 (19H, m, NH-CH($\underline{CH_3}$)$_2$ + CH-$\underline{(CH_2)_4}$ + CH-$\underline{CH_3}$ + $\underline{CH_2}$-CH($CH_3$)$_2$), 2.15 (1H, m, $\underline{CH}$-CO), 2.56 (1H, m, $\underline{CH}$-CO), 2.60 (3M, s, N-$\underline{CH_3}$), 2.96 (2H, m, $\underline{CH_2}$-NH), 3.36 (m, NH-$\underline{CH}$($CH_3$)$_2$) 4.35 (1H, m, NH-$\underline{CH}$-CO).

Preparation Example 64

$N^a$-[4-(Hydroxyamino)-2(R)-isobutyl-3(R or S)-[5-(pyridin-4-ylmethylimino)pentyl]succinyl]-L-alanine N-methylamide • 1 acetate

The procedure of Preparation Example 59 was repeated using $N^a$-[4-(hydroxyamino)-2(R)-isobutyl-3(R or S)-(5-aminopentyl)-succinyl)-L-alanine N-methylamide • 1 acetate, except that acetone was replaced with 4-pyridylaldehyde, to give the title compound as a white solid.

FABMS ($M^+$ ): 450.
$^1$H-NMR ($CD_3OD$)δ ppm; 0.8-0.95 (6H, m, CH($\underline{CH_3}$)$_2$), 1.06 (1H, m, $\underline{CH}$($CH_3$)$_2$), 1.1-1.7 (13H, m, CH-$\underline{(CH_2)_4}$ + CH-$\underline{CH_3}$ + $\underline{CH_2}$-CH($CH_3$)$_2$), 1.93 (3H, s, $\underline{CH_3}CO_2H$), 2.13 (1H, m, $\underline{CH}$-CO), 2.54 (1H, m, $\underline{CH}$-CO), 2.71 (3H, s, N-$\underline{CH_3}$), 2.87 (2H, m, $\underline{CH_2}$-NH), 4.02 (2H, m, NH-$\underline{CH_2}$-Pyridyl), 4.40 (1H, m, NH-$\underline{CH}$-CO), 7.47 and 8.55 (2H each, m, aromatic-H).

Preparation Example 65

$N^a$-[4-(Hydroxyamino)-2(R)-isobutyl-3(R or S)-[3-(p-methoxycarbonylphenyl)propyl]succinyl]-L-lysine N-methylamide • 1 acetate

The procedure of Preparation Example 23 was repeated using 6-(p-methoxycarbonylphenyl)-3(RS)-tert-butyloxycarbonyl-2(R)-isobutylhexanoic acid and Compound No. 1 to give the title compound as a white solid.

FABMS ($M^+$ + 1): 508.
$^1$H-NMR ($CD_3OD$)δ ppm; 0.8-0.9 (6H, m, CH$\underline{(CH_3)_2}$), 1.04 (1H, m, $\underline{CH}(CH_3)_2$), 1.2-1.8 (12H, m, CH-$\underline{(CH_2)_3}$ + CH-$\underline{(CH_2)_2}$ + $\underline{CH_2}$-CH$(CH_3)_2$), 1.91 (6H, s, $\underline{CH_3}CO_2H$), 2.20 (1H, m, $\underline{CH}$-CO), 2.53 (1H, m, $\underline{CH}$-CO), 2.63 (2H, m, $C_6H_4$-$\underline{CH_2}$), 2.71 (3H, s, N-$\underline{CH_3}$), 2.89 (2H, m, $\underline{CH_2}$-$NH_2$), 3.88 (3H, s, -$CO_2\underline{CH_3}$), 4.24 (1H, m, NH-$\underline{CH}$-CO), 7.26 and 7.90 (2H each, m, aromatic-H).

Preparation Example 66

$N^a$-[4-(Hydroxyamino)-2(R)-isobutyl-3(R or S)-[2-(2-ethoxyethoxy)ethyl]succinyl]-L-lysine N-methylamide

The procedure of Preparation Example 23 was repeated using 5-(2-ethoxyethoxy)-3(RS)-tert-butyloxycarbonyl-2(R)-isobutylpentanoic acid and Compound No. 1 to give the title compound as a white solid.

FABMS ($M^+$ + 1) 448.
$^1$H-NMR ($CD_3OD$)δ ppm; 0.8-0.95 (6H, m, CH$\underline{(CH_3)_2}$), 1.07 (1H, m, $\underline{CH}(CH_3)_2$), 1.20 (3H,t, J = 7.0Hz, $CH_2$-$\underline{CH_3}$), 1.3-1.9 (10H, m, CH-$\underline{(CH_2)_3}$ + CH-$\underline{CH_2}$ +$\underline{CH_2}$-CH$(CH_3)_2$), 2.32 (1H, m, $\underline{CH}$-CO), 2.58 (1H, m, $\underline{CH}$-CO), 2.72 (3H, s, N-$\underline{CH_3}$), 2.92 (2H, m, $\underline{CH_2}$-$NH_2$), 3.2-3.4 (8H, m, O-$\underline{CH_2} \times 4$), 4.32 (1H, m, NH-$\underline{CH}$-CO).

Preparation Example 67

$N^a$-[4-(Hydroxyamino)-2(R)-isobutyl-3(R or S)-[3-(p-carboxyphenyl)propyl]succinyl]-L-lysine N-methylamide

Alkaline hydrolysis of Compound No. 65 yielded the title compound as a white solid.

FABMS ($M^+$ ): 493.
$^1$H-NMR ($D_2O$-NaOD) δ ppm; 0.8-0.9 (6H, m, CH$\underline{(CH_3)_2}$), 0.95-1.7 (13H, m, CH-$\underline{(CH_2)_3}$ + CH-$\underline{(CH_2)_2}$ + $\underline{CH_2}$-$\underline{CH}(CH_3)_2$), 2.15 (1H, m, $\underline{CH}$-CO), 2.4-2.6 (3H, m, $\underline{CH}$-CO + $\underline{CH_2}$-$NH_2$), 2.6-2.8 (5H, m, N-$\underline{CH_3}$ + $C_6H_4$-$\underline{CH_2}$), 4.02 (1H, m, NH-$\underline{CH}$-CO), 7.26 and 7.81 (2H each, m, aromatic-H).

Preparation Example 68

$N^a$-[4-(Hydroxyamino)-2(R)-isobutyl-3(R or S)-(8-hydroxyoctyl)-succinyl]-L-lysine N-methylamide • 1 acetate

The procedure of Preparation Example 23 was repeated using 11-(tetrahydropyranyloxy)-3(RS)-tert-butyloxycarbonyl-2(R)-isobutylundecanoic acid and Compound No. 1 to give the title compound as a white solid.

FABMS ($M^+$ ): 459.
$^1$H-NMR ($CD_3OD$)δ ppm; 0.8-0.95 (6H, m, CH$\underline{(CH_3)_2}$), 1.05 (1H, m, $\underline{CH}(CH_3)_2$), 1.1-1.9 (22H, m, CH-$\underline{(CH_2)_7}$ + CH-$\underline{(CH_2)_3}$ + $\underline{CH_2}$-CH$(CH_3)_2$), 1.92 (3H, s, $\underline{CH_3}CO_2H$), 2.13 (1H, m, $\underline{CH}$-CO), 2.56 (1H, m, $\underline{CH}$-CO), 2.72 (3H, s, N-$\underline{CH_3}$), 2.91 (2H, m, $\underline{CH_2}$-$NH_2$), 3.53 (2H,t, J=6.5Hz, $\underline{CH_2}$-OH), 4.31 (1H, m, NH-$\underline{CH}$-CO).

Preparation Example 69

$N^a$-[4-(Hydroxyamino)-2(R)-isobutyl-3(R or S)-(2-n-butyloxyethyl)succinyl]-L-lysine N-methylamide • 1 acetate

The procedure of Preparation Example 23 was repeated using 5-(n-butyloxy)-3(RS)-tert-butyloxycarbonyl-2(R)-isobutylpentanoic acid and Compound No. 1 to give the title compound as a white solid.

FABMS ($M^+$ 1): 432.
$^1$H-NMR ($CD_3OD$)δ ppm; 0.8-0.95 (9H, m, CH$\underline{(CH_3)_2}$ + $CH_2$-$\underline{CH_3}$), 1.06 (1H, m, $\underline{CH}(CH_3)_2$), 1.2-1.9 (14H, m, CH-

$\underline{CH_2}$ + CH-$\underline{(CH_2)_3}$ + $\underline{(CH_2)_2}$-$CH_3$ + $\underline{CH_2}$-CH$(CH_3)_2$), 1.91 (3H, s, $\underline{CH_3}CO_2H$), 2.29 (1H, m, $\underline{CH}$-CO), 2.58 (1H, m, $\underline{CH}$-CO), 2.72 (3H, s, N-$\underline{CH_3}$), 2.91 (2H, m, $\underline{CH_2}$-$NH_2$), 3.2-3.4 (m, O-$\underline{CH_2}$× 2), 4.31 (1H, m, NH-$\underline{CH}$-CO).

Preparation Example 70

$N^a$-[4-(Hydroxyamino)-2(R)-isobutyl-3(R or S)-(2-isobutyloxyethyl)succinyl]-L-lysine N-methylamide • 1 acetate

The procedure of Preparation Example 23 was repeated using 5-(isobutyloxy)-3(RS)-tert-butyloxycarbonyl-2(R)-isobutylpentanoic acid and Compound No. 1 to give the title compound as a white solid.

FABMS ($M^+$ ): 431.
$^1$H-NMR ($CD_3OD$)δ ppm; 0.8-0.95 (12H, m, CH$\underline{(CH_3)_2}$ × 2), 1.07 (1H, m, $\underline{CH}(CH_3)_2$), 1.3-1.9 (11H, m, CH-$\underline{CH_2}$ + CH-$\underline{(CH_2)_3}$ + O-$CH_2$-$\underline{CH}$ + $\underline{CH_2}$-CH$(CH_3)_2$), 1.92 (3H, s, $\underline{CH_3}CO_2H$), 2.30 (1H, m, $\underline{CH}$-CO), 2.58 (1H, m, $\underline{CH}$-CO), 2.72(3H, s, N-$\underline{CH_3}$), 2.92 (2H, m, $\underline{CH_2}$-$NH_2$), 3.13 (2H, d, J = 6.6Hz, O-$\underline{CH_2}$-CH), 3.2-3.4 (m, O-$\underline{CH_2}$), 4.32 (1H, m, NM-$\underline{CH}$-CO).

Preparation Example 71

$N^a$-[4-(Hydroxyamino)-2(R)-isobutyl-3(R or S)-[3-(m-methoxycarbonylphenyl)propyl]succinyl]-L-lysine N-methylamide • 1 acetate

The procedure of Preparation Example 23 was repeated using 6-(m-methoxycarbonylphenyl)-3(RS)-tert-butyloxycarbonyl-2(R)-isobutylhexanoic acid and Compound No. 1 to give the title compound as a white solid.

FABMS ($M^+$ + 1): 508.
$^1$H-NMR ($CD_3OD$)δ ppm; 0.8-1.0 (6H, m, CH$\underline{(CH_3)_2}$), 1.06 (1H, m, $\underline{CH}(CH_3)_2$), 1.2-1.85 (12H, m, CH-$\underline{(CH_2)_3}$ + CH-$\underline{(CH_2)_2}$ + $\underline{CH_2}$-CH$(CH_3)_2$), 1.91 (3H, s, $\underline{CH_3}CO_2H$), 2.20 (1H, m, $\underline{CH}$-CO), 2.5-2.8 (6H, m, $\underline{CH}$-CO + N-$CH_3$ + $\underline{CH_2}$-$NH_2$), 2.91 (2H, m, $C_6H_4$-$\underline{CH_2}$), 3.90 (3H, s, -$CO_2\underline{CH_3}$), 4.27 (1H, m, NH-$\underline{CH}$-CO), 7.39 and 7.82 (2H each, m, aromatic-H).

Preparation Example 72

$N^a$-[4-(Hydroxyamino)-2(R)-isobutyl-3(R or S)-[3-(p-hydroxyphenyl)propyl]succinyl]-L-lysine N-methylamide • 1 acetate

The procedure of Preparation Example 23 was repeated using 6-(p-benzyloxyphenyl)-3(RS)-tert-butyloxycarbonyl-2(R)-isobutylhexanoic acid and Compound No. 1 to give the title compound as a white solid.

FABMS ($M^+$ ): 465.
$^1$H-NMR ($CD_3OD$)δ ppm; 0.8-0.95 (6H, m, CH$\underline{(CH_3)_2}$), 1.04 (1H, m, $\underline{CH}(CH_3)_2$), 1.2-1.85 (12H, m, CH-$\underline{(CH_2)_3}$ + CH-$\underline{(CH_2)_2}$ + $\underline{CH_2}$-CH$(CH_3)_2$), 1.92 (3H, s, $\underline{CH_3}CO_2H$), 2.17 (1H, m, $\underline{CH}$-CO), 2.35-2.6 (3H, m, $\underline{CH}$-CO + $C_6H_4$-$\underline{CH_2}$), 2.71 (3H, m, N-$\underline{CH_3}$), 2.88 (2H, m, $\underline{CH_2}$-$NH_2$), 4.25 (1H, m, NH-$\underline{CH}$-CO), 6.67 and 6.94 (2H each, m, aromatic-H).

Preparation Example 73

$N^a$-[4-(Hydroxyamino)-2(R)-isobutyl-3(R or S)-[3-(morpholin-4-yl)propyl]succinyl]-L-lysine N-methylamide • 1 acetate

The procedure of Preparation Example 23 was repeated using 6-morpholino-3(RS)-tert-butyloxycarbonyl-2(R)-isobutylhexanoic acid and Compound No. 1 to give the title compound as a white solid.

FABMS ($M^+$ ): 458.
$^1$H-NMR ($CD_3OD$)δ ppm; 0.7-0.9 (6H, m, CH$\underline{(CH_3)_2}$), 1.02 (1H, m, $\underline{CH}(CH_3)_2$), 1.2-1.8 (12H, m, CH-$\underline{(CH_2)_3}$ + CH-$\underline{(CH_2)_2}$ + $\underline{CH_2}$-CH$(CH_3)_2$), 1.89 (3H, s, $\underline{CH_3}CO_2H$), 2.20 (1H, m, $\underline{CH}$-CO), 2.5-3.0 (12H, m, $\underline{CH}$-CO + N-$\underline{CH_2}$ × 3 + N-$\underline{CH_3}$ + $\underline{CH_2}$-$NH_2$), 3.74 (4H, m, O-$\underline{CH_2}$× 2), 4.28 (1H, m, NH-$\underline{CH}$-CO).

Preparation Example 74

N[a]-[4-(Hydroxyamino)-2(R)-isopropyl-3(R or S)-(3-phenylpropyl)succinyl]-L-lysine N-methylamide • 1 acetate

The procedure of Preparation Example 23 was repeated using 6-phenyl-3(RS)-tert-butyloxycarbonyl-2(R)-isopropylhexanoic acid and Compound No. 1 to give the title compound as a white solid.

FABMS ($M^+$ + 1): 436.
$^1$H-NMR ($CD_3OD$)δ ppm; 0.85-1.0 (6H, m, $CH\underline{(CH_3)_2}$), 1.3-1.9 (11H, m, $\underline{CH}(CH_3)_2$ + CH-$\underline{(CH_2)_3}$ + CH-$\underline{(CH_2)_2}$), 1.90 (3H, s, $\underline{CH_3}CO_2H$), 2. 38 (1H, m, $\underline{CH}$-CO), 2.4-2.65(3H, m, $\underline{CH}$-CO + $\underline{CH_2}$-$C_6H_5$), 2.72 (3H, m, N-$\underline{CH_3}$), 2.89 (2H, m, $\underline{CH_2}$-$NH_2$), 4.27(1H, m, NH-$\underline{CH}$-CO), 7.0-7.3 (5H, m, aromatic-H).

Preparation Example 75

N[a]-[4-(Hydroxyamino)-2(R)-isobutyl-3(R or S)-[3-(m-carboxyphenyl)propyl]succinyl]-L-lysine N-methylamide • 1 acetate

Alkaline hydrolysis of Compound No. 71 yielded the title compound as a white solid.

FABMS ($M^+$ + 1): 494.
$^1$H-NMR ($CD_3OD$)δ ppm; 0.8-0.9 (6H, m, $CH\underline{(CH_3)_2}$), 1.01 (1H, m, $\underline{CH}(CH_3)_2$), 1.1-1.8 (12H, m, CH-$\underline{(CH_2)_3}$, + CH-$\underline{(CH_2)_2}$ + $\underline{CH_2}$-$CH(CH_3)_2$), 1.96 (3H, s, $\underline{CH_3}CO_2H$), 2.13 (1H, m, $\underline{CH}$-CO), 2.49 (1H, m, $\underline{CH}$-CO), 2.55-2.85 (5H, m, N-$\underline{CH_3}$ + $C_6H_4$-$\underline{CH_2}$), 2.84 (2H, m, $\underline{CH_2}$-$NH_2$), 4.11 (1H, m, NH-$\underline{CH}$-CO), 7.26 and 7.73 (2H each, m, aromatic-H).

Preparation Example 76

N[a]-[4-(Hydroxyamino)-2(R)-isobutyl-3(R or S)-[3-(piperidin-1-yl)propyl]succinyl]-L-lysine N-methylamide • 2 acetate

The procedure of Preparation Example 23 was repeated using 6-(piperidin-1-yl)-3(RS)-tert-butyloxycarbonyl-2(R)-isobutylhexanoic acid and Compound No. 1 to give the title compound as a white solid.

FABMS ($M^+$ +1); 457.
$^1$H-NMR ($CD_3OD$)δ ppm; 0.8-0.95 (6H, m, $CH\underline{(CH_3)_2}$), 1.07 (1H, m, $\underline{CH}(CH_3)_2$), 1.2-1.85 (18H, m, $\underline{CH_2}$-$CH(CH,)_2$ + CH-$\underline{(CH_2)_2}$ + CH-$\underline{(CH_2)_3}$-$CH_3$-$NH_2$ + N-$CH_2$-$\underline{(CH_2)_3}$), 1.91 (6H, s, $\underline{CH_3}CO_2H \times 2$), 2.18 (1H, m, $\underline{CH}$-CO), 2.57 (1H, m, $\underline{CH}$-CO), 2.72 (3H, s, N-$\underline{CH_3}$), 2.7-3.1 (8H, m, N-$\underline{CH_2} \times 3$ + $\underline{CH_2}$-$NH_2$), 4.28 (1H, m, NH-$\underline{CH}$-CO).

Preparation Example 77

N[a]-[4-(Hydroxyamino)-2(R)-isobutyl-3(R or S)-methylsuccinyl]-N[e]-benzimidoyl-L-lysine N-methylamide • 1 acetate

The procedure of Preparation Example 22 was repeated using Compound No. 18 and Compound No. 22-a, except that ethyl acetimidate hydrochloride was replaced with methyl benzimidate hydrochloride, to give the title compound as a white solid.

FABMS ($M^+$ ): 448.
$^1$H-NMR ($CD_3OD$)δ ppm; 0.7-0.9 (6H, m, $\underline{CH(CH_3)_2}$), 0.94 (3H, d, J=7.0Hz, CH-$\underline{CH_3}$), 1.01 (1H, m, $\underline{CH}(CH_3)_2$), 1.2-1.7 (8H, m, $\underline{CH_2}$-$CH(CH_3)_2$+ CH-$\underline{(CH_2)_3}$), 1.80 (3H, s, $\underline{CH_3}CO_2H$), 2.15 (1H, m, $\underline{CH}$-CO), 2.48 (1H, m, $\underline{CH}$-CO), 2.64 (3H, s, N-$\underline{CH_3}$), 3.35 (2H, m, $\underline{CH_2}$-$NH_2$), 4.26 (1H, m, NH-$\underline{CH}$-CO), 7.4-7.7 (5H, m, aromatic-H).

Preparation Example 78

N[4]-[3-Amino-1(S)-methylcarbamoylpropyl)-N[1]-hydroxy-2(R or S)-[3-(p-aminomethylphenyl)propyl]-3(R)-isobutylsuccinamide • 2 acetate

The procedure of Preparation Example 23 was repeated using 6-(p-benzyloxycarbonylaminomethylphenyl)-3(RS)-tert-butyloxycarbonyl-2(R)-isobutylhexanoic acid and 2(S)-tert-butyloxycarbonylamino-4-benzyloxycarbonylaminobutanoic acid N-methylamide to give the title compound as a white solid.

FABMS ($M^+$ ): 450.
$^1$H-NMR ($CD_3OD$)δ ppm; 0.75-0.9 (6H, m, CH($CH_3$)$_2$), 1.07 (1H, m, CH($CH_3$)$_2$), 1.2-1.7 (6H, m, $CH_2$-CH($CH_3$)$_2$ + CH-($CH_2$)$_2$), 1.85 (6H, s, $CH_3CO_2H$× 2), 1.8-2.1 (2H, m, CH-$CH_2$), 2.18 (1H, m, CH-CO), 2.52 (3H, m, CH-CO + $C_6H_4$-$CH_2$), 2.69 (3H, s, N-$CH_3$), 2.88 (2H, m, $CH_2$-$NH_2$), 3.99 (2H, s, $C_6H_4$-$CH_2$-$NH_2$), 4.36 (1H, m, NH-CH-CO), 7.17 and 7.29 (2H each, m, aromatic-H).

Preparation Example 79

$N^a$-[4-(Hydroxyamino)-2(R)-isobutyl-3(R or S)-[3-(p-aminomethylphenyl)propyl]succinyl]-L-tert-leucine N-methylamide • 1 acetate

The procedure of Preparation Example 23 was repeated using 6-(p-benzyloxycarbonylaminomethylphenyl)-3(RS)-tert-butyloxycarbonyl-2(R)-isobutylhexanoic acid and $N^a$-tert-butyloxycarbonyl-L-tert-leucine N-methylamide to give the title compound as a white solid.

FABMS ($M^+$ +1): 464.
$^1$H-NMR ($CD_3OD$)δ ppm; 0.7-0.85 (6H, m, CH($CH_3$)$_2$), 0.94 (9H, s, C($CH_3$)$_3$), 1.02(1H, m, CH($CH_3$)$_2$), 1.2-1.7 (6H, m, $CH_2$-CH($CH_3$)$_2$ + CH-($CH_2$)$_2$ , 1.88 (3H, m, $CH_3CO_2H$), 2.14 (1H, m, CH-CO), 2.4-2.6 (3H, m, CH-CO + $C_6H_4$-$CH_2$), 2.65 (3H, s, N-$CH_3$), 4.00 (2H, s, $CH_2$-$NH_2$), 4.18 (1H, m, NH-CH-CO), 7.16 and 7.28 (2H each, m, aromatic-H).

Preparation Example 80

$N^a$-[4-(Hydroxyamino)-2(R)-isobutyl-3(R or S)-[3-(p-aminomethylphenyl)propyl]succinyl]-L-ornithine N-methylamide • 1 acetate

The procedure of Preparation Example 23 was repeated using 6-(p-benzyloxycarbonylaminomethylphenyl)-3(RS)-tert-butyloxycarbonyl-2(R)-isobutylhexanoic acid and Compound No. 7 to give the title compound as a white solid.

FABMS ($M^+$ +1): 465.
$^1$H-NMR ($CD_3OD$)δ ppm; 0.8-0.9 (6H, m, CH($CH_3$)$_2$), 1.05 (1H, m, CH($CH_3$)$_2$), 1.2-1.85 (12H, m, $CH_2$-CH($CH_3$)$_2$ + CH-($CH_2$)$_3$ + CH-($CH_2$)$_2$ , 1.89 (3H, s, $CH_3CO_2H$), 2.20 (1H, m, CH-CO), 2.5-2.7 (3H, m, CH-CO + $C_6H_4$-$CH_2$), 2.72 (3H, s, N-$CH_3$), 2.89 (2H, s, $CH_2$-$NH_2$), 4.01 (2H, s, $C_6H_4$-$CH_2$-$NH_2$), 4.29 (1H, m, NH-CH-CO), 7.22 and 7.31 (2H each, m, aromatic-H).

Preparation Example 81

$N^a$-[4-(Hydroxyamino)-2(R)-isobutyl-3(R or S)-[3-(p-guanidomethylphenyl)propyl]succinyl]-L-ornithine N-methylamide • 2 acetate

The procedure of Preparation Example 23 was repeated using 6-[p-($N^1$,$N^2$-dibenzyloxycarbonylguanidomethyl)phenyl]-3(RS)-tert-butyloxycarbonyl-2(R)-isobutylhexanoic acid and Compound No. 7 to give the title compound as a white solid.

FABMS ($M^+$ ): 506.
$^1$H-NMR ($CD_3OD$)δ ppm; 0.7-0.85 (6H, m, CH($CH_3$)$_2$), 0.99 (1H, m, CH($CH_3$)$_2$, 1.15-1.8 (10H, m, $CH_2$-CH($CH_3$)$_2$ + CH-($CH_2$)$_2$ × 2), 1.81 (6H, s, $CH_3CO_2H$× 2), 2.12 (1H, m, CH-CO), 2.4-2.6 (3H, m, CH-CO + $C_6H_4$-$CH_2$), 2.67 (3H, s, N-$CH_3$), 2.82 (2H, s, $CH_2$-$NH_2$), 4.22 (1H, m, NH-CH-CO), 4.26 (2H, s, $C_6H_4$-$CH_2$-NH), 7.0- 7.2 (4H, m, aromatic-H).

Preparation Example 82

$N^a$-[4-(Hydroxyamino)-2(R)-isobutyl-3(R or S)-(3-(3,4,4-trimethyl-2,5-dioxo-imidazolidin-1-yl)propyl]succinyl]-L-lysine N-methylamide

The procedure of Preparation Example 23 was repeated using 6-(3,4,4-trimethyl-2,5-dioxo-imidazolidin-1-yl)-3(RS)-tert-butyloxycarbonyl-2(R)-isobutylhexanoic acid and Compound No. 1 to give the title compound as a white

solid.

FABMS ($M^+$ +1): 514.
$^1$H-NMR ($CD_3OD$)δ ppm; 0.8-0.9 (6H, m, CH$\underline{(CH_3)_2}$), 1.03 (1H, m, $\underline{CH}(CH_3)_2$), 1.2-1.9 (18H, m, $\underline{CH_2}$-$CH(CH_3)_2$ + CH-$\underline{(CH_2)_2}$ + CH-$\underline{(CH_2)_3}$) + C$\underline{(CH_3)_2}$, 2.11 (1H, m, $\underline{CH}$-CO), 2.54 (1H, m, $\underline{CH}$-CO), 2.72 (3H, s, NH-$\underline{CH_3}$), 2.87 (3H, s, N-$\underline{CH_3}$), 3.00 (2H,t, J=7.5Hz, $\underline{CH_2}$-$NH_2$), 3.42 (2H,t, J=6.1Hz, $\underline{CH_2}$-N), 4.30 (1H, m, NH-$\underline{CH}$-CO).

Preparation Example 83

$N^a$-[4-(Hydroxyamino)-3(S)-isobutyl-2(R or S)-(8-hydroxyoctyl)-succinyl]-L-lysine N-methylamide • 1 acetate

The procedure of Preparation Example 23 was repeated using 10-tetrahydropyranyloxy-2(RS)-[1-(S)-tert-butyloxycarbonyl-3-methylbutyl]decanoic acid and Compound No. 1 to give the title compound as a white solid.

FABMS ($M^+$ +1): 460.
$^1$H-NMR ($CD_3OD$)δ ppm; 0.8-0.9 (6H, m, CH$\underline{(CH_3)_2}$), 1.0 (1H, m, $\underline{CH}(CH_3)_2$), 1.1-1.85 (22H, m, $\underline{CH_2}$-$CH(CH_3)_2$ + CH-$\underline{(CH_2)_3}$ + CH-$\underline{(CH_2)_7}$), 1.91 (3H, s, $\underline{CH_3}CO_2H$), 2. 29 (1H, m, $\underline{CH}$-CO), 2.41 (1H, m, $\underline{CH}$-CO), 2.73 (3H, s, N-$\underline{CH_3}$), 2.90 (2H,t, J=7.6Hz, $\underline{CH_2}$-$NH_2$), 3.52 (2H, m, $\underline{CH_2}$-OH), 4.31 (1H, m, NH-$\underline{CH}$-CO).

Preparation Example 84

$N^a$-[4-(Hydroxyamino)-2(R),3(R or S)-diisobutylsuccinyl]-4'-aminomethyl-L-phenylalanine N-(2-carboxyethyl)amide • 1 acetate

The procedure of Preparation Example 23 was repeated using Compound No. 17 and $N^a$-tert-butyloxycarbonyl-L-4'-cyanophenylalanine N-(2-benzyloxycarbonylethyl)amide to give the title compound as a white solid. .

FABMS ($M^+$ ): 493.
$^1$H-NMR ($CD_3OD$)δ ppm; 0.75-1.1 (14H, m, $CH_2$-$\underline{CH(CH_3)_2}$ × 2), 1.2-1.6 (4H, m, $\underline{CH_2}$-$CH(CH_3)_2$× 2), 1.97 (3H, s, $\underline{CH_3}CO_2H$), 1.9-2.2 (3H, m, $\underline{CH}$-CO + $\underline{CH_2}$-$CO_2H$), 2.52 (1H, m, $\underline{CH}$-CO), 2.99 (2H, m, $\underline{CH_2}$-$C_6H_4$), 3.25-3.4 (m, NH-$\underline{CH_2}$), 4.02 (2H, m, $C_6H_4$-$\underline{CH_2}$-$NH_2$), 4.53 (1H, m, NH-$\underline{CH}$-CO), 7.32 (4H, m, aromatic-H).

Preparation Example 85

$N^a$-[4-(Hydroxyamino)-2(R),3(R or S)-diisobutylsuccinyl]-4'-acetimidoyliminomethyl-L-phenylalanine N-(2-carboxyethyl)amide • 1 acetate

The procedure of Preparation Example 23 was repeated using Compound No. 17 and $N^a$-tert-butyloxycarbonyl-L-4'-cyanophenylalanine N-(2-benzyloxycarbonylethyl)amide to give the title compound as a white solid.

FABMS ($M^+$ ): 534.
$^1$H-NMR ($CD_3OD$)δ ppm; 0.7-0.9 (12H, m, CH$\underline{(CH_3)_2}$ × 2), 1.0-1.1 (2H, m, $\underline{CH}(CH_3)_2$× 2), 1.3-1.7 (4H, m, $\underline{CH_2}$-$CH(CH_3)_2$× 2), 1.94 (3H, s, $\underline{CH_3}CO_2H$), 2.0-2.3 (6H, m, $\underline{CH}$-CO + $\underline{CH_2}$-$CO_2H$ + C-$\underline{CH_3}$), 2.52 (1H, m, $\underline{CH}$-CO), 2.9-3.1 (2H, m, $\underline{CH_2}$-$C_6H_4$), 3.2-3.4 (m, NH-$\underline{CH_2}$), 4.38 (2H, s, $C_6H_4$-$\underline{CH_2}$-NH), 4.55 (1H, m, NH-$\underline{CH}$-CO), 7.28 (4H, m, aromatic-H).

Preparation Example 86

4 1 $N^4$-[2-Amino-2-methyl-1(RS)-methylcarbamoylpropyl]-$N^1$-hydroxy-2(R or S)-[3-(p-aminomethylphenyl)propyl]-3(R)-isopropylsuccinamide • 2 acetate

The procedure of Preparation Example 23 was repeated using 6-(p-benzyloxycarbonylaminomethylphenyl)-3(R5)-tert-butyloxycarbonyl-2(R)-isopropylpentanoic acid and 2(RS)-tert-butyloxycarbonylamino-3-benzyloxycarbonylamino-3-methylbutanoic acid N-methylamide to give the title compound as a white solid.

FABMS ($M^+$ ): 450.
$^1$H-NMR ($CD_3OD$)δ ppm; 0.85-1.0 (6H, m, CH$\underline{(CH_3)_2}$),1.2-1.8 (11H, m, $\underline{CH}(CH_3)_2$ + C-$\underline{CH_3}$ × 2 + CH-$\underline{(CH_2)_2}$), 1.91 (6H, s, $\underline{CH_3}CO_2H$× 2), 2.31 (1H, m, $\underline{CH}$-CO), 2.4-2.6 (3H, m, $\underline{CH}$-CO + $C_6H_4$-$\underline{CH_2}$), 2.65 (3H, s, N-$\underline{CH_3}$), 4.01 (2H,

s, $\underline{CH_2}$-$NH_2$), 4.72 (1H, m, NH-$\underline{CH}$-CO), 7.17 and 7.29 (2H each, m, aromatic-H).

Preparation Example 87

$N^a$-[4-(Hydroxyamino)-2(R)-isobutyl-3(R or S)-methylsuccinyl]-4'-aminomethyl-L-phenylalanine N-(morpholin-4-yl)amide • 1 acetate

The procedure of Preparation Example 23 was repeated using Compound No. 2 and Compound No. 18 to give the title compound as a white solid.

FABMS ($M^+$ ): 464.
$^1$H-NMR ($CD_3OD$)δ ppm; 0.62, 0.71 and 0.80 (3H each, m, CH$\underline{(CH_3)_2}$× 2 + CH-$\underline{CH_3}$), 0.91 (1H, m, $\underline{CH}(CH_3)_2$), 1.2-1.5 (2H, m, $\underline{CH_2}$-$CH(CH_3)_2$), 1.81 (3H, s, $\underline{CH_3}CO_2H$), 2.01 (1H, m, $\underline{CH}$-CO), 2.39 (1H, m, $\underline{CH}$-CO), 2.5-2.6 (4H, m, N-$\underline{CH_2}$ × 2), 2.8-3.0 (2H, m, $\underline{CH_2}$-$C_6H_4$), 3.61 (4H, m, O-$\underline{CH_2}$ × 2), 3.93 (2H, s, $C_6H_4$-$\underline{CH_2}$-$NH_2$), 4.48 (1H, m, NH-$\underline{CH}$-CO), 7.29 (4H, m, aromatic-H).

Preparation Example 88

$N^a$-[4-(Hydroxyamino)-2(R)-isobutyl-3(S)-hydroxysuccinyl]-4'-aminomethyl-L-phenylalanine N-methylamide • 1 acetate

The procedures of Preparation Examples 23-b and c except omitting removal of a tert-butyl group with trifluoroacetic acid were repeated using Compound No. 43-b to provide the title compound as a white solid.

FABMS ($M^+$ + 1): 395.
$^1$H-NMR ($OD_3OD$)δ ppm; 0.87 (6H, m, CH$\underline{(CH_3)_2}$), 1.1-1.6 (3H, m, $\underline{CH_2}$-$\underline{CH}(CH_3)_2$), 1.98 (3H, s, $\underline{CH_3}CO_2H$), 2.6-2.8 (4H, s + m, N-$\underline{CH_3}$ + $\underline{CH}$-CO), 2.9-3.1 (2H, m, $\underline{CH_2}$-$C_6H_4$), 3.93 (2H, m, $\underline{CH_2}$-$NH_2$), 4.4-4.6 (2H, m, NH-$\underline{CH}$-CO + HO-$\underline{CH}$-CO), 7.30(4H, m, aromatic-H).

Preparation Example 89

$N^a$-[4-(Hydroxyamino)-2(R),3(R or S)-diisobutylsuccinyl]-4'-aminomethyl-L-phenylalanine N-(2-N',N'-dimethylaminoethyl)amide • 2 acetate

The procedure of Preparation Example 23 was repeated using Compound No. 17 and $N^a$-tert-butyloxycarbonyl-L-4'-cyanophenylalanine N-(2-N',N'-dimethylaminoethyl)amide to provide the title compound as a white solid.

FABMS ($M^+$ +1): 493.
$^1$H-NMR ($OD_3OD$)δ ppm; 0.6-0.9 (12H, m, CH$\underline{(CH_3)_2}$× 2), 1.02 (2H, m, $\underline{CH}(CH_3)_2$ × 2), 1.3-1.6 (4H, m, $\underline{CH_2}$-$CH(CH_3)_2$× 2), 1.91 (6H, s, $\underline{CH_3}CO_2H$) × 2), 2.16 (1H, m, $\underline{CH}$-CO), 2.4-2.55 (7H, s + m, N-$\underline{CH_3}$ × 2 + $\underline{CH}$-CO), 2.71 (2H, m, $\underline{CH_2}$-N), 2.9-3.1 (2H, m, $\underline{CH_2}$-$C_6H_4$), 3.3-3.5 (2H, m, NH-$\underline{CH_2}$), 4.03 (2H, s, $C_6H_4$-$\underline{CH_2}$-$NH_2$), 4.60 (1H, m, NH-$\underline{CH}$-CO), 7.38 (4H, m, aromatic-H).

Preparation Example 90

$N^a$-[4-(Hydroxyamino)-2(R),3(R or S)-diisobutylsuccinyl]-4' acetimidoyliminomethyl-L-phenylalanine N-(2-N',N'-dimethylaminoethyl)amide • 2 acetate

The procedure of Preparation Example 22 was repeated using Compound No. 17 and $N^a$-tert-butyloxycarbonyl-L-4'-cyanophenylalanine N-(2-N',N'-dimethylaminoethyl)amide to provide the title compound as a white solid.

$^1$H-NMR ($CD_3OD$)δ ppm; 0.6-0.9 (13H, m, CH$\underline{(CH_3)_2}$ + $\underline{CH(CH_3)_2}$), 0.95-1.1 (1H, m, $\underline{CH}(CH_3)_2$), 1.4-1.6 (4H, m, $\underline{CH_2}$-$CH(CH_3)_2$× 2), 1.91 (6H, s, $\underline{CH_3}CO_2H$× 2), 2.16 (1H, m, $\underline{CH}$-CO), 2.24 (3H, s, C-$\underline{CH_3}$), 2.37 (6H, s, N-$CH_3$× 2), 2.4-2.5 (3H, m, $\underline{CH}$-CO + $\underline{CH_2}$-N), 2.9-3.1 (2H, m, $\underline{CH_2}$-$C_6H_4$), 3.2-3.4 (m, NH-$\underline{CH_2}$), 4.40 (2H, s, $C_6H_4$-$\underline{CH_2}$-NH), 4.60 (1H, m, NH-$\underline{CH}$-CO), 7.31 (4H, m, aromatic-H).

Preparation Example 91

$N^a$-[4-(Hydroxyamino)-2(R),3(RS)-diisobutylsuccinyl]-4'-acetimidoyliminomethyl-L-phenylalanine N-(2-hydroxyethyl)amide • 2 acetate

The procedure of Preparation Example 22 was repeated using Compound No. 14 and Compound No. 17 to provide the title compound as a white solid.

$^1$H-NMR ($CD_3OD$)δ ppm; 0.6-0.9 (13H, m, $CH(\underline{CH_3})_2$ + $\underline{CH}(CH_3)_2$), 1.00 (1H, m, C$\underline{H}$($CH_3$)$_2$ ), 1.3-1.6 (4H, m, $\underline{CH_2}$-$CH(CH_3)_2$ × 2), 1.89 (3H, s, $\underline{CH_3}CO_2H$), 2.15(1H, m, $\underline{CH}$-CO), 2.24 (3H, s, C-$\underline{CH_3}$), 2.46 (1H, m, $\underline{CH}$-CO), 2.9-3.1 (2H, m, $\underline{CH_2}$-$C_6H_4$), 3.2-3.6 (m, NH-$\underline{CH_2}$-$\underline{CH_2}$-OH), 4.40 (2H, s, $C_6H_4$-$\underline{CH_2}$-$NH_2$), 4.65 (1H, m, NH-$\underline{CH}$-CO), 7.31 (4H, m, aromatic-H).

Preparation Example 92

$N^a$-[4-(Hydroxyamino)-2(R)-isobutyl-3(R or S)-[3-(p-aminophenyl)propyl]succinyl]-$N^e$-acetimidoyl-L-lysine N-(2-N',N'-dimethylaminoethyl)amide • 3 acetate

The procedure of Preparation Example 22 was repeated using 6-(p-benzyloxycarbonylaminophenyl)-3(RS)-tert-butyloxycarbonyl-2(R)-isobutylhexanoic acid and Compound No. 3 to provide the title compound as a white solid.

FABMS ($M^+$ + 1): 563.
$^1$H-NMR ($CD_3OD$)δ ppm; 0.7-0.95 (6H, m, $CH(\underline{CH_3})_2$), 1.09 (1H, m, $\underline{CH}(CH_3)_2$), 1.1-1.8 (12H, m, $\underline{CH_2}$-$CH(CH_3)_2$ + CH-$(\underline{CH_2})_2$ + CH-$(\underline{CH_2})_3$), 1.96 (9H, s, $\underline{CH_3}CO_2H$× 3), 2.21 (3H, s, C-$\underline{CH_3}$), 2.3-2.6 (4H, m, $\underline{CH}$-CO × 2 + $\underline{CH_2}$-$C_6H_4$), 2.76 (6H, m, N-$\underline{CH_3}$ × 2), 2.9-3.5 (m, $\underline{CH_2}$-$NH_2$ + NH-$\underline{CH_2}$-$\underline{CH_2}$-N), 4.13 (1H, m, NH-$\underline{CH}$-CO), 6.64 , 6.89, 7.07 and 7.41 (1H each, m, aromatic-H).

Preparation Example 93

$N^a$-[4-(Hydroxyamino)-2(R),3(RS)-diisobutylsuccinyl]-4'-acetimidoyliminomethyl-L-phenylalanine N-(2-hydroxy-1,1-dimethylethyl)amide • 1 acetate

The procedure of Preparation Example 22 was repeated using Compound No. 17 and $N^a$-tert-butyloxycarbonyl-L-4'-cyanophenylalanine N-(1,1-dimethyl-2-hydroxyethyl)amide to provide the title compound as a white solid.

FABMS ($M^+$ + 1): 535.
$^1$H-NMR ($CD_3OD$)δ ppm; 0.7-0.95 (12H, m, $CH(\underline{CH_3})_2$ × 2), 1.0-1.1 (2H, m, $\underline{CH}(CH_3)_2$× 2 ), 1.1-1.8 (10H, m, $C(\underline{CH_3})_2$ + $\underline{CH_2}$-$CH(CH_3)_2$× 2), 1.89 (3H, s, $\underline{CH_3}CO_2H$), 2.15 (1H, m, $\underline{CH}$-CO), 2.24 (3H, s, C-$\underline{CH_3}$), 2.46 (1H, m, $\underline{CH}$-CO), 3.0-3.4 (2H, m, $\underline{CH_2}$-$C_6H_4$), 3.4-3.6 (2H, m, $\underline{CH_2}$-OH), 4.41 (2H, s, $C_6H_4$-$\underline{CH_2}$-NH), 4.62 (1H, m, NH-$\underline{CH}$-CO), 7.31 (4H, m, aromatic-H).

Preparation Example 94

$N^4$-[3-Amino-2,2-dimethyl-1(RS)-methylcarbamoylpropyl]-$N^1$-hydroxy-2(R or S)-[3-(p-aminomethylphenyl)propyl]-3(R)-isopropylsuccinamide • 2 acetate

The procedure of Preparation Example 23 was repeated using 6-(p-benzyloxycarbonylaminomethylphenyl)-3(RS)-tert-butyloxycarbonyl-2(R)-isopropylhexanoic acid and 2(RS)-tert-butyloxycarbonylamino-4-benzyloxycarbonylamino-3,3-dimethylbutanoic acid N-methylamide to provide the title compound as a white solid.

FABMS ($M^+$ ): 464.
$^1$H-NMR ($CD_3OD$)δ ppm; 0.8-1.0 (6H, m, $CH(\underline{CH_3})_2$), 1.2-1.8 (11H, m, $\underline{CH}(CH_3)_2$ +C-$\underline{CH_3}$× 2 + CH-$(\underline{CH_2})_2$), 1.89 (6H, s, $\underline{CH_3}CO_2H$× 2), 2.27 (1H, m, $\underline{CH}$-CO), 2.4-2.6 (3H, m, $\underline{CH}$-CO + $C_6H_4$-$\underline{CH_2}$), 2.6-2.8 (5H, s, $\underline{CH_2}$ -$NH_2$ + N-$\underline{CH_3}$), 4.00(2H, s, $C_6H_4$-$\underline{CH_2}$-$NH_2$), 4.70 (1H, m, NH-$\underline{CH}$-CO), 7.17 and 7.29 (2H each, m, aromatic-H).

Preparation Example 95

$N^4$-[2-Amino-1(S)-methylcarbamoylpropyl)-$N^1$-hydroxy-2(R or S)-[3-(p-aminomethylphenyl)propyl]-3(R)-isopropyl succinamide • 2 acetate

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$ ): 436.
$^1$H-NMR ($CD_3OD$)δ ppm; 0.8-1.0 (6H, m, CH$\underline{(CH_3)_2}$), 1.1-1.2 (4H, m, $\underline{CH}(CH_3)_2$ +CH-$\underline{CH_3}$), 1.2-1.8 (4H, m, CH-$\underline{(CH_2)_2}$, 1.88 (6H, s, $\underline{CH_3}CO_2H$ × 2), 2.21 (1H, m, $\underline{CH}$-CO), 2.4-2.6 (3H, m, $\underline{CH}$-CO + $C_6H_4$-$\underline{CH_2}$), 2.66 (3H, s, N-$\underline{CH_3}$), 3.2-3.4 (m, $\underline{CH}$-$NH_2$), 4.00 (2H, s, $\underline{CH_2}$-$NH_2$), 4.65 (1H, m, NH-$\underline{CH}$-CO), 7.21 (4H, m, aromatic-H).

Preparation Example 96

$N^a$-[4-(Hydroxyamino)-2(R)-isopropyl-3(R or S)-[3-(p-aminomethylphenyl)propyl]succinyl]-O-(2,3,4,6-tetra-O-acetyl-β -D-glucopyranosyl)-L-serine N-methylamide • 1 acetate

The procedure of Preparation Example 23 was repeated using 6-(p-benzyloxycarbonylaminomethylphenyl)-3(RS)-tert-butyloxycarbonyl-2(R)-isopropylhexanoic acid and $N^a$-tert-butyloxycarbonyl-O-(2,3,4,6-tetra-O-acetyl-β -D-glucopyranosyl)-L-serine N-methylamide to provide the title compound as a white solid.

FABMS ($M^+$ ): 753.
$^1$H-NMR ($CD_3OD$)δ ppm; 0.8-1.0 (6H, m, CH$\underline{(CH_3)_2}$), 1.1-1.2 (17H, m, $\underline{CH}(CH_3)_2$ +CH-$\underline{(CH_2)_2}$ + $CH_3CO$- × 4), 1.90 (3H, s, $\underline{CH_3}CO_2H$), 2.30 (1H, m, $\underline{CH}$-CO), 2.4-2.75 (3H, m, $\underline{CH}$-CO + $C_6H_4$-$\underline{CH_2}$), 2.78 (3H, s, N-$\underline{CH_3}$), 3.78 (2H, m, $\underline{CH_2}$-O), 3.95-4.6 (7H, m, NH-$\underline{CH}$-CO + $\underline{CH_2}$-$NH_2$ + 2-H, 5-H and 6-H (glucopyranosyl)], 4.9-5.35 [3H, m, 1-H, 3-H and 4-H (glucopyranosyl)], 7.20 (4H, m, aromatic-H).

Preparation Example 97

$N^a$-[4-(Hydroxyamino)-2(R)-isopropyl-3(R or S)-[3-(p-aminomethylphenyl)propyl]succinyl]-O-(β -D-glucopyranosyl)-L-serine N-methylamide • 1 acetate

Hydrolysis of Compound No. 96 yielded the title compound as a white solid.

FABMS ($M^+$ + 1): 586.
$^1$H-NMR ($D_2O$)δ ppm; 0.7-0.9 (6H, m, CH$\underline{(CH_3)_2}$), 1.1-1.7 (5H, m, $\underline{CH}(CH_3)_2$ + CH-$\underline{(CH_2)_2}$, 1.78 (3H, s, $\underline{CH_3}CO_2H$), 2.15 (1H, m, $\underline{CH}$-CO), 2.4-2.6 (3H, m, $\underline{CH}$-CO + $C_6H_4$-$\underline{CH_2}$), 2.70 (3H, s, N-$\underline{CH_3}$), 3.43 [1H, m, 5-H (glucopyranosyl)), 3.5-4.0 [7H, m, $\underline{CH_2}$-O + 2-H, 3-H, 4-H and 6-H (glucopyranosyl)], 4.00 (2H, m, $\underline{CH_2}$-$NH_2$), 4.32 (1H, m, NH-$\underline{CH}$-CO), 5.25 [1H, m, 1-H (glucopyranosyl)], 7.18 (4H, m, aromatic-H).

Preparation Example 98

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-aminomethylphenyl)propyl]-3(R)-isopropylsuccinamide • 1 acetate

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-5-(3,4,4-trimethyl-2,5-dioxo-imidazolidin-1-yl)-pentanoic acid N-methylamide to provide the title compound as a white solid.

FABMS ($M^+$ ): 575.
$^1$H-NMR ($CD_3OD$)δ ppm; 0.8-1.0 (6H, m, CH$\underline{(CH_3)_2}$), 1.1-1.8 (15H, s + m, $\underline{CH}(CH_3)_2$ + CH-$\underline{(CH_2)_2}$ × 2 + C-$\underline{CH_3}$, × 2), 1.89 (3H, s, $\underline{CH_3}CO_2H$), 2.20 (1H, m, $\underline{CH}$-CO), 2.4-2.6 (3H, m, $\underline{CH}$-CO + $C_6H_4$-$\underline{CH_2}$), 2.67 (3H, s, N-$\underline{CH_3}$), 2.89 (3H, s, N-$\underline{CH_3}$), 3.56 (2H,t, J = 6.3Hz, N-$\underline{CH_2}$), 4.01 (2H, s, $\underline{CH_2}$-$NH_2$), 4.38 (1H, m, NH-$\underline{CH}$-CO), 7.20 (4H, m, aromatic-H).

Preparation Example 99

$N^4$-[2-Amino-2-methyl-1(RS)-methylcarbamoylpropyl]-$N^1$-hydroxy-2(R or S)-[3-(p-guanidomethylphenyl)propyl)-3(R)-isopropylsuccinamide • 2 acetate

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(RS)-tert-butyloxycarbonylamino-3-benzyloxycarbonylamino-3-methylbutanoic acid N-methylamide to provide the title compound as a white solid.

FABMS ($M^+$ ): 492.
$^1$H-NMR ($CD_3OD$)δ ppm; 0.8-1.0 (6H, m, CH$\underline{(CH_3)_2}$), 1.1-1.8 (11H, m, $\underline{CH}(CH_3)_2$ +C-$\underline{CH_3}$× 2 + CH-$\underline{(CH_2)_2}$), 1.89 (6H, s, $\underline{CH_3}CO_2H$× 2), 2.27 (1H, m, $\underline{CH}$-CO), 2.4-2.6 (3H, m, $\underline{CH}$-CO + $C_6H_4$-$\underline{CH_2}$), 2.72 (3H, s, N-$\underline{CH_3}$), 4.21 (1H, m, NH-$\underline{CH}$-CO), 4.26 (2H, s, $C_6H_4$-$\underline{CH_2}$-NH), 7.0-7.2 (4H, m, aromatic-H).

Preparation Example 100

$N^4$-(3-Amino-2,2-dimethyl-1(RS)-methylcarbamoylpropyl]-$N^1$-hydroxy-2(R or S)-[3-(p-guanidomethylphenyl)propyl]-3(R)-isopropylsuccinamide • 2 acetate

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(RS)-tert-butyloxycarbonylamino-4-benzyloxycarbonylamino-3,3-dimethylbutanoic acid N-methylamide to provide the title compound as a white solid.

FABMS ($M^+$ ): 506.
$^1$H-NMR ($CD_3OD$)δ ppm; 0.85-1.0 (6H, m, CH$\underline{(CH_3)_2}$), 1.1-1.8 (11H, m, $\underline{CH}(CH_3)_2$ + C-$\underline{CH_3}$ × 2 + CH-$\underline{(CH_2)_2}$), 1.90 (6H, s, $\underline{CH_3}CO_2H$ × 2), 2.31 (1H, m, $\underline{CH}$-CO), 2.4-2.6 (3H, m, $\underline{CH}$-CO + $C_6H_4$-$\underline{CH_2}$), 2.6-2.8 (5H, s, $\underline{CH_2}$-$NH_2$ + N-$\underline{CH_3}$), 4.22(1H, m, NH-$\underline{CH}$-CO), 4.28 (2H, s, $C_6H_4$-$\underline{CH_2}$-NH), 7.1-7.4 (4H, m, aromatic-H).

Preparation Example 101

$N^4$-[2-Amino-1(S)-methylcarbamoylpropyl]-$N^1$-hydroxy-2(R or S)-[3-(p-guanidomethylphenyl)propyl]-3(R)-isopropylsuccinamide • 2 acetate

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$ ): 478.
$^1$H-NMR ($CD_3OD$)δ ppm; 0.8-1.0 (6H, m, CH$\underline{(CH_3)_2}$), 1.1-1.2 (4H, m, $\underline{CH}(CH_3)_2$ + C-$\underline{CH_3}$), 1.3-1.7 (4H, m, CH-$\underline{(CH_2)_2}$), 1.90 (6H, s, $\underline{CH_3}CO_2H$ × 2), 2.34 (1H, m, $\underline{CH}$-CO), 2.4-2.7 (3H, m, $\underline{CH}$-CO + $C_6H_4$-$\underline{CH_2}$), 2.73 (3H, s, N-$\underline{CH_3}$), 3.2-3.4 (m, $\underline{CH}$-$NH_2$), 4.28 (1H, m, NH-$\underline{CH}$-CO), 4.33 (2H, s, $\underline{CH_2}$-$NH_2$), 7.19 (4H, m, aromatic-H).

Preparation Example 102

$N^a$-[4-(Hydroxyamino)-2(R)-isopropyl-3(R or S)-[3-(p-guanidomethylphenyl)propyl]succinyl]-O-(2,3,4,6-tetra-O-acetyl-β -D-glucopyranosyl)-L-serine N-methylamide • 1 acetate

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and $N^a$-tert-butyloxycarbonyl-O-(2,3,4,6-tetra-O-acetyl-β -D-glucopyranosyl)-L-serine N-methylamide to provide the title compound as a white solid.

FABMS ($M^+$ ): 795.
$^1$H-NMR ($CD_3OD$)δ ppm; 0.8-1.0 (6H, m, CH$\underline{(CH_3)_2}$), 1.1-2.1 (17H, m, $\underline{CH}(CH_3)_2$ + CH-$\underline{(CH_2)_2}$ + $CH_3$CO-× 4), 1.89 (3H, s, $\underline{CH_3}CO_2H$), 2.35 (1H, m, $\underline{CH}$-CO), 2.4-2.7 (3H, m, $\underline{CH}$-CO + $C_6H_4$-$\underline{CH_2}$), 2.80 (3H, s, N-$\underline{CH_3}$), 3.76 (2H, m, $\underline{CH_2}$-O), 4.0-4.6 [7H, m, NH-$\underline{CH}$-CO + $\underline{CH_2}$-$NH_2$ + 2-H, 5-H and 6-H (glucopyranosyl)], 4.9-5.3 [3H, m, 1-H, 3-H and 4-H (glucopyranosyl)], 7.1-7.3 (4H, m, aromatic-H).

Preparation Example 103

$N^a$-[4-(Hydroxyamino)-2(R)-isopropyl-3(R or S)-[3-(p-guanidomethylphenyl)propyl]succinyl]-O-(β -D-glucopyranosyl)-L-serine N-methylamide • 1 acetate

Hydrolysis of Compound No. 102 yielded the title compound as a white solid. .

FABMS ($M^+$ +1): 628.
$^1$H-NMR ($D_2O$)δ ppm; 0.7-0.9 (6H, m, CH$\underline{(CH_3)_2}$), 1.0-1.7 (5H, m, $\underline{CH}(CH_3)_2$ + CH-$\underline{(CH_2)_2}$), 1.80 (3H, s, $\underline{CH_3}CO_2H$), 2.17 (1H, m, $\underline{CH}$-CO), 2.4-2.6 (3H, m, $\underline{CH}$-CO + $C_6H_4$-$\underline{CH_2}$), 2.71 (3H, s, N-$\underline{CH_3}$), 3.41 [1H, m, 5-H (glucopyranosyl)], 3.5-4.0 [7H, m, $\underline{CH_2}$-O + 2-H, 3-H, 4-H and 6-H (glucopyranosyl)], 4.2-4.4 (3H, m, $C_6H_4$-$\underline{CH_2}$-$NH_2$ + NH-$\underline{CH}$-CO), 5.26 [1H, m, 1-H (glucopyranosyl)], 7.20(4H, m, aromatic-H).

Preparation Example 104

$N^4$-[4-(3,4,4-Trimethyl-2,5-dioxo-imidazolidin-1-yl)-1(S)-methylcarbamoylbutyl]-$N^1$-hydroxy-2(R or S)-[3-(p-guanidomethylphenyl)propyl]-3(R)-isopropylsuccinamide • 1 acetate

The procedure of Preparation Example 23 was repeated using 3(RS)-tert-butyloxycarbonyl 6-[p-($N^1$,$N^2$-dibenzyloxycarbonylguanidomethyl)phenyl]-2(R)-isopropylhexanoic acid and 2(S)-tert-butyloxycarbonylamino-5-(3,4,4-trimethyl-2,5-dioxo-imidazolidin-1-yl)pentanoic acid N-methylamide to provide the title compound as a white solid.

FABMS ($M^+$ ): 617.
$^1$H-NMR ($CD_3OD$)δ ppm; 0.8-1.0 (6H, m, CH$\underline{(CH_3)_2}$), 1.1-1.8 (15H, s + m, $\underline{CH}(CH_3)_2$ + CH-$\underline{(CH_2)_2}$ × 2 + C-$\underline{CH_3}$ x 2), 1.92 (3H, s, $CH_3CO_2H$), 2.23 (1H, m, $\underline{CH}$-CO), 2.45-2.6 (3H, m, $\underline{CH}$-CO + $C_6H_4$-$\underline{CH_2}$), 2.70 (3H, s, N-$\underline{CH_3}$), 2.89 (3H, s, N-$\underline{CH_3}$), 3.56 (2H, t, J=6.3Hz, N-$\underline{CH_2}$), 4.01 (2H, s, $\underline{CH_2}$-$NH_2$), 4.3-4.4 (3H, m, $C_6H_4$-$\underline{CH_2}$-NH + NH-CH-CO), 7.18 (4H, m, aromatic-H).

Preparation Example 105

$N^a$-[4-(Hydroxyamino)-2(R)-isobutyl-3(R or S)-[3-(p-carbamoylphenyl)propyl]succinyl]-L-lysine N-methylamide • 1 acetate

A mixture of Compound No. 65 (300 mg, 0.592 mmol) and 28% aqueous ammonia (30 ml) was stirred for 10 days at room temperature. The reaction mixture was concentrated, dissolved in 0.1N acetic acid, and purified by column reversed phase chromatography (25 g of Chromatorex ODS-1020T, Fuji Silysia Chemical, Japan; eluted with a gradient of 1 to 20% methanol/0.1% aqueous acetic acid), and then lyophilized to give the title compound as a white solid.

FABMS ($M^+$ + 1): 493.
$^1$H-NMR ($CD_3OD$)δ ppm; 0.8-0.9 (6H, m, CH$\underline{(CH_3)_2}$), 1.05 (1H, m, $\underline{CH}(CH_3)_2$), 1.2-1.8 (12H, m, CH-$\underline{(CH_2)_3}$ + CH-$\underline{(CH_2)_2}$ + $\underline{CH_2}$-$CH(CH_3)_2$), 1.92 (6H, s, $\underline{CH_3}CO_2H$), 2.20 (1H, m, $\underline{CH}$-CO), 2.53 (1H, m, $\underline{CH}$-CO), 2.65 (2H, m, $C_6H_4$-$\underline{CH_2}$), 2.71 (3H, s, N-$\underline{CH_3}$), 2.86 (2H,t, J=7.7Hz, $\underline{CH_2}$-$NH_2$), 4.21 (1H, m, NH-$\underline{CH}$-CO), 7.25 and 7.78 (2H each, m, aromatic-H).

Preparation Example 106

$N^a$-[4-(Hydroxyamino)-2(R)-isopropyl-3(RS)-[3-(p-aminomethylphenyl)propyl]succinyl]-L-ornithine N-methylamide • 2 acetate

The procedure of Preparation Example 23 was repeated using 6-(p-benzyloxycarbonylaminomethylphenyl)-3(RS)-tert-butyloxycarbonyl-2(R)-isopropylhexanoic acid and Compound No. 7 to provide the title compound as a white solid.

FABMS ($M^+$ + 1): 451.
$^1$H-NMR (DMSO-$d_6$)δ ppm; 0.85-0.86 (6H, m, CH$\underline{(CH_3)_2}$), 1.1-1.9 (15H, m, $\underline{CH}(CH_3)_2$ + CH-$\underline{(CH_2)_2}$ × 2 + $\underline{CH_3}CO_2H$ × 2), 3.83 (2H, s, $\underline{CH_2}$-$NH_2$), 7.0-7.3 (4H, m, aromatic-H).

Preparation Example 107

$N^4$-[2-Amino-1(S)-methylcarbamoylethyl]-$N^1$-hydroxy-2(R or S)-[3-(p-aminomethylphenyl)propyl]-3(R)-isopropyl succinamide • 2 acetate

The procedure of Preparation Example 23 was repeated using 6-(p-benzyloxycarbonylaminomethylphenyl)-3(RS)-tert-butyloxycarbonyl-2(R)-isopropylhexanoic acid and 2(S)-tert-butyloxycarbonylamino-3-benzyloxycarbonylaminobutanoic acid N-methylamide to provide the title compound as a white solid.

FABMS ($M^+$ + 1): 422.
$^1$H-NMR ($CD_3OD$)δ ppm; 0.8-1.0 (6H, m, CH($\underline{CH_3}$)$_2$), 1.1-1.2 (1H, m, $\underline{CH}$($CH_3$)$_2$), 1.2-1.8 (4H, m, CH-($\underline{CH_2}$)$_2$), 1.9 (6H, s, $\underline{CH_3}CO_2H \times 2$), 2.28 (1H, m, $\underline{CH}$-CO), 2.4-2.6 (3H, m, $\underline{CH}$-CO + $C_6H_4$-$\underline{CH_2}$), 2.68 (3H, s, N-$\underline{CH_3}$), 4.02 (2H, s, Ph-$\underline{CH_2}$-$NH_2$), 4.3 (1H, m, NH-$\underline{CH}$-CO), 7.21 (4H, m, aromatic-H).

Biological Example 1

Assay for inhibition of collagenase

The efficacy of compounds of the present invention to act as inhibitors of human fibroblast collagenase was determined by the procedure of Y. Murawaki et al. (Journal of Hepatology, 18, p.328-334, 1993), compared with a reference compound.

Procollagenase was activated by incubation with 2 mM 4-aminophenylmercuric acetate (APMA) at 35°C for 2 hours. The inhibition was assayed using, as a substrate, fluorescein-labeled bovine type I collagen. To a solution of the substrate (0.5 mg/ml) in a 50 mM Tris-HCl buffer, pH 7.5, containing 0.4M aqueous sodium chloride and 10 mM aqueous potassium chloride was added the activated collagenase. The resultant solution was incubated at 35°C for 2 hours. The digestion of the substrate with the enzyme was stopped by addition of 80 mM o-phenanthroline, followed by addition of a porcine elastase solution formed by dissolving 25 μg/ml porcine elastase in the aforementioned Tris-HCl buffer. The mixture was incubated at 37°C for 10 minutes. To the resulting solution was added 70% ethanol, and a 170 mM Tris-HCl buffer, pH 9.5, containing 0.67M aqueous sodium chloride. Undigested substrates were precipitated by centrifugation at 3000 x g for 20 minutes. The supernatant was collected and the fluorescence was read using an excitation wavelength of 495 nm and an emission wavelength of 520 nm. The inhibitory potency of the compounds was calculated. $IC_{50}$ represents the concentration of each test compound requited for 50% inhibition of cleavage of substrates by the enzyme alone. The resulting assay data for representative examples are shown in Table 1, compared with the reference compound. The Reference Compound No. 1 is N-[4-(N-hydroxyamino)-2(R)-n-propyloxymethyl-3(S)-isopropyl-thiomethylsuccinyl)-O-methyl-L-tyrosine-N-methylamide which is synthesized according to the procedure of USP No. 5,442,110.

Table 1

| Inhibitory Activity on MMP-1 ($IC_{50}$, nM) | | | | |
|---|---|---|---|---|
| Compounds | Inhibition of MMP-1 | | Compounds | Inhibition of MMP-1 |
| Compound No. 24 | 5 | | Compound No. 71 | 4 |
| Compound No. 25 | 10 | | Compound No. 73 | 11 |
| Compound No. 27 | 7 | | Compound No. 78 | 18 |
| Compound No. 28 | 10 | | Compound No. 79 | 5 |
| Compound No. 30 | 10 | | Compound No. 80 | 9 |
| Compound No. 31 | 12 | | Compound No. 81 | 13 |
| Compound No. 34 | 13 | | Compound No. 82 | 4 |
| Compound No. 36 | 14 | | Compound No. 84 | 11 |
| Compound No. 46 | 15 | | Compound No. 91 | 17 |
| Compound No. 50 | 13 | | Reference Compound No. 1 | 11 |
| Compound No. 51 | 9 | | | |

Biological Example 2

Assay for inhibition of stromelysin

The efficacy of compounds of the present invention to act as inhibitors of human fibroblast stromelysin was determined by the procedure of Twining (Anal. Biochem., 143, p.30, 1984), compared with a reference compound.

Prostromelysin was activated by incubation with 20 μg/ml human plasmin at 37°C for 2 hours, and the reaction was stopped by addition of 2.8 mg/ml aqueous diisopropyl fluorophosphate. The inhibition was assayed using, as a substrate, fluorescein-labeled casein. To a solution of the substrate (1 mg/ml) in a 50 mM Tris-HCl buffer, pH 7.8, containing 10mM aqueous calcium chloride was added the activated stromelysin. The resultant solution was incubated at 37°C for 2 hours. The digestion of the substrate with the enzyme was stopped by addition of 5% trichloroacetic acid. Undigested substrates were precipitated by centrifugation at 3000 x g for 20 minutes. The supernatant was collected, followd by additon of a 0.5M Tris-HCl buffer, pH 8.5. The fluorescence was read using an excitation wavelength of 495 nm and an emission wavelength of 520 nm. The inhibitory potency of the compounds was calculated. $IC_{50}$ represents the concentration of each test compound requited for 50% inhibition of cleavage of substrates by the enzyme alone. The resulting assay data for representative examples are shown in Table 2, compared with the reference compound.

All isomers of the compounds of the present invention having the same optical configurations as the Reference Compound exert more intense inhibition than those of the Reference Compound.

Table 2

| Inhibitory Activity on MMP-3 ($IC_{50}$, nM) | | | | |
|---|---|---|---|---|
| Compounds | Inhibition of MMP-3 | | Compounds | Inhibition of MMP-3 |
| Compound No. 23 | 16 | | Compound No. 42 | 24 |
| Compound No. 24 | 18 | | Compound No. 44 | 17 |
| Compound No. 26 | 40 | | Compound No. 46 | 42 |
| Compound No. 27 | 60 | | Compound No. 50 | 9 |
| Compound No. 30 | 12 | | Compound No. 51 | 2 |
| Compound No. 31 | 11 | | Compound No. 52 | 23 |
| Compound No. 32 | 12 | | Compound No. 54 | 10 |
| Compound No. 33 | 12 | | Compound No. 61 | 23 |
| Compound No. 34 | 11 | | Compound No. 68 | 14 |
| Compound No. 35 | 19 | | Compound No. 69 | 20 |
| Compound No. 36 | 9 | | Compound No. 70 | 28 |
| Compound No. 37 | 50 | | Compound No. 74 | 62 |
| Compound No. 41 | 18 | | Reference Compound No. 1 | 100 |

Biological Example 3

Assay for inhibition of TNF-α production

Human monocytic leukemia cell line U937 (human histiocytic lymphoma cell line U937) cells were cultured in RPMI 1640 (Nissui Pharmaceutical Co., Ltd., Japan) supplemented with 5% fetal calf serum (FCS; IBL, Japan) in an incubator at 37°C under 5% $CO_2$, then transferred to multiplates at a density of 1 x $10^6$ cells/1 ml/well, and incubated overnight in the presence of $10^{-7}$ M phorbol 12-myristate 13-acetate (Wako Pure Chemical Industries, Ltd., Japan) to form differentiated macrophage-like cells. To the resultant cells was added E. coli O127:B8 lipopolysaccaride (LPS, 0.1 μg/ml; Sigma) alone or along with each compound of the present invention, and the treated cells were then incubated for 6 hours in an incubator under 5% $CO_2$ at 37°C. After the incubation, the cultures were collected and centrifuged at 3000 rpm for 10 minutes to give supernatants which were diluted with purified water and then assayed using a TNF-α assay kit, Genzyme (Table 3). The Reference Compound No. 2 is $N^2$-[3S-hydroxy-4-(N-hydroxyamino)-2R-isobutylsuccinyl]-L-tert-leucine-$N^1$-methylamide which is synthesized according to the procedure of GB 2,268,934 A.

Table 3

| Inhibitory Activity on TNF-α Convertase ($IC_{50}$ nM) | |
|---|---|
| Compounds | Inhibition of TACE |
| Compound No. 22 | 2080 |
| Compound No. 23 | 1193 |
| Compound No. 24 | 480 |
| Compound No. 34 | 1040 |
| Compound No. 41 | 1012 |
| Compound No. 44 | 129 |
| Compound No. 46 | 460 |
| Compound No. 47 | 210 |
| Compound No. 50 | 599 |
| Compound No. 51 | 426 |
| Compound No. 54 | 340 |
| Compound No. 55 | 279 |
| Compound No. 58 | 942 |
| Compound No. 61 | 1098 |
| Compound No. 62 | 2893 |
| Compound No. 65 | 421 |
| Compound No. 67 | 527 |
| Compound No. 71 | 352 |
| Compound No. 72 | 250 |
| Compound No. 74 | 603 |
| Compound No. 81 | 737 |
| Compound No. 89 | 1900 |
| Reference Compound No. 2 | 3300 |

Biological Example 4

Assay for Oral Absorption in Rat

Each compound of the present invention was dissolved in distilled water for injection and orally administered to rats (Lewis strain line, male, body weight: about 200 g) at a dose of 100mg/kg. From 30 minutes to 4 hours after the administration, a series of blood chronologically collected from rat fundus oculi were centrifuged (3000rpm for 10 minutes at 4°C) to give plasma samples. Each plasma sample (200 µl) was filtered through an ultrafiltration membrane by centrifugation at 1500 x G for 3 minutes using a micropartition UF unit (Centrifree-MPS-3: 30,000 MW cut-off, Amicon Corp., Beverly, MA). The resultant filtrate (10 µl) was mixed with 20 µl of purified rabbit MMP-3 activated with 1.5mM APMA, along with 60 µl of a synthetic substrate solution (MocAc-Arg-Pro-Lys-Pro-Val-Glu-Nva-Trp-Arg-Lys(Dnp)-$NH_2$, Peptide Institute Inc., Japan), and the mixture was incubated at 37°C for 2 hours. The reaction was stopped by adding 900 µl of 3% aqueous acetic acid to the reaction mixture. The fluorescence of the resulting solution was read using an excitation wavelength of 325 nm and an emission wavelength of 393 nm. The concentration of each test compound in rat plasma samples was calculated using a standard curve which was prepared based on a variety of doses. An area under the dose plasma concentration-time curve from 0.5 to 4 hours after the drug administration ($AUC_{0.5-4}$) for each compound was calculated from the resulting data (Table 4).

Table 4

| Area under Dose Plasma Concentration-Time Curve of Compound ($AUC_{0.5-4}$) | |
|---|---|
| Compounds | $AUC_{0.5-4}$ (ng • h/ml) |
| Compound No. 22 | 9128 |
| Compound No. 23 | 4757 |
| Compound No. 39 | 14996 |
| Compound No. 41 | 3131 |
| Compound No. 42 | 6255 |
| Compound No. 43 | 3289 |
| Compound No. 44 | 1929 |
| Compound No. 45 | 7067 |
| Compound No. 46 | 3379 |
| Compound No. 48 | 20203 |
| Compound No. 49 | 15292 |
| Compound No. 53 | 4816 |
| Compound No. 61 | 13765 |
| Compound No. 63 | 5662 |
| Compound No. 69 | 3339 |
| Compound No. 72 | 4042 |
| Compound No. 74 | 9025 |
| Reference Compound No. 2 | 1574 |

Biological Example 5

Acute Toxicity Study

Each compound of the present invention was dissolved in distilled water for injection to make the concentration 10 mg/ml. The resulting solution was intravenously injected into a mouse tail at a dose of 10 mg/kg. During 8 days, the conditions of rats were observed. The animals received each compound of the present invention (Compound Nos. 22 to 46) were found to neither die nor reduce their body weight.

Described below are pharmaceutical preparations containing the compound (I) provided by the present invention.

Formulation Example 1

Ointments:

Ointments each containing the following ingredients were prepared according to conventional techniques:

A;

| Ingredients | Amount |
|---|---|
| White Petrolatum | 93 g |

(continued)

| Ingredients | Amount |
|---|---|
| Liquid Paraffin | 5 g |
| Compound No. 51 | 2 g |
| Total Amount | 100 g |

B;

| Ingredients | Amount |
|---|---|
| White Petrolatum | 94 g |
| Purified Lanolin | 5 g |
| Compound No. 47 | 1 g |
| Total Amount | 100 g |

C;

| Ingredients | Amount |
|---|---|
| White Petrolatum | 96 g |
| Compound No. 56 | 4 g |
| Total Amount | 100 g |

Formulation Example 2

Ophthalmic solutions:

Ophthalmic solutions each containing the following ingredients were prepared according to conventional techniques:

A;

| Ingredients | Amount |
|---|---|
| Compound No. 23 | 2 g |
| Sodium Chloride | 0.33 g |
| Sterile Purified Water | qs |
| Total volume | 100 ml |

B;

| Ingredients | Amount |
|---|---|
| Compound No. 36 | 5 g |
| Sodium Chloride | 0.26 g |
| Sodium Dihydrogenphosphate anhydrous | 0.56 g |
| Disodium Phosphate anhydrous | 0.28 g |
| 0.002% Aqueous Benzalkonium Chloride | qs |
| Total Volume | 100 ml |

C;

| Ingredients | Amount |
|---|---|
| Compound No. 25 | 1 g |
| Sodium Chloride | 0.33 g |
| Sodium Sulfite Anhydrous | 0.10 g |
| Sterile Purified Water | qs |
| Total Volume | 100 ml |

D;

| Ingredients | Amount |
|---|---|
| Compound No. 55 | 1 g |
| Sodium Chloride | 0.33 g |
| Sterile Purified Water | qs |
| Total volume | 100 ml |

<u>Formulation Example 3</u>

<u>Injections</u>:

Preparations for injection each containing the following ingredients were formulated according to conventional techniques:

A;

| Ingredients | Amount |
|---|---|
| Compound No. 81 | 1 g |
| Distilled Water for Injection | qs |

(continued)

| Ingredients | Amount |
|---|---|
| Total Volume | 100 ml |

B;

| Ingredients | Amount |
|---|---|
| Compound No. 82 | 2 g |
| Sodium Chloride | 0.9 g |
| Distilled Water for Injection | qs |
| Total Volume | 100 ml |

C;

| Ingredients | Amount |
|---|---|
| Compound No. 58 | 1 g |
| Sodium Dihydrogenphosphate anhydrous | 28 mg |
| Disodium Phosphate anhydrous | 167 mg |
| Sodium Chloride | 0.9 g |
| Distilled Water for Injection | qs |
| Total volume | 100 ml |

Formulation Example 4

Tablets:

Tablets each containing the following ingredients were prepared according to conventional techniques:

A;

| Ingredients | In Each |
|---|---|
| Compound No. 9 | 100 mg |
| Lactose | 98 mg |
| Corn starch | 46 mg |
| Hydroxypropyl Cellulose | 2 mg |
| Magnesium stearate | 4 mg |
| Total Amount | 250 mg |

B;

| Ingredients | In Each |
|---|---|
| Compound No. 23 | 50 mg |
| Lactose | 120 mg |
| Corn starch | 15 mg |
| Hydroxypropyl Cellulose | 4 mg |
| Carboxymethylcellulose Calcium | 10 mg |
| Magnesium stearate | 1 mg |
| Total Amount | 200 mg |

Formulation Example 5

Granules:

Granules each containing the following ingredients were prepared according to conventional techniques:

| Ingredients | Amount |
|---|---|
| Compound No. 74 | 2 g |
| Lactose | 1.85 g |
| Corn starch | 1.1 g |
| Hydroxypropyl Cellulose | 50 mg |
| Total Amount | 5 g |

Formulation Example 6

Capsules:

Capsules each containing the following ingredients were prepared according to conventional techniques:

| Ingredients | Amount |
|---|---|
| Compound No. 80 | 100 mg |
| Lactose | 35 mg |
| Corn starch | 60 mg |
| Magnesium stearate | 5 mg |
| Total Amount | 200 mg |

The following simbols are intended to have the meanings set forth below in the specification and the appended claims:

$$N^{a} = N^{\alpha}$$
$$N^{e} = N^{\varepsilon}$$
$$N^{d} = N^{\delta}$$
$$N^{g} = N^{g}$$
$$N^{w} = N^{\omega}$$

Industrial Applicability

The compounds provided by the present invention, for example, the compounds of the formula (I), exert potent metalloproteinase-inhibiting activity and have not only excellent inhibitory actions on MMPs and/or TNF-α convertases but also remarkably improved bioavailablity, in comparison with the prior art compounds. Accordingly, these compounds can be applied to diseases and/or disorders associated with tissue degradation, leading to unexpectedly excellent actions and effects and promising therapeutic and/or prophylactic results.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

**Claims**

**1.** A compound having the following formula (I):

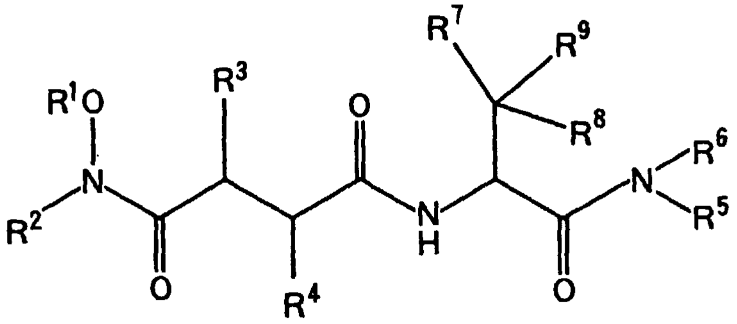

(I)

wherein $R^1$ is selected from the group consisting of hydrogen, unsubstituted or optionally substituted aralkyl, silyl which may optionally have three substituents, tetrahydropyranyl, unsubstituted or optionally substituted aralkyloxycarbonyl, unsubstituted or optionally substituted alkyloxycarbonyl, unsubstituted or optionally substituted alkyl, and a hydroxy-protecting group;

$R^2$ is selected from the group consisting of hydrogen, unsubstituted or optionally substituted aralkyloxycarbonyl, unsubstituted or optionally substituted alkyloxycarbonyl, 9-fluorenylmethyloxycarbonyl, and an amino-protecting group;

$R^3$ is selected from the group consisting of hydrogen, hydroxy, unsubstituted or optionally substituted alkyl, and unsubstituted or optionally substituted aralkyl;

$R^4$ is selected from the group consisting of unsubstituted or optionally substituted alkyl, and unsubstituted or optionally substituted aralkyl;

$R^5$ and $R^6$, which may be identical or different, are each independently selected from the group consisting of hydrogen, unsubstituted or optionally substituted alkyl, unsubstituted or optionally substituted cycloalkyl, an

unsubstituted or optionally substituted heterocyclic group, and an amino-protecting group, or $R^5$ and $R^6$ taken together with the nitrogen atom to which they are attached form an unsubstituted or optionally substituted heterocyclic group;

$R^7$ is selected from the group consisting of hydrogen, hydroxy, unsubstituted or optionally substituted alkyl, and unsubstituted or optionally substituted aralkyl;

$R^8$ is selected from the group consisting of hydrogen, hydroxy, unsubstituted or optionally substituted alkyl, and unsubstituted or optionally substituted aralkyl; and

$R^9$ is selected from the group consisting of hydrogen, hydroxy, amino, and a group of the formula: -X-Y

wherein X is selected from the group consisting of unsubstituted or optionally substituted ($C_1$-$C_6$) alkylene, and unsubstituted or optionally substituted phenylene, and

Y is a group of the formula: -A-B or -B,

wherein A is selected from the group consisting of unsubstituted or optionally substituted ($C_1$-$C_6$) alkylene, oxygen, sulfur, imino, and unsubstituted or optionally substituted ($C_1$-$C_6$) alkyleneimino, and

B is selected from the group consisting of hydrogen, amino, amidino, acylimidoyl, unsubstituted or optionally substituted imidazolyl, unprotected or optionally protected bis(phosphono)methyl, and unprotected or optionally protected bis(phosphono)hydroxymethyl;

or a pharmaceutically acceptable salt or solvate thereof.

**2.** The compound according to claim 1 wherein

$R^1$ and $R^2$ are hydrogen;

$R^3$ is selected from the group consisting of ($C_1$-$C_9$) alkyl, ($C_3$-$C_7$) cycloalkyl-substituted lower ($C_1$-$C_4$) alkyl, hydroxy, amino-substituted ($C_1$-$C_6$)alkyl, phenyl-lower ($C_1$-$C_4$) alkyl, guanido-substituted phenyl-lower ($C_1$-$C_4$) alkyl, amino-substituted phenyl-lower ($C_1$-$C_4$) alkyl, carboxy-substituted phenyl-lower ($C_1$-$C_4$) alkyl, carbamoyl-substituted phenyl-lower ($C_1$-$C_4$) alkyl, hydroxy-substituted phenyl-lower ($C_1$-$C_4$) alkyl, guanido-substituted lower ($C_1$-$C_4$) alkyl-substituted phenyl-lower ($C_1$-$C_4$) alkyl, unprotected or optionally protected amino-substituted lower ($C_1$-$C_4$) alkyl-substituted phenyl-lower ($C_1$-$C_4$) alkyl, hydroxy-substituted lower ($C_1$-$C_4$) alkyl-substituted phenyl-lower ($C_1$-$C_4$) alkyl, lower ($C_1$-$C_4$) alkoxycarbonyl-substituted phenyl-lower ($C_1$-$C_4$) alkyl, lower ($C_1$-$C_4$) alkylimino-substituted ($C_1$-$C_6$) alkyl, lower ($C_1$-$C_4$) acylimidoylimino-substituted ($C_1$-$C_6$) alkyl, arylmethylimino-substituted ($C_1$-$C_6$) alkyl, nitrogen-containing heterocyclic radical-substituted lower ($C_1$-$C_4$) alkylimino-substituted ($C_1$-$C_6$) alkyl, nitrogen-containing heterocyclic radical-substituted lower ($C_1$-$C_4$) alkyl, oxygen-containing ($C_1$-$C_8$) straight chain or branched alkyl, arylsulfonamido-substituted lower ($C_1$-$C_4$) alkyl-substituted phenyl-lower ($C_1$-$C_4$) alkyl, alkylsulfonamido-substituted lower ($C_1$-$C_4$) alkyl-substituted phenyl-lower ($C_1$-$C_4$) alkyl, aryloxy-substituted lower ($C_1$-$C_4$) alkyl, and hydroxy-substituted ($C_1$-$C_8$) alkyl;

$R^4$ is selected from the group consisting of ($C_3$-$C_9$) alkyl, hydroxy-substituted ($C_3$-$C_8$) alkyl, and unsubstituted or optionally substituted aryl-lower ($C_1$-$C_4$) alkyl;

$R^5$ is selected from the group consisting of lower ($C_1$-$C_4$) alkyl, ($C_3$-$C_7$) cycloalkyl, mono- or di-lower ($C_1$-$C_4$) alkylamino-substituted lower ($C_1$-$C_4$) alkyl, carboxy-substituted lower ($C_1$-$C_4$) alkyl, hydroxy-substituted ($C_1$-$C_6$) alkyl, bis(phosphono)hydroxymethyl-substituted ($C_1$-$C_{11}$) alkyl, tetrabenzyl bis(phosphono)hydroxymethyl-substituted ($C_1$-$C_{11}$) alkyl, and a nitrogen-containing heterocyclic radical;

$R^6$ is hydrogen;

$R^7$ is hydrogen or lower ($C_1$-$C_4$) alkyl;

$R^8$ is hydrogen or lower ($C_1$-$C_4$) alkyl; and

$R^9$ is selected from the group consisting of hydrogen, hydroxy, amino, and a group of the formula: -X-Y

wherein X is ($C_1$-$C_6$) alkylene or phenylene, and

Y is a group of the formula: -A-B or -B

wherein B is selected from the group consisting of hydrogen, amino, amidino, lower ($C_1$-$C_4$) acylimidoyl, unsubstituted or optionally substituted benzimidoyl, bis(phosphono)methyl, tetra-lower ($C_1$-$C_4$) alkyl bis(phosphono)methyl, tri-lower ($C_1$-$C_4$) alkyl bis(phosphono)methyl, bis(phosphono)hydroxymethyl, tetrabenzyl bis(phosphono)hydroxymethyl, and lower ($C_1$-$C_4$) alkyl-substituted imidazol-3-yl, and

A is selected from the group consisting of lower ($C_1$-$C_4$) alkylene, imino, and lower ($C_1$-$C_4$) alkylene-imino.

**3.** The compound according to claim 1 wherein

$R^1$ and $R^2$ are hydrogen;

$R^3$ is selected from the group consisting of hydroxy, methyl, isobutyl, aminopropyl, phenylpropyl, guanidophenylpropyl, aminophenylpropyl, hydroxyphenylpropyl, carboxyphenylpropyl, carbamoylphenylpropyl, aminomethylphenylpropyl, guanidomethylphenylpropyl, hydroxymethylphenylpropyl, aminomethylbenzyl, toluenesulfonamidomethylbenzyl, methanesulfonamidomethylbenzyl, isobutyliminomethylbenzyl, phthalimidomethylbenzyl, phenoxyethyl, aminopentyl, acetimidoyliminopentyl, isobutyliminopentyl, pyridylmethyliminopentyl, methoxycarbonylphenylpropyl, ethoxyethoxyethyl, hydroxyoctyl, butoxyethyl, iso-butyloxyethyl, morpholinopropyl, (3,4,4-trimethyl-2,5-dioxo-imidazolidin-1-yl )propyl, cyclohexylpropyl, and piperidinopropyl;
$R^4$ as selected from the group consisting of naphthylmethyl, phenylpropyl, isobutyl, tert-butyl, isopropyl, and hydroxyoctyl;
$R^5$ is selected from the group consisting of methyl, cyclopropyl, 2-(N,N-dimethylamino)ethyl, 2-carboxyethyl, 2-hydroxyethyl, 2-hydroxy-1,1-dimethylethyl, 2-hydroxy-1-methylethyl, 6,6-bis(phosphono)-6-hydroxyhexyl, tetrabenzyl 6,6-bis(phosphono)-6-hydroxyhexyl, piperidyl, and morpholinyl;
$R^6$ is hydrogen;
$R^7$ is hydrogen or methyl;
$R^8$ is hydrogen or methyl; and
$R^9$ is selected from the group consisting of hydrogen, hydroxy, amino, and a group of the formula: -X-Y

wherein x is selected from the group consisting of methylene, ethylene, trimethylene, tetramethylene, pentamethylene, hexamethylene, and phenylene, and
Y is a group of the formula: -A-B or -B
wherein B is selected from the group consisting of amino, amidino, acetimidoyl, propionimidoyl, benzimidoyl, bis(phosphono)methyl, tetraethyl bis(phosphono)methyl, triethyl bis(phosphono)methyl, tetramethyl bis(phosphono)methyl, trimethyl bis(phosphono)methyl, bis(phosphono)hydroxymethyl, tetrabenzyl bis(phosphono)hydroxymethyl, and 2-methyl-imidazol-3-yl, and
A is selected from the group consisting of imino, methyleneimino, and methylene.

**4.** A pharmaceutical or veterinary composition which comprises (a) an effective amount of at least a member selected from the group consisting of a compound of the formula (I):

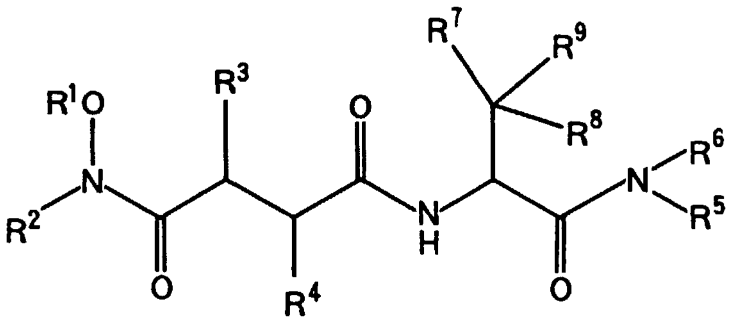

(I)

wherein $R^1$ to $R^9$, all have the same meanings as defined in claim 1, and a pharmaceutically or veterinarily acceptable salt or solvate thereof, and (b) a pharmaceutically or veterinarily acceptable excipient or carrier.

**5.** A metalloproteinase inhibitor which comprises an effective amount of at least a member selected from the group consisting of a compound of the formula (I):

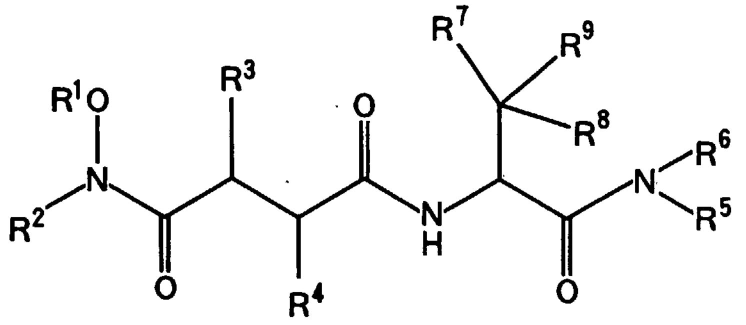

(I)

wherein $R^1$ to $R^9$, all have the same meanings as defined in claim 1, and a pharmaceutically acceptable salt or solvate thereof.

**6.** The inhibitor according to claim 5 wherein the metalloproteinase is selected from the group consisting of matrix metalloproteinases and the inhibitor is a kind of inhibitors of matrix metalloproteinase.

**7.** The inhibitor according to claim 5 wherein the metalloproteinase is selected from the group consisting of tumor necrosis factor-α (TNF-α)-converting enzymes and the inhibitor is a kind of inhibitors of TNF- α convertase.

**8.** Use of a compound of the formula (I) according to claim 1 for prophylactically and/or therapeutically treating diseases and/or disorders associated with tissue degradation.

**9.** A process for producing a compound having the following formula (I):

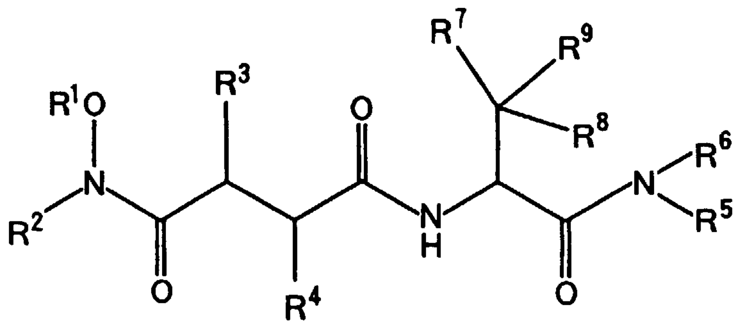

(I)

or a pharmaceutically acceptable salt or solvate thereof, which comprises:

(a) converting an ester moiety of a compound of the formula (IV):

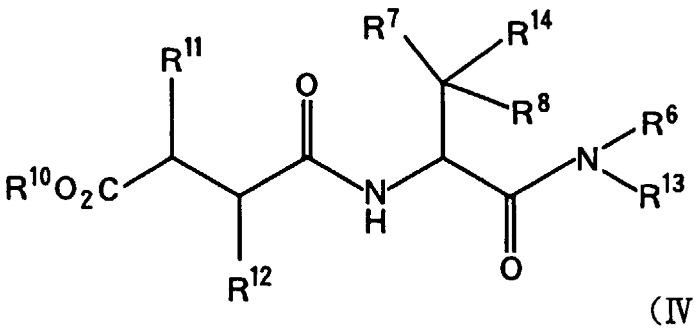

(IV)

into an amide bond-containing moiety thereof, and, if desired, further optionally converting $R^{11}$, $R^{12}$, $R^{13}$, and/or $R^{14}$ into the target functional group(s), $R^3$, $R^4$, $R^5$, and/or $R^9$, respectively, or

(b) if desired, optionally converting $R^{11}$, $R^{12}$, $R^{13}$, and/or $R^{14}$ in the compound of the formula (IV) into the target functional group(s), $R^3$, $R^4$, $R^5$, and/or $R^9$, respectively, and then converting an ester moiety thereof into an amide bond-containing moiety thereof,

wherein $R^1$ to $R^9$, all have the same meanings as defined in claim 1;
$R^{10}$ is selected from the group consisting of unsubstituted or optionally substituted alkyl, unsubstituted or optionally substituted aralkyl, and a carboxy-protecting group;
$R^{11}$ has the same meaning as defined for $R^3$, or is selected from the group consisting of protected hydroxy, protected guanido-substituted phenyl-lower ($C_1$-$C_4$) alkyl, protected amino-substituted phenyl-lower ($C_1$-$C_4$) alkyl, nitro-substituted phenyl-lower ($C_1$-$C_4$) alkyl, protected amino-substituted ($C_1$-$C_6$) alkyl, nitro-substituted ($C_1$-$C_6$) alkyl, protected carboxy-substituted phenyl-lower ($C_1$-$C_4$) alkyl, protected hydroxy-substituted phenyl-lower ($C_1$-$C_4$) alkyl, protected guanido-substituted lower ($C_1$-$C_4$) alkyl-substituted phenyl-lower ($C_1$-$C_4$) alkyl, protected amino-substituted lower ($C_1$-$C_4$) alkyl-substituted phenyl-lower ($C_1$-$C_4$) alkyl, protected hydroxy-substituted lower ($C_1$-$C_4$) alkyl-substituted phenyl-lower ($C_1$-$C_4$) alkyl, protected carboxy-substituted lower ($C_1$-$C_4$) alkyl-substituted phenyl-lower ($C_1$-$C_4$) alkyl, protected hydroxy-containing ($C_1$-$C_8$) straight chain or branched alkyl, and cyano-substituted phenyl-lower ($C_1$-$C_4$) alkyl;
$R^{12}$ has the same meaning as defined for $R^4$, or is protected hydroxy-substituted ($C_1$-$C_8$) alkyl;
$R^{13}$ has the same meaning as defined for $R^5$, or is selected from the group consisting of protected carboxy-substituted lower ($C_1$-$C_4$) alkyl, protected hydroxy-substituted lower ($C_1$-$C_4$) alkyl, protected bis(phosphono)hydroxymethyl-substituted ($C_1$-$C_{11}$) alkyl, and a protected nitrogen-containing heterocyclic group; and
$R^{14}$ has the same meaning as defined for $R^9$, or is selected from the group consisting of protected amino, protected hydroxy, and a group of the formula: -X-E or -X-A-E

wherein X and A, both have the same meanings as given above, and E is selected from the group consisting of nitro, cyano, amino, carboxyl, ($C_1$-$C_{11}$) hydroxyalkyl, protected amino, protected guanido, protected amidino, protected acylimidoyl, protected benzimidoyl, protected bis(phosphono)methyl, protected bis(phosphono)hydroxymethyl, and protected ($C_1$-$C_{11}$) alkyl-substituted imidazol-3-yl.

**10.** The process according to claim 9 wherein

$R^{10}$ is selected from the group consisting of ($C_1$-$C_6$) alkyl, benzyl, substituted benzyl, phenacyl, and 2,2,2-trichloroethyl;
$R^{11}$ has the same meaning as defined for $R^3$, or is selected from the group consisting of protected guanido-phenylpropyl, protected amino-phenylpropyl, nitro-phenylpropylene, protected aminopropyl, nitropropyl, protected hydroxy-phenylpropyl, protected carboxy-phenylpropyl, protected aminomethyl-phenylpropyl, protected guanidomethyl-phenylpropyl, protected hydroxymethyl-phenylpropyl, protected aminomethyl-benzyl, cyano-phenylpropyl, protected aminopentyl, cyano-benzyl, protected hydroxyoctyl, and nitropentyl;
$R^{12}$ has the same meaning as defined for $R^4$, or is protected hydroxyoctyl;
$R^{13}$ has the same meaning as defined for $R^5$, or is selected from the group consisting of protected 2-carboxyethyl, protected 2-hydroxyethyl, protected 2-hydroxy-1,1-dimethylethyl, protected 2-hydroxy-1-methylethyl,

and protected 6,6-bis-phosphono-6-hydroxy-hexyl;

$R^{14}$ has the same meaning as defined for $R^9$, or is selected from the group consisting of protected amino, protected hydroxy, and a group of the formula: -X-F or -X-A-F

wherein X and A, both have the same meanings as given above, and F is selected from the group consisting of nitro, cyano, amino, carboxyl, hydroxymethyl, protected amino, protected guanido, protected amidino, protected acetimidoyl, protected propionimidoyl, protected benzimidoyl, protected bis(phosphono)methylimino, and protected bis(phosphono)hydroxymethyl.

**11.** A process for producing a compound having the following formula (I):

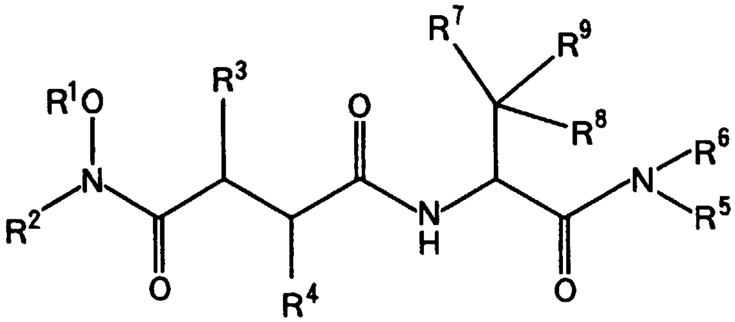

(I)

or a pharmaceutically acceptable salt or solvate thereof, which comprises:

(a) reacting a hydroxamic acid backbone-containing succinic acid derivative of the formula (V):

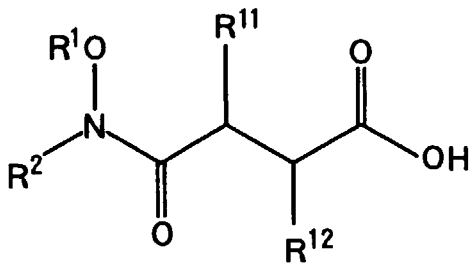

(V)

with an amine derivative of the formula (III):

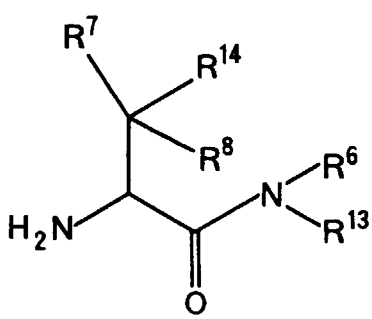

(Ⅲ)

to form a compound of the formula (VI):

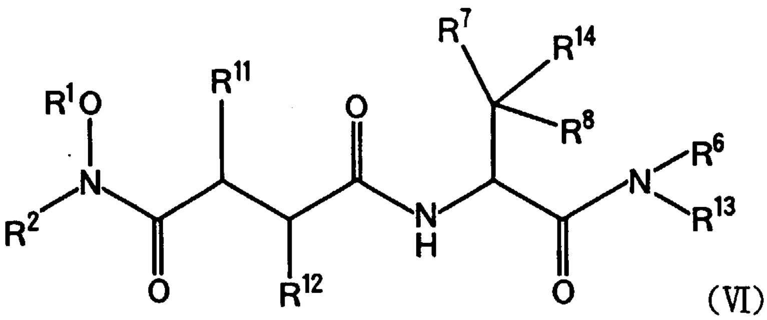

(VI)

and, if desired, optionally converting $R^{11}$, $R^{12}$, $R^{13}$ and/or $R^{14}$ into the target functional group(s), $R^3$, $R^4$, $R^5$ and/or $R^9$, respectively,

wherein $R^1$ to $R^9$, all have the same meanings as defined in claim 1, and $R^{11}$ to $R^{14}$, all have the same meanings as defined in claim 9.

**12.** A compound having the following formula (VI):

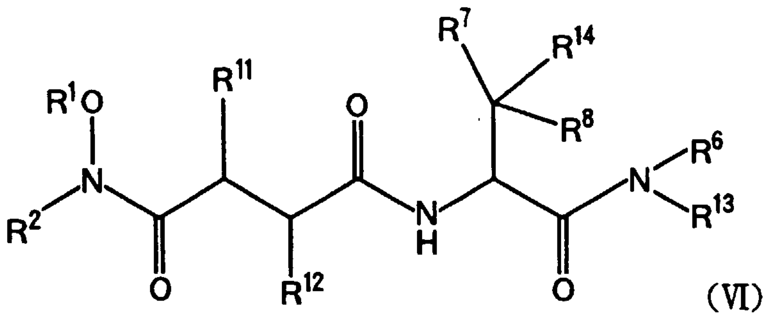

(VI)

wherein $R^1$, $R^2$, and $R^6$ to $R^8$, all have the same meanings as defined in claim 1, and $R^{11}$ to $R^{14}$, all have the same meanings as defined in claim 9,
or a salt thereof.

**13.** A compound having the following formula (IV):

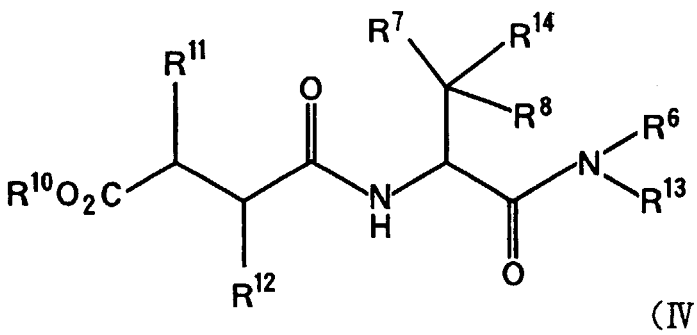

(IV)

wherein $R^6$ to $R^8$, all have the same meanings as defined in claim 1, and $R^{10}$ to $R^{14}$, all have the same meanings

as defined in claim 9, or a salt thereof.

**14.** A process for producing a compound having the following formula (IV):

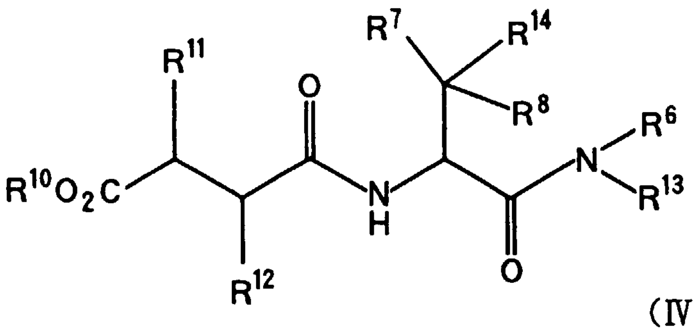

(IV)

or a salt thereof, which comprises reacting a succinic acid derivative of the formula (II):

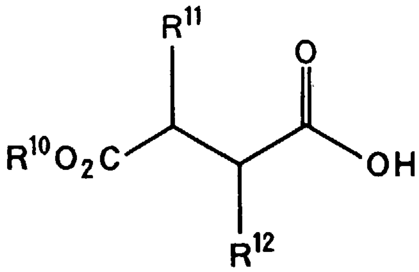

(II)

with an amine derivative of the formula (III):

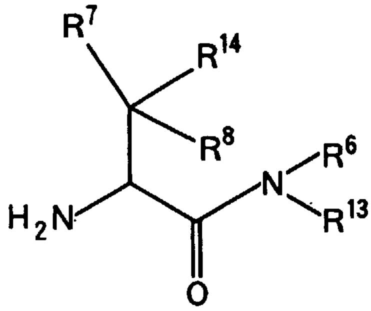

(III)

wherein $R^6$ to $R^8$, all have the same meanings as defined in claim 1, and $R^{10}$ to $R^{14}$, all have the same meanings as defined in claim 9.

**15.** A compound having the following formula (III):

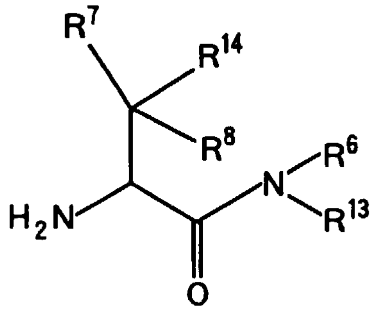

(Ⅲ)

wherein $R^6$ to $R^8$, all have the same meanings as defined in claim 1, and $R^{13}$ and $R^{14}$, both have the same meanings as defined in claim 9,
or a pharmaceutically acceptable salt or solvate thereof.

# INTERNATIONAL SEARCH REPORT

International application No.
PCT/JP98/05620

A. CLASSIFICATION OF SUBJECT MATTER
Int.Cl[6] C07C259/06, C07C237/22, C07C213/74, C07D233/74, C07D295/18, C07F9/38, C07H13/04, A61K31/215, A61K31/275, A61K31/27,

According to International Patent Classification (IPC) or to both national classification and IPC

B. FIELDS SEARCHED

Minimum documentation searched (classification system followed by classification symbols)
Int.Cl[6] C07C259/06, C07C237/22, C07C213/74, C07D233/74, C07D295/18, C07F9/38, C07H13/04, A61K31/215, A61K31/275, A61K31/27,

Documentation searched other than minimum documentation to the extent that such documents are included in the fields searched

Electronic data base consulted during the international search (name of data base and, where practicable, search terms used)
CAPLUS (STN), CAOLD (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| A<br>Y | JP, 4-352757, A (F.Hoffmann-La Roche AG.), 7 December, 1992 (07. 12. 92) & EP, 497192, A2 & US, 5304549, A | 1-7, 9-12<br>14 |
| X | JP, 57-116043, A (Tetsuo Suami), 19 July, 1982 (19. 07. 82) & US, 4452814, A | 15 |
| X<br>Y | JP, 56-158746, A (The Wellcome Foundation Ltd.), 7 December, 1981 (07. 12. 81) & EP, 38046, A2 | 13<br>14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>9 March, 1999 (09. 03. 99) | Date of mailing of the international search report<br>16 March, 1999 (16. 03. 99) |
|---|---|
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP98/05620

**Box I Observations where certain claims were found unsearchable (Continuation of item 1 of first sheet)**

This international search report has not been established in respect of certain claims under Article 17(2)(a) for the following reasons:

1. [x] Claims Nos.: 8
because they relate to subject matter not required to be searched by this Authority, namely:
It pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1992)

INTERNATIONAL SEARCH REPORT

International application No.
PCT/JP98/05620

A. (Continuation) CLASSIFICATION OF SUBJECT MATTER

A61K31/16, A61K31/18, A61K31/24, A61K31/70, A61K31/445, A61K31/535, A61K31/44, A61K31/415

B. (Continuation) FIELDS SEARCHED

A61K31/16, A61K31/18, A61K31/24, A61K31/70, A61K31/445, A61K31/535, A61K31/44, A61K31/415

Form PCT/ISA/210 (extra sheet) (July 1992)